# EUROPEAN PATENT APPLICATION

(11) **EP 2 615 094 A2**
(43) Date of publication of application: **17.07.2013**
(21) Application number: 11823786.6
(22) Date of filing: 07.09.2011
(51) Int. Cl.: C07D 493/10, C07D 493/20, A61K 31/352, A61P 3/00, A61P 9/00

(54) **SESTERTERPENE COMPOUNDS AND USE THEREOF**

(30) Priority: 07.03.2011 KR 20110020118; 07.09.2010 KR 20100087552
(71) Applicant: SNU R & DB Foundation, Gwanak-gu, Seoul 151-015 (KR)
(72) Inventor: KANG, Heonjoong, Seoul 137-060 (KR); WON, Dong Hwan, Yongin-si Gyeonggi-do 446-930 (KR); YANG, Inho, Seoul 135-280 (KR); KIM, Eun Oh, Seoul 151-050 (KR); KIM, Jung Ah, Daegu 705-023 (KR); GIRI, Awadut Gajendra, Seoul 151-050 (KR); MALLEPALLY, Venkat Reddy, Seoul 151-050 (KR)
(74) Representative: Herrmann, Uwe
(86) International application number: PCT/KR2011/006638
(87) International publication number: WO 2012/033353

(57) **Abstract**

The present invention relates to sesterterpene compounds, to the precursors thereof that are hydrolysable in a living body, or to the pharmaceutically acceptable salts thereof, and also relates to the prevention and treatment efficacy of the sesterterpene compounds with respect to non-insulin dependent diabetes mellitus, diabetic complications (renal failure and foot ulcers caused by diabetes), alcoholic, non-alcoholic, and viral fatty liver diseases, obesity, hyperlipidemia, atherosclerosis, cardiovascular diseases such as atherosclerotic stroke, and cerebropathies (Parkinsonism, schizophrenia and Alzheimer's disease). In addition, the present invention relates to compositions for functional foods, functional beverages, functional cosmetics, and functional feed.

## Description

### [Technical Field]

The present invention relates to noble sesterterpene compounds and preventive efficacy of the sesterterpene compounds to prevent and treat diabetes, obesity, fatty liver diseases (alcoholic, non-alcoholic, and viral fatty liver diseases), cardiovascular diseases (hyperlipidemia, atherosclerosis) and brain diseases (Parkinson's and Alzheimer's disease). The sesterterpene compounds of the present invention may be used as an effective component of a composition for preventing and treating Type 2 diabetes and secondary diseases caused by diabetes (renal failure and foot ulcer) by controlling expressions of proteins having antidiabetic efficacy and intercellular signals. In addition, the sesterterpene compounds of the present invention may be used as an effective component of a composition for preventing and treating alcoholic, non-alcoholic, and viral fatty liver diseases by inhibiting the formation of fatty acids in the liver and promoting beta-oxidation by which the fatty acids are burn to release the heat to thereby significantly reduce fat accumulation in the liver. In addition, the sesterterpene compounds of the present invention may be used as an effective component of a composition for preventing and treating obesity by inhibiting the differentiation of adipocytes. Moreover, the sesterterpene compounds of the present invention may be used as an effective component of a composition for preventing and treating cardiovascular diseases such as hyperlipidemia, atherosclerosis, and atherosclerotic stroke, alone or in a complex agent together with an agonist of the existing nucleus receptor LXR, by inhibiting activity and generation of pro-inflammatory cytokine as an atherosclerosis factor and inhibiting the formation of NO that controls vasoconstriction. Moreover, the sesterterpene compounds of the present invention may be used as an effective component of a composition for preventing and treating central nervous system diseases such as Parkinson's disease, schizophrenia, and manic-depression, by increasing expression and activity of genes that regulate the function of dopaminergic neuronal cells. Moreover, the sesterterpene compounds of the present invention may be used as an agent for preventing and treating Alzheimer's disease in a complex agent together with agonists of the existing nuclear receptor LXR. Therefore, the sesterterpene compounds developed through the present invention may be used as an effective component of a composition for preventing and treating insulin-independent diabetes mellitus, fatty liver diseases (alcoholic, non-alcoholic, and viral), obesity, cardiovascular diseases (hyperlipidemia and atherosclerosis), and brain disorders (Parkinson's disease and Alzheimer's disease).

### [Background Art]

Changes in modern man lifestyle, such as nutrient excesses and exercise reduction, have resulted in rapid increases in metabolic diseases such as diabetes, fatty acid, obesity, hyperlipidemia, and atherosclerosis, and central nervous system disorders such as Parkinson's disease and Alzheimer's disease.

Diabetes alone is a major life-threatening disease, and is the direct cause of secondary diseases caused by diabetes, that is, blindness due to chronic complications, end-stage renal failure, neurological disease, and foot ulceration. Of these, the most threatening diseases are cerebrovascular diseases and cardiovascular diseases. It has been known that patients with diabetes are 2 times more likely at risk of getting coronary artery disease and about 3 times more likely at risk of getting peripheral vascular diseases as compared with a normal person. It has been that the reasons are hyperglycemia, dyslipidemia, hyperinsulinemia, hypertension, and changes in the blood clotting mechanism.

Nuclear receptors are ligand-dependent transcription factors to regulate physiological functions such as in vivo metabolism, cell differentiation and apoptosis, and development. Of these, NR4A is considered an orphan nuclear receptor since it does not have a binding region with the co-activator and the accurate ligands thereof have been currently established (Wang et al., Nature, 2003, 423, 555-560). Accordingly, the physiological functions of NR4A have been currently found mainly through studies on overexpression of this gene (gain of function) or selective removal of this gene (loss of function). However, since the recent studies reveal that NR4A also is a ligand-dependent transcription factor, NR4A has emerged as a new medicine molecular target for metabolic disease treatment (Zhan et al., Nat. Chem. Biol., 2008, 4, 548-556).

NR4A reduces expression of sterol regulatory element binding protein-1c (SREBP1C) regulating fatty acid synthesis in the liver (Pei et al., Nat. Med., 2006, 12, 1048-1055; Pols TW et. al., Biochem. Biophys. Res. Commun., 2008, 366, 910-916). It increases the expression of glucose transporter associated with glucose absorption and activates the insulin signaling, resulting in increasing the glucose metabolism, in the muscle and liver (Fu et al., J. Biol. Chem., 2007, 282, 31525-31533). In addition, it increases expressions of uncoupling proteins (UCPs) regulating thermogenesis and PGC-1α and ß, lipoprotein lipase (LPL), fatty acid binding protein 4 (FABP4), pyruvate dehydrogenase kinase, and isozyme 4 (PDK4), which regulate fatty acid oxidation, to increase fat metabolism (Pearen, MA et al., Endocrinology, 2006, 147, 5217-5227; Weyrich, P et al., Diabetes, 2009, 58, 2788-2796; Pearen, MA et al., Endocrinology, 2007, 149, 2853-2865; Chao, LC et al., Mol. Endocrinol., 2007, 21, 2152-2163). Therefore, NR4A is developed to have superior efficacy, which can be used as a therapeutic agent for diabetes, fatty liver, and obesity.

As a result of experiment using NR4A in the macrophage, expression of the pro-inflammatory cytokine by LPS or ox-LDL was inhibited by NR4A (Peter I. Bonta et al., Arterioscler. Thromb. Vasc. Biol., 2006, 26, 2288-2294). In addition, NR4A inhibited the proliferation of smooth muscle cells by increasing expression of p27^{Kip1}, cell cycle inhibitor protein, in the vascular smooth muscle and inhibited the occurrence of atherosclerosis by reducing expression of pro-inflammatory cytokines such as IL-1ß, TNFα, and MCP-1 (Peter I. Bonta et al., Circulation, 2010, 121, 2023-2032). Therefore, NR4A is developed to have superior efficacy, which can be used as a therapeutic agent for atherosclerosis.

LXR agonists reduce serum low-density lipoprotein (LDL) cholesterol and increase high-density lipoprotein (HDL) cholesterol and thus can be developed as a therapeutic agent for hyperlipidemia, and reduce expression of cytokine causing an inflammatory reaction to inhibit atherosclerosis (Dai, Inflammation, 2007, 30, 105-117). In addition, they remove beta amyloid, which is a cause of Alzheimer's disease in the brain, to thereby improve Alzheimer's disease (Riddell, Mol Cell Neuroscie, 2007, 34, 621-628). However, even though the existing LXR agonists have efficacy to treat hyperlipidemia, atherosclerosis, and Alzheimer's disease, they cause fatty acid, resulting in severe hepatotoxicity (Barunowski, J Physiol. Pharmacol., 2008, 59, 31-55; Chisholm, J. Lipid Res. 2003, 44, 2039-2048). The LXR agonist increase lipid synthesis and lipid peroxidation in the liver, to thereby cause alcoholic, non-alcoholic, and viral fatty liver diseases (Hwahng et al, Hepatology, 2009, 49, 1913-1925; Na et al., Hepatology. 2009, 1122-1131). Moreover, the lipid peroxidation by the LXR agonist inhibits functions of mitochondria to accelerate the occurrence of diabetes (Chu, Mol Cell Biol., 2006, 26, 6786-6798; Liang, J Biol. Chem., 2002, 277, 9520-9528; Barunowski, J Physiol Pharmacol., 2008, 59, 31-55). That is, since the LXR agonist that have been developed until the present time cause complications such as diabetes or fatty liver in the liver, the LXR agonist in the form of a single agent thereof is not appropriate in being used as a therapeutic agent for hyperlipidemia, atherosclerosis, and Alzheimer's disease. Therefore, the compound inhibiting a side effect (fatty liver) of the existing LXR agonist may be used as an effective component of a composition for preventing and treating diseases such as hyperlipidemia, atherosclerosis, and Alzheimer's disease, in a complex agent together with the developed LXR agonists. Accordingly, the present compound may be used as an effective component of a composition for preventing and treating hyperlipidemia, atherosclerosis, and Alzheimer's disease, in a complex agent together with the existing LXR agonists.

Central nervous system disorders are closely associated with death of dopaminergic neuronal cells. Nurr1, which is one kind of NR4A, is a nuclear receptor protein mainly expressed in the dopaminergic neuronal cell in the midbrain, and directly regulates the expression of genes associated with dopamine synthesis (tyrosine hydroxylase) and dopamine transport (dopamine transporter (Sakurada et al., Development 1999, 40174026; Sacchetti et al., J. Neurochem. 2001, 15651572). It was found from the result of analysis of family history of Parkinson's syndrome that mutation of the Nurr1 gene causes Parkinson's syndrome (Le et al., Nat Genet., 2002, 33, 85-89). In addition, it was proven that the Nurr1-deficient animal model confirmed the deficient of dopaminergic neuronal cells, and thus the Nurr1 gene is pathologically important in nervous diseases such as Parkinson's syndrome and the like (Joseph et al., Proc. Natl. Acad. Sci. U.S.A., 2002, 100, 15619-15624). Therefore, a Nurr1-activating material may be used as an effective component of a composition for preventing and treating central nervous system disorders such as Parkinson's disease, schizophrenia, and manic-depression.

The present inventors developed new sesterterpene compounds as an NR4A agonist, for preventing and treating insulin-independent diabetes, obesity, fatty liver disease, cardiovascular diseases (hyperlipidemia and atherosclerosis), and brain disorders (Parkinson's disease, schizophrenia, and manic-depression), and then completed the present invention. Further, the present inventors developed new sesterterpene compounds of selectively inhibiting activity of the LXR protein in the liver in order to overcome a side effect of the existing LXR activating ligand (fatty liver), and then completed the present invention. Further, the present inventors verified efficacy of the sesterterpene compounds to prevent and treat diabetes, vascular diseases, and brain disorders (Parkinson's disease) in disease animal models, and then completed the present invention.

### [Disclosure]

### [Technical Problem]

The present invention has the following objects.

A first object of the present invention is to provide a compound having superior efficacy to prevent, treat, and improve diabetes and diabetes complications (foot ulcer and renal failure).

A second object of the present invention is to provide a compound having superior efficacy to prevent, treat, and improve vascular diseases such as hyperlipidemia, atherosclerosis, and atherosclerotic stroke.

A third object of the present invention is to provide a compound having superior efficacy to prevent, treat, and improve alcoholic, non-alcoholic, and viral fatty liver diseases.

A fourth object of the present invention is to provide a compound having superior efficacy to prevent, treat, and improve obesity by inhibiting the differentiation of adipocytes.

A fifth object of the present invention is to provide a compound having superior efficacy to prevent, treat, and improve central nervous system disorders such as Parkinson's disease, schizophrenia, and manic-depression.

A sixth object of the present invention is to provide a compound having superior activity to Nurr1.

A seventh object of the present invention is to provide a compound having superior activity to LXR.

An eighth object of the present invention is to provide a pharmaceutical composition for preventing and treating diabetes and diabetes complications (foot ulcer and renal failure), vascular diseases (hyperlipidemia, atherosclerosis, and atherosclerotic stroke), fatty liver disease, obesity, or central nervous system disorders (Parkinson's disease, schizophrenia, and manic-depression), containing a compound of Chemical Formula I below as an effective component.

A ninth object of the present invention is to provide a pharmaceutical composition for preventing and treating vascular diseases such as hyperlipidemia, atherosclerosis, and atherosclerotic stroke, containing a compound of Chemical Formula I below as an effective, in a complex agent together with the existing LXR agonists.

A tenth object of the present invention is to provide a pharmaceutical composition for preventing and treating Alzheimer's disease, containing a compound of Chemical Formula I below as an effective component, in a complex agent together with the existing LXR agonists.

An eleventh object of the present invention is to provide a composition for functional food and functional beverage for preventing and improving diabetes and diabetes complications (foot ulcer and renal failure), vascular diseases (hyperlipidemia, atherosclerosis, and atherosclerotic stroke), fatty liver disease, obesity, or central nervous system disorders (Parkinson's disease, schizophrenia, and manic-depression), containing a compound of Chemical Formula I below as an effective component.

A twelfth object of the present invention is to provide a composition for functional food and functional beverage for preventing and improving vascular diseases such as hyperlipidemia, atherosclerosis, and atherosclerotic stroke, containing a compound of Chemical Formula I below as an effective component, in a complex agent together with the existing LXR agonists.

A thirteenth object of the present invention is to provide a composition for functional food and functional beverage for preventing and improving Alzheimer's disease, containing a compound of Chemical Formula I below as an effective component, in a complex agent together with the existing LXR agonists.

A fourteenth object of the present invention is to provide a composition for functional cosmetics for preventing and improving obesity, containing a compound of Chemical Formula I below as an effective component.

A fifth object of the present invention is to provide a composition for functional feedstuff for preventing and improving diabetes and diabetes complications (foot ulcer and renal failure), vascular diseases (hyperlipidemia, atherosclerosis, and atherosclerotic stroke), fatty liver disease, obesity, or central nervous system disorders (Parkinson's disease, schizophrenia, and manic-depression), containing a compound of Chemical Formula I below as an effective component.

A sixteenth object of the present invention is to provide a composition for functional feedstuff for preventing and improving vascular diseases such as hyperlipidemia, atherosclerosis, and atherosclerotic stroke, containing a compound of Chemical Formula I below as an effective component, in a complex agent together with the existing LXR agonists.

A seventeenth object of the present invention is to provide a composition for functional feedstuff for preventing and improving Alzheimer's disease, containing a compound of Chemical Formula I below as an effective component, in a complex agent together with the existing LXR agonists.

An eighteenth object of the present invention is to provide a pharmaceutical composition for preventing and treating diseases through Nurr1 activation.

A nineteenth object of the present invention is to provide a pharmaceutical composition for preventing and treating diseases through LXR inhibitory activity.

A twentieth object of the present invention is to provide a composition for functional food and function beverage for preventing and improving diseases through Nurr1 activation.

A twenty-first object of the present invention is to provide a composition for functional food and functional beverage for preventing and treating diseases through LXR inhibitory activity.

### [Technical Solution]

The present invention is directed to a noble sesterterpene compound, a stereoisomer thereof, an enantiomer thereof, an in vivo-hydrolysable precursor thereof, or a pharmaceutically acceptable salt thereof, and uses of these, and the sesterterpene compound provides superior activity to prevent and treat diabetes, foot ulcer and renal failure due to diabetes, alcoholic, non-alcoholic, and viral fatty liver diseases, obesity, vascular diseases such as hyperlipidemia, atherosclerosis, and atherosclerotic stroke, or central nervous system disorders such as Parkinson's disease, Alzheimer's disease, schizophrenia, and manic-depression.

The sesterterpene compound of the present invention is represented by Chemical Formula I below:

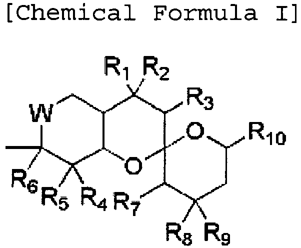

[in Chemical Formula I,

W is C(=A) or CR₁₁R₁₂, A is O, S, or NR₁₃, and R₁₃ is hydrogen, OH, (C1-C8)alkyl, (C2-C8)alkenyl, (C2-C8)alkynyl, (C3-C8)cycloalkyl, (C6-C20)aryl or (C4-C20)heteroaryl;

R₁₁ and R₁₂ each are independently hydrogen, -L-R₁₄, halogen, -N₃, (C2-C20)heteroaryl, (C6-C20)aryl, -NR₁₅R₁₆, 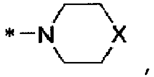 (C2-C8)alkenyl, or (C2-C8)alkynyl, but both R₁₁ and R₁₂ are not hydrogen;

L is O, NR₁₃, S, SO₂, or Se;

R₁₄ is hydrogen, (C1-C8)alkyl, (C1-C8)alkylcarbonyl, (C6-C20)aryl, -SO₂R₁₇, -(CH₂)ₘ-R₁₈, (C2-C8)alkenyl, (C2-C8)alkynyl, (C3-C8)cycloalkyl, (C4-C20)heteroaryl, amino acid, glucose, or

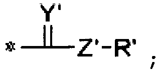

R₁₇ is (C6-C20)aryl, (C1-C8)alkyl, (C2-C8)alkenyl, (C2-C8)alkynyl, (C3-C8)cycloalkyl, (C4-C20)heteroaryl, amino acid, glucose, or

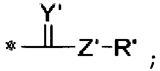

R₁₅ and R₁₆ each are independently hydrogen, (C1-C8)alkyl, (CH₂)ₘ-R₁₈, (C2-C8)alkenyl, (C2-C8)alkynyl, (C3-C8)cycloalkyl, (C6-C20)aryl, (C4-C20)heteroaryl, amino acid, glucose, or

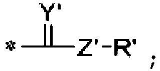

R₁₈ is (C2-C8)alkenyl, (C2-C8)alkynyl, -NR₁₉R₂₀, or (C2-C20)heteroaryl;

X is a single bond, CR₂₀, O, S, or NR₂₀;

R₁₉ and R₂₀ each are independently hydrogen, (C1-C8)alkyl, (C6-C20)aryl, (C2-C8)alkenyl, (C2-C8)alkynyl, (C3-C8)cycloalkyl, (C4-C20)heteroaryl, amino acid, or

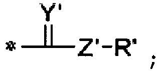

R₁ is -CH₂R₂₁, -COOH, -C(=O)R₂₂, (C2-C20)heteroaryl,

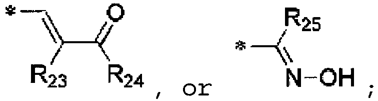

R₂₁ is -OR₂₆, N₃, -NR₁₅R₁₆, halogen, CN, NO₂, (C2-C20)heteroaryl, 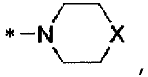 -SR₂₀, -SO₂R₁₇, -SeR₂₀, glucose, or amino acid;

R₂₂ to R₂₅ each are independently hydrogen, CN, halogen, (C1-C8)alkyl, OR₁₄, 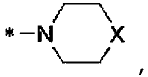 or NR₁₅R₁₆;

R₂₆ is hydrogen, (C1-C8)alkyl, (C6-C20)aryl, (C1-C8)alkyldi(C6-C20)arylacetyl, halo(C1-C8)alkyldi(C6-C20)arylacetyl, (C1-C8)alkyldi(C6-C20)arylsilyloxy, - (CH₂)mR₂₇, -C(O)R₂₇, -S(=O)₂R₂₈, -P(=O)(R₂₈)₂, (C2-C8)alkenyl, (C2-C8)alkynyl, (C3-C8)cycloalkyl, (C4-C20)heteroaryl, amino acid,

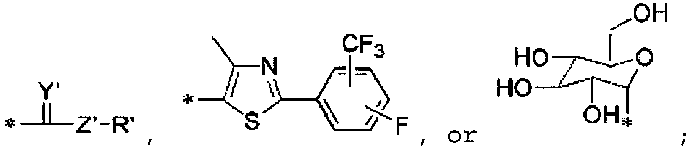

Y' is O, S, or NR"; Z' is a single bond, NH, O, S, or Se; R' and R" each are independently hydrogen, (C1-C8)alkyl, (C2-C8)alkenyl, (C2-C8)alkynyl, (C3-C8)cycloalkyl, (C6-C20)aryl, or (C4-C20)heteroary;

R₂₇ is (C1-C8)alkyl, (C2-C8)alkenyl, (C2-C8)alkynyl, (C6-C20)aryl, (C2-C20)heteroaryl, 5- to 6- membered heterocycloalkyl, or

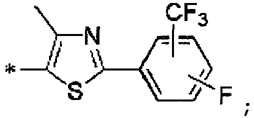

R₂₈ is (C1-C8)alkyl, (C6-C20)aryl, (C1-C8)alkoxy, (C6-C20)aryloxy, (C2-C20)heteroaryl, OH, or OM;

M is alkali metal;

R₄ is hydrogen, (C1-C8)alkyl, or -L-R₂₉; R₂₉ is hydrogen, (C1-C8)alkyl, (C1-C8)alkylcarbonyl, (C6-C20)aryl, or -SO₂R₁₇;

R₆ is hydrogen, (C1-C8)alkyl, or OH;

R₂, R₃, R₅, R₇ and R₈ each are independently hydrogen, (C1-C8)alkyl, (C6-C20)aryl, or (C2-C20)heteroaryl, and R₂ and R₃, R₅ and R₆, and R₇ and R₈ are independently linked to each other to form a double bond, linked via oxygen (O) to form epoxide, or linked via sulfur (S) to form thiirane;

R₉ is (C1-C8)alkyl, CHO, or COOH, and R₉ may form a double bond together with R₈;

R₁₀ is -(CH₂)ₘR₃₀, CHO, COOH,

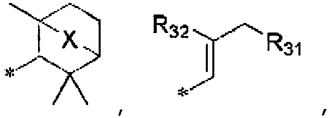

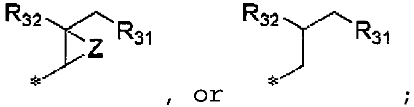

R₃₀ is hydrogen, halogen, OH, -NR₁₅R₁₆, SH, or SeH;

R₃₁ is (C1-C8)alkyl,

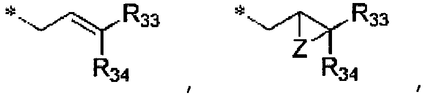

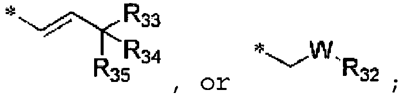

R₃₂ to R₃₅ each are independently hydrogen, (C1-C8) alkyl, (C6-C20) aryl, -OH, -SH, -NR₁₅R₁₆, -O-OH, amino acid, glucose, or

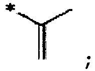

Z is O or S;

m is an integer of 1 to 5;

the heteroaryl of R₁ and R₂₁, the alkyl, aryl, and heteroaryl of R₂, R₃, R₅, R₇ and R₈, the alkyl of R₄, R₆, R₉, R₂₂ to R₂₅ and R₃₁, the heteroaryl, aryl, alkenyl, and alkynyl of R₁₁ and R₁₂, the alkyl, alkenyl, alkynyl, cycloalkyl, aryl, heteroaryl of R₁₃, R₁₅, R₆₁, R₁₉, R₂₀, R' and R", the alkyl, alkylcarbonyl, aryl, alkenyl, alkynyl, cycloalkyl, and heteroaryl of R₁₄, the aryl and alkyl of R₁₇ and R₃₂ to R₃₅, the alkenyl, alkynyl, and heteroaryl of R₁₈, the alkyl, aryl, alkyldiarylsilyloxy, alkenyl, alkynyl, cycloalkyl, heteroaryl of R₂₆, the alkyl, alkenyl, alkynyl, aryl, heteroaryl, heterocycloalkyl of R₂₇, the alkyl, aryl, alkoxy, aryloxy, and heteroaryl of R₂₈, the alkyl, alkylcarbonyl, and aryl of R₂₉ each may be further substituted with at least one substituent selected from a group consisting of (C1-C8)alkyl, halogen, (C6-C20)aryl, (C6-C20)ar(C1-C8)alkyl, halo(C1-C8)alkyl, cyano, nitro, (C1-C8)alkoxy, (C6-C20)aryloxy, (C1-C8)alkylthio, (C6-C20)arylthio, amino, mono- or di- (C1-C8)alkylamino, mono- or di- (C6-C20)arylamino, (C1-C8)alkyl(C6-C20)arylamino, (C1-C8)alkylcarbonyl, (C6-C20)arylcarbonyl, (C1-C8)alkoxycarbonyl, (C6-C20)aryloxycarbonyl, (C2-C20)heteroaryl, hydroxy, formyl, and carboxyl; and

the heteroaryl and heterocycloalkyl contains at least one hetero atom selected from N, O, and S.]

Specific examples of the compound of Chemical Formula I are as follows:

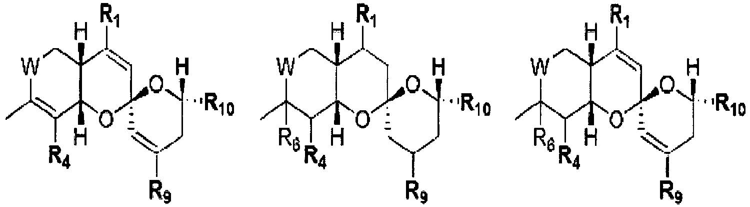

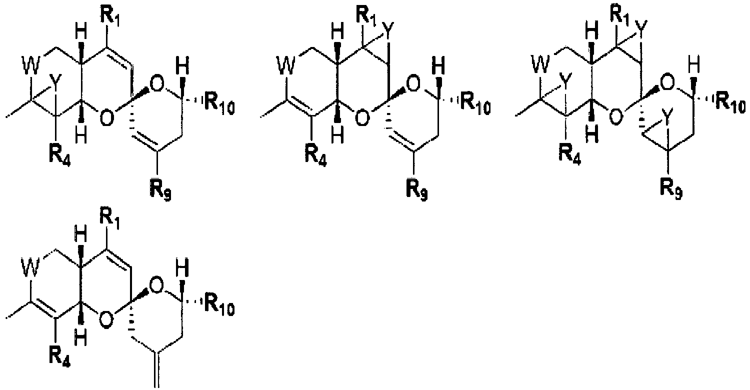

[Y is O or S;

W is

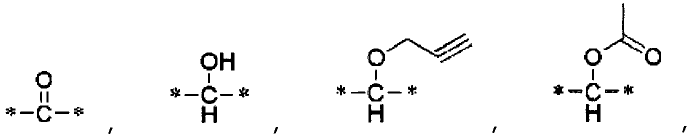

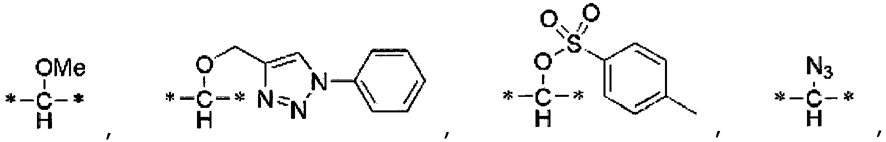

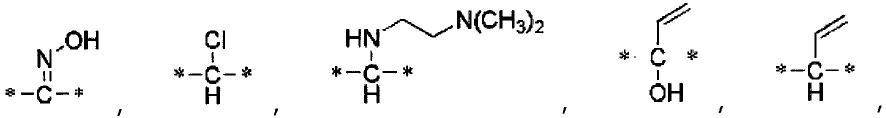

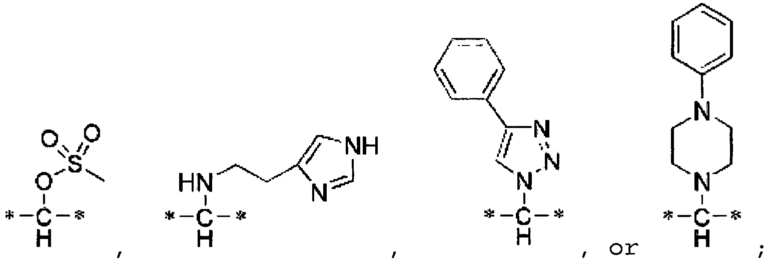

R₁ is

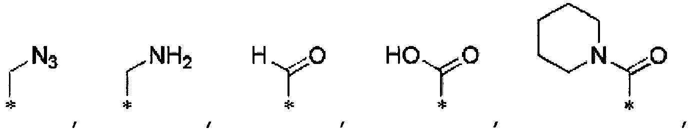

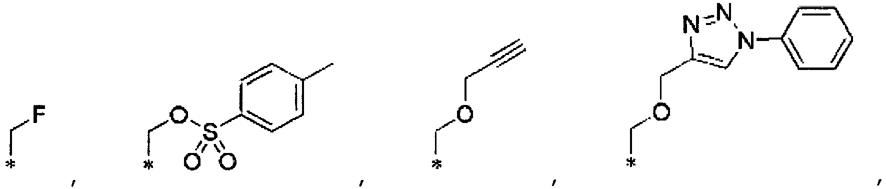

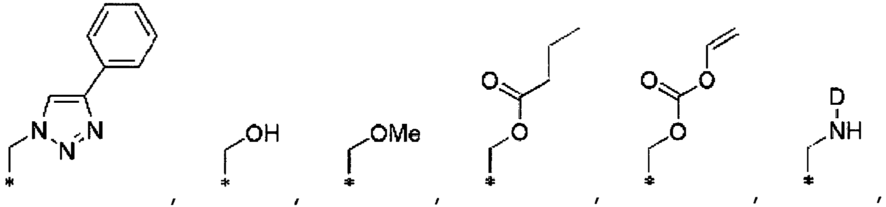

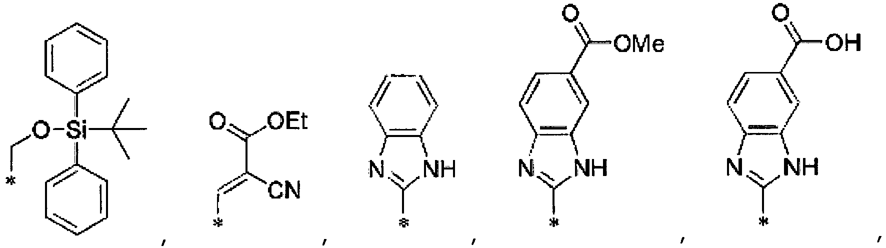

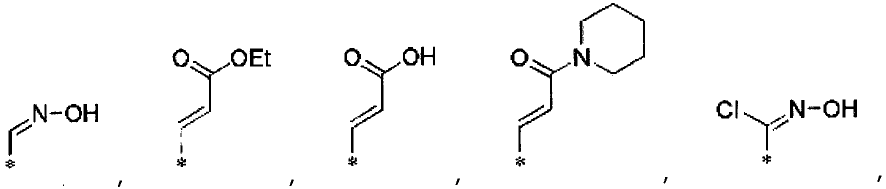

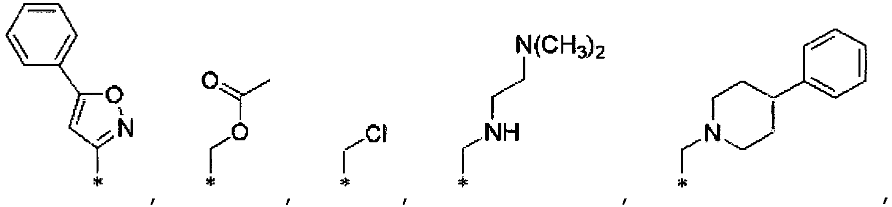

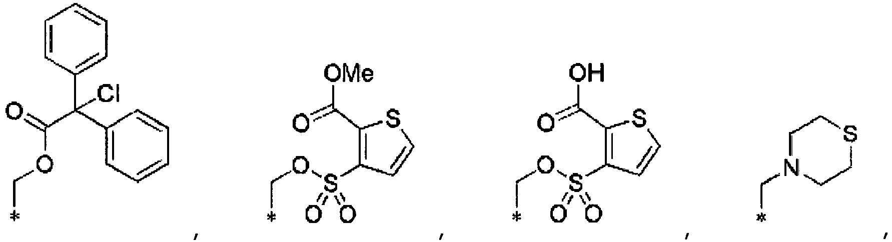

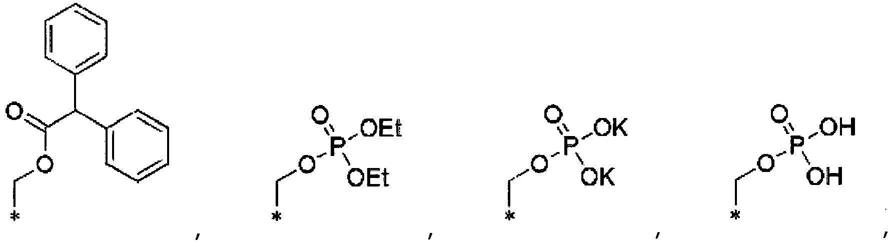

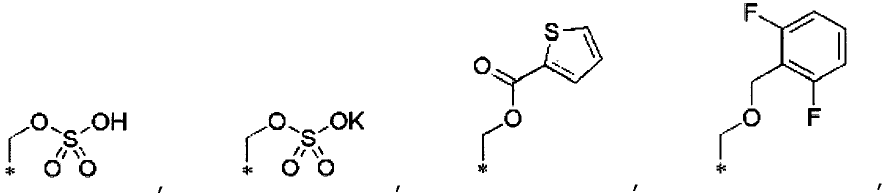

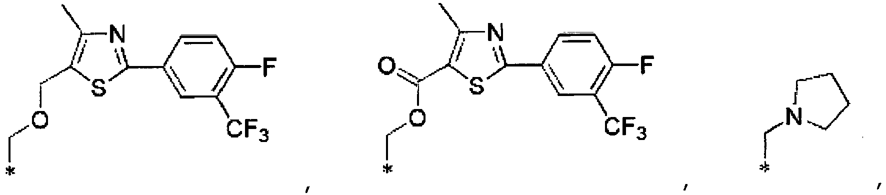

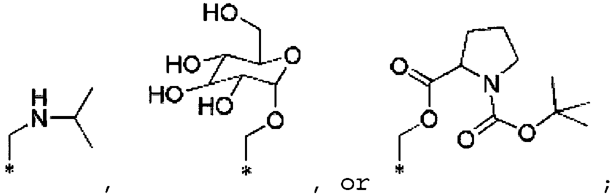

D is amino acid;

R₄ is H, CH₃, OH, SH, SeH, OTs, OMe, OAc, C(CN)₂, or

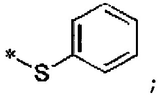

R₆ is H or

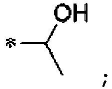

R₉ is CH₃, CH₂OH, CHO, or COOH;

R₁₀ is CH₂CH₃, CHO, COOH, CH₂Cl, CH₂F, CH₂NH₂, CH₂OH, CH₂SH, CH₂SeH,

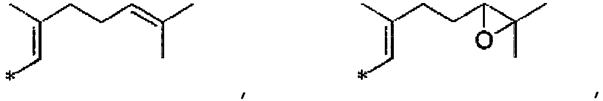

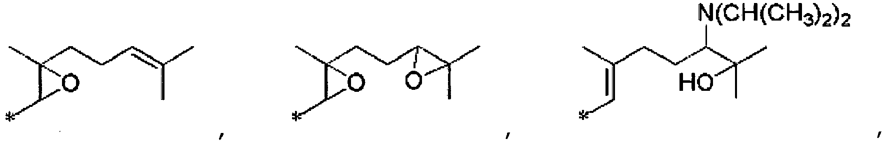

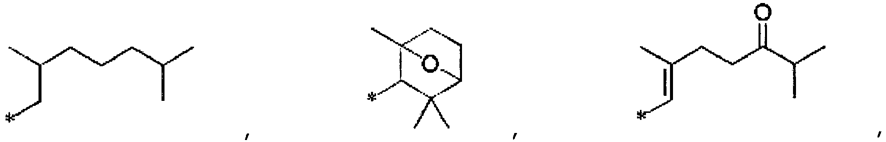

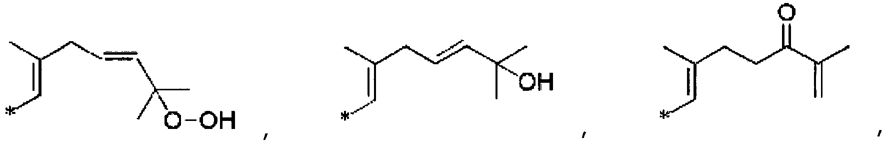

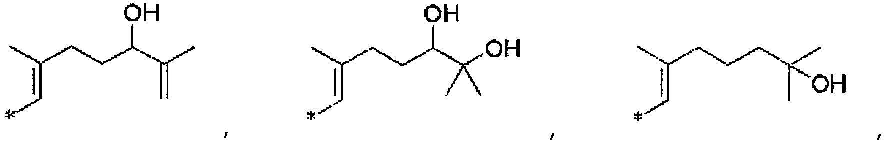

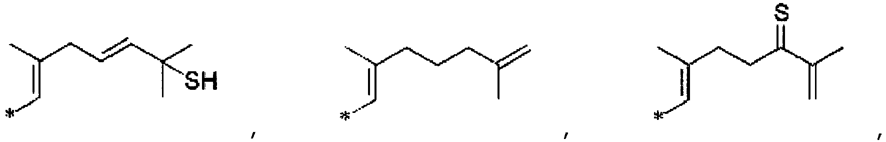

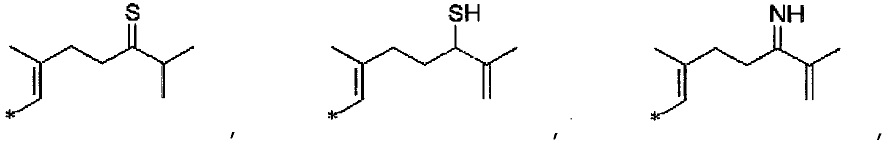

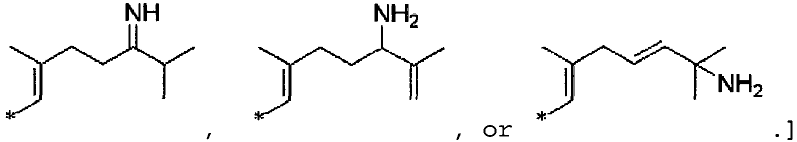

The sesterterpene compound of Chemical Formula I may be represented by Chemical Formula II below, and the present invention provides a novel sesterterpene compound of Chemical Formula II below:

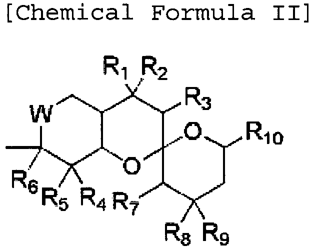

[In Chemical Formula II,

W is C(=A) or CR₁₁R₁₂, A is O, S, or NR₁₃, and R₁₃ is hydrogen, OH, (C1-C8)alkyl, (C2-C8)alkenyl, (C2-C8)alkynyl, (C3-C8)cycloalkyl, (C6-C20)aryl or (C4-C20)heteroaryl;

R₁₁ and R₁₂ each are independently hydrogen, -L-R₁₄, halogen, -N₃, (C2-C20)heteroaryl, (C6-C20)aryl, -NR₁₅R₁₆, 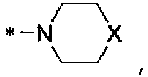 (C2-C8)alkenyl, or (C2-C8)alkynyl, but both R₁₁ and R₁₂ are not hydrogen;

L is O, NR₁₃, S, SO₂, or Se;

R₁₄ is hydrogen, (C1-C8)alkyl, (C1-C8)alkylcarbonyl, (C6-C20)aryl, -SO₂R₁₇, (C2-C8)alkenyl, (C2-C8)alkynyl, (C3-C8)cycloalkyl, (C4-C20)heteroaryl, amino acid, glucose, or - (CH₂)ₘ-R₁₈; and R₁₇ is (C6-C20)aryl, (C2-C8)alkenyl, (C2-C8)alkynyl, (C3-C8)cycloalkyl, (C4-C20)heteroaryl, amino acid, glucose, or (C1-C8)alkyl;

R₁₅ and R₁₆ each are independently hydrogen, (C1-C8)alkyl, (C2-C8)alkenyl, (C2-C8)alkynyl, (C3-C8)cycloalkyl, (C6-C20)aryl, (C4-C20)heteroaryl, amino acid, glucose, or - (CH₂)ₘ-R₁₈;

R₁₈ is (C2-C8)alkenyl, (C2-C8)alkynyl, -NR₁₉R₂₀, or (C2-C20)heteroaryl;

X is a single bond, CR₂₀, O, S, or NR₂₀; R₁₉ and R₂₀ each are independently hydrogen, (C1-C8)alkyl, (C2-C8)alkenyl, (C2-C8)alkynyl, (C3-C8)cycloalkyl, (C4-C20)heteroaryl, amino acid, or (C6-C20)aryl;

R₁ is -CH₂R₂₁, -COOH, -C(=O)R₂₂, (C2-C20)heteroaryl,

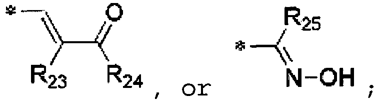

R₂₁ is -OR₂₆, N₃, -NR₁₅R₁₆, halogen, CN, NO₂, (C2-C20)heteroaryl, 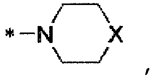 -SO₂R₁₇, -SeR₂₀, glucose, amino acid, or -SR₂₀;

R₂₂ to R₂₅ each are independently hydrogen, CN, halogen, (C1-C8)alkyl, OR₁₄, 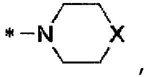 or -NR₁₅R₁₆;

R₂₆ is hydrogen, (C1-C8)alkyl, (C6-C20)aryl, (C1-C8)alkyldi(C6-C20)arylacetyl, halo(C1-C8)alkyldi(C6-C20)arylacetyl, (C1-C8)alkyldi(C6-C20)arylsilyloxy, - (CH₂)ₘR₂₇, -C(O)R₂₇, -S(=O)₂R₂₈, -P(=O)(R₂₈)₂, (C2-C8)alkenyl, (C2-C8)alkynyl, (C3-C8)cycloalkyl, (C4-C20)heteroaryl, amino acid,

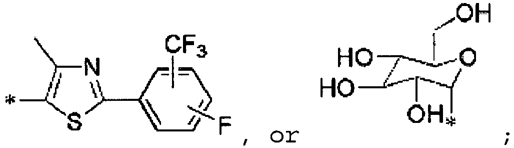

R₂₇ is (C1-C8)alkyl, (C2-C8)alkenyl, (C2-C8)alkynyl, (C6-C20)aryl, (C2-C20)heteroaryl, 5- to 6- membered heterocycloalkyl, or

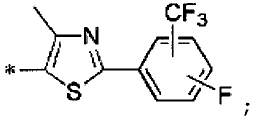

R₂₈ is (C1-C8)alkyl, (C6-C20)aryl, (C1-C8)alkoxy, (C6-C20)aryloxy, (C2-C20)heteroaryl, OH, or OM;

M is alkali metal;

R₄ is hydrogen, (C1-C8)alkyl, or -L-R₂₉; R₂₉ is hydrogen, (C1-C8)alkyl, (C1-C8)alkylcarbonyl, (C6-C20)aryl, or -SO₂R₁₇;

R₆ is hydrogen, (C1-C8)alkyl, or OH;

R₂, R₃, R₅, R₇ and R₈ each are independently hydrogen, (C1-C8)alkyl, (C6-C20)aryl, or (C2-C20)heteroaryl, and R₂ and R₃, R₅ and R₆, and R₇ and R₈ are independently linked to each other to form a double bond, linked via oxygen (O) to form epoxide, or linked via sulfur (S) to form thiirane;

R₉ is (C1-C8)alkyl, CHO, or COOH;

R₁₀ is -(CH₂)ₘR₃₀, CHO, COOH,

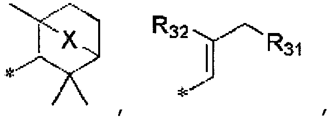

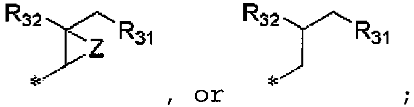

R₃₀ is hydrogen, halogen, OH, -NR₁₅R₁₆, SH, or SeH;

R₃₁ is (C1-C8)alkyl,

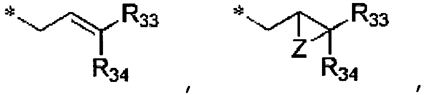

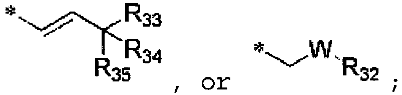

R₃₂ to R₃₅ each are independently hydrogen, (C1-C8) alkyl, (C6-C20)aryl, -OH, -SH, -NR₁₅R₁₆. amino acid, glucose, -O-OH, or

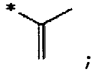

Z is O or S;

m is an integer of 1 to 5;

the heteroaryl of R₁, R₁₈, and R₂₁, the alkyl, aryl, and heteroaryl of R₂, R₃, R₅, R₇, R₈ and R₂₈, the alkyl of R₄, R₆, R₉, R₁₅, R₁₆, R₂₂ to R₂₅ and R₃₁, the heteroaryl and aryl of R₁₁ and R₁₂, the alkyl and aryl of R₁₄, R₁₇, R₁₉, R₂₀, R₂₆, R₂₉, and R₃₂ to R₃₅, and the alkyl, aryl, heteroaryl, and heterocycloalkyl of R₂₇ each may be further substituted with at least one substituent selected from a group consisting of (C1-C8)alkyl, halogen, (C6-C20)aryl, (C6-C20)ar(C1-C8)alkyl, halo(C1-C8)alkyl, cyano, nitro, (C1-C8)alkoxy, (C6-C20)aryloxy, (C1-C8)alkylthio, (C6-C20)arylthio, amino, mono- or di- (C1-C8)alkylamino, mono- or di- (C6-C20)arylamino, (C1-C8)alkyl(C6-C20)arylamino, (C1-C8)alkylcarbonyl, (C6-C20)arylcarbonyl, (C1-C8)alkoxycarbonyl, (C6-C20)aryloxycarbonyl, (C2-C20)heteroaryl, hydroxy, formyl, and carboxyl; and

the heteroaryl and heterocycloalkyl contains at least one hetero atom selected from N, O, and S.]

Specific examples of the compound of Chemical Formula II are as follows:

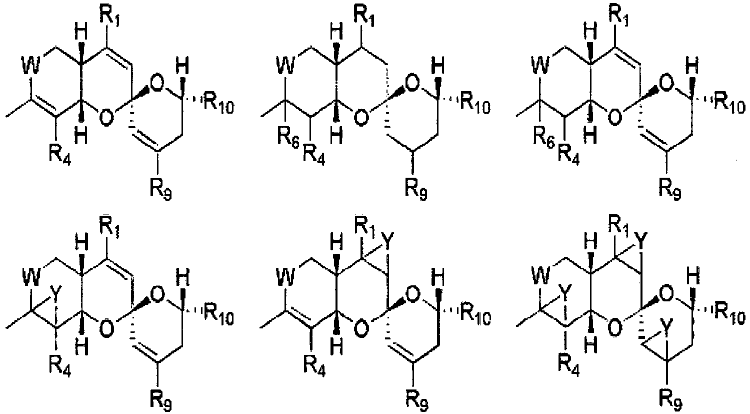

[Y is O or S;

W is

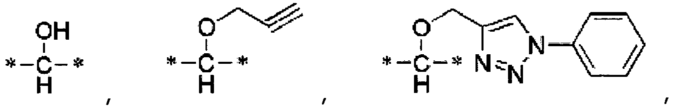

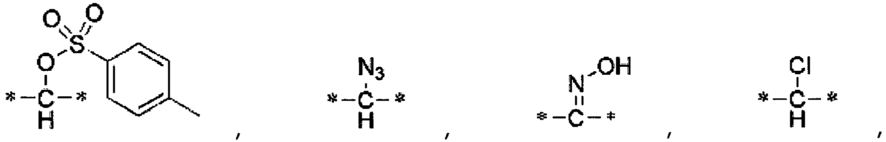

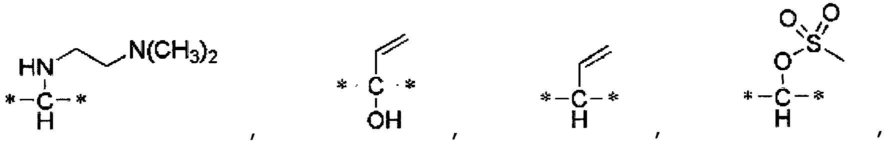

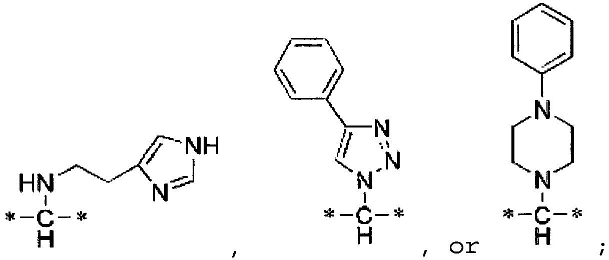

R₁ is

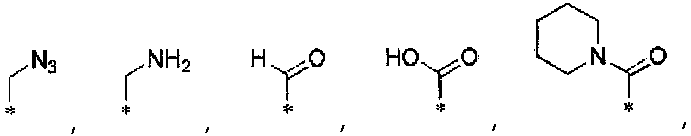

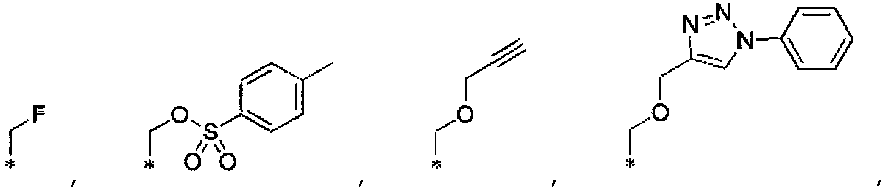

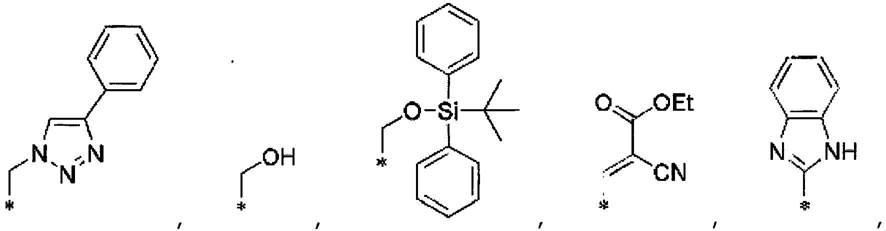

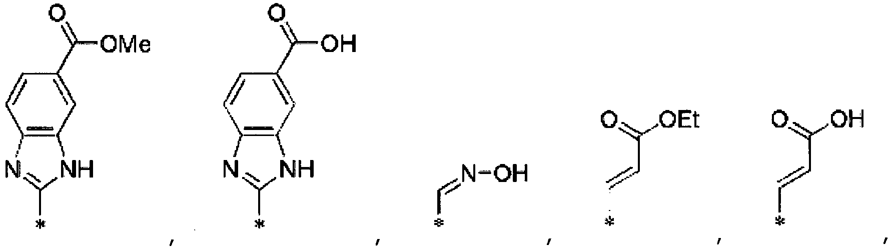

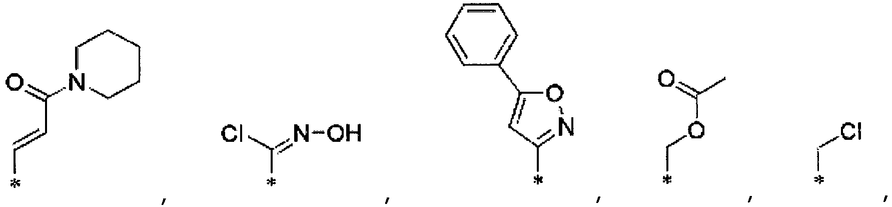

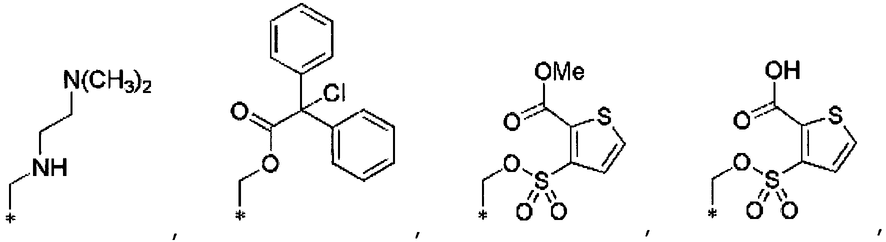

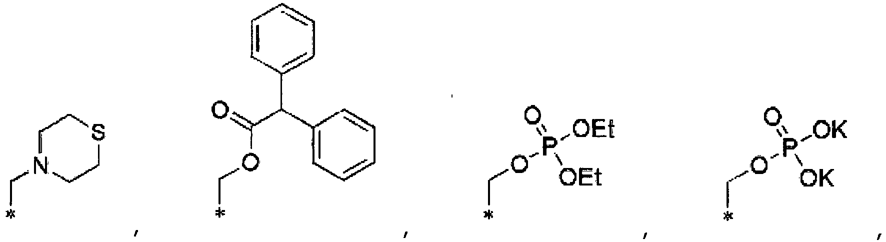

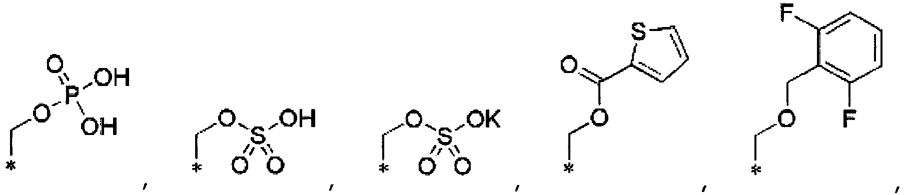

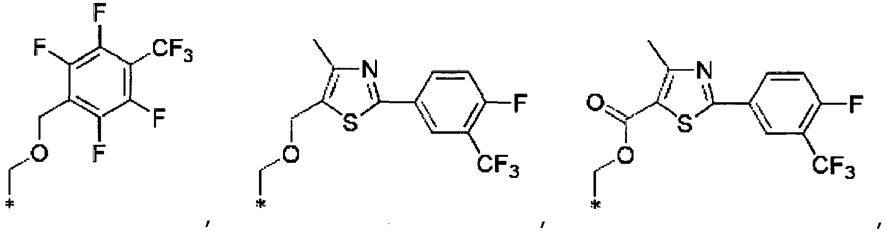

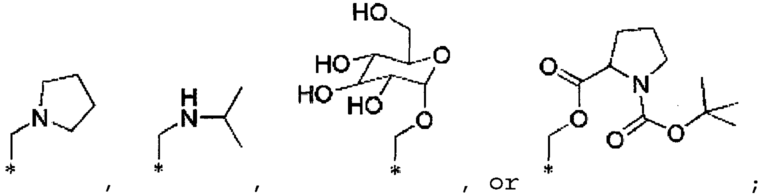

R₄ is H, CH₃, OH, SH, SeH, OTs, OMe, OAc, C(CN)₂, or

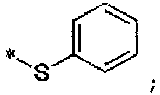

R₆ is H or

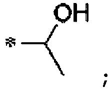

R₉ is CH₃, CH₂OH, CHO, or COOH;

R₁₀ is CH₂CH₃, CHO, COOH, CH₂Cl, CH₂F, CH₂NH₂, CH₂OH, CH₂SH, CH₂SeH,

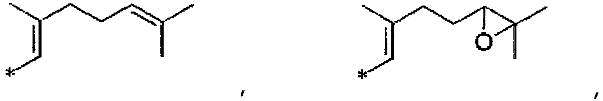

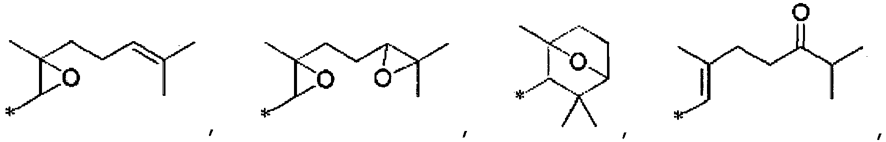

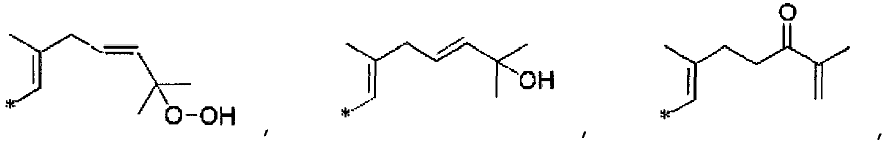

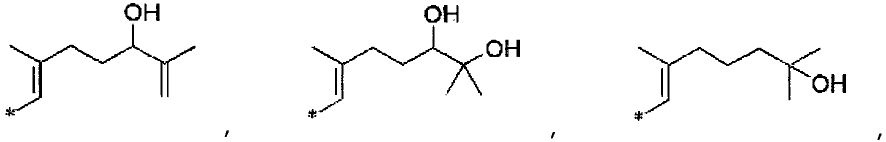

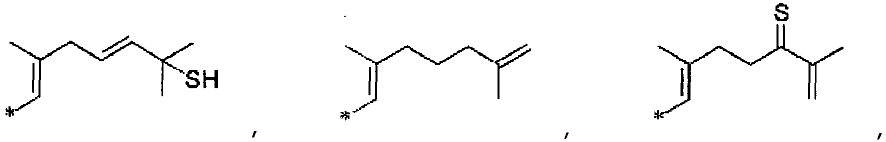

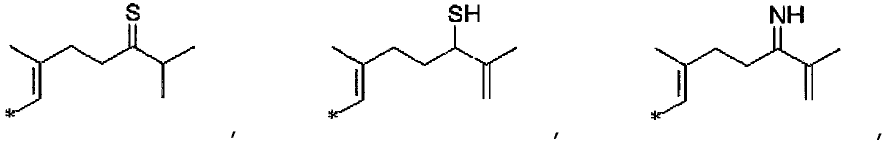

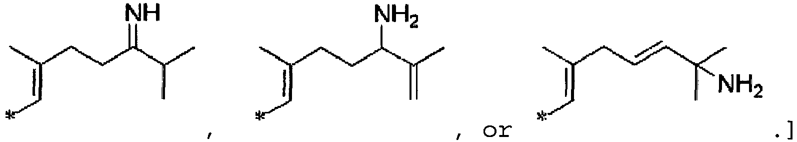

Further, the present invention provides a pharmaceutical composition for preventing and treating diabetes and diabetes complications (foot ulcer or renal failure), the pharmaceutical composition containing the compound of Chemical Formula I, a stereoisomer thereof, an enantiomer thereof, an in vivo-hydrolysable precursor thereof, or a pharmaceutically acceptable salt thereof, as a pharmaceutically acceptable carrier and an effective component.

Further, the present invention provides a pharmaceutical composition for preventing and treating vascular diseases such as hyperlipidemia, atherosclerosis, and atherosclerotic stroke, the pharmaceutical composition containing the compound of Chemical Formula I, a stereoisomer thereof, an enantiomer thereof, an in vivo-hydrolysable precursor thereof, or a pharmaceutically acceptable salt thereof, as a pharmaceutically acceptable carrier and an effective component.

Further, the present invention provides a pharmaceutical composition for preventing and treating alcoholic, non-alcoholic, and viral fatty liver diseases, the pharmaceutical composition containing the compound of Chemical Formula I, a stereoisomer thereof, an enantiomer thereof, an in vivo-hydrolysable precursor thereof, or a pharmaceutically acceptable salt thereof, as a pharmaceutically acceptable carrier and an effective component.

Further, the present invention provides a pharmaceutical composition for preventing and treating obesity, the pharmaceutical composition containing the compound of Chemical Formula I, a stereoisomer thereof, an enantiomer thereof, an in vivo-hydrolysable precursor thereof, or a pharmaceutically acceptable salt thereof, as a pharmaceutically acceptable carrier and an effective component.

Further, the present invention provides a pharmaceutical composition for preventing and treating central nervous system disorders such as Parkinson's disease, schizophrenia, and manic-depression, the pharmaceutical composition containing the compound of Chemical Formula I, a stereoisomer thereof, an enantiomer thereof, an in vivo-hydrolysable precursor thereof, or a pharmaceutically acceptable salt thereof, as a pharmaceutically acceptable carrier and an effective component.

Further, the present invention provides a pharmaceutical composition for preventing and treating vascular diseases such as hyperlipidemia, atherosclerosis, and atherosclerotic stroke, the pharmaceutical composition containing the compound of Chemical Formula I, a stereoisomer thereof, an enantiomer thereof, an in vivo-hydrolysable precursor thereof, or a pharmaceutically acceptable salt thereof, as a pharmaceutically acceptable carrier and an effective component, in a complex agent together with the existing LXR agonists.

Further, the present invention provides a pharmaceutical composition for preventing and treating Alzheimer's disease, the pharmaceutical composition containing the compound of Chemical Formula I, a stereoisomer thereof, an enantiomer thereof, an in vivo-hydrolysable precursor thereof, or a pharmaceutically acceptable salt thereof, as a pharmaceutically acceptable carrier and an effective component, in a complex agent together with the existing LXR agonists.

Further, the present invention provides a pharmaceutical composition for Nurr1 activation, the pharmaceutical composition containing the compound of Chemical Formula I, a stereoisomer thereof, an enantiomer thereof, an in vivo-hydrolysable precursor thereof, or a pharmaceutically acceptable salt thereof, as a pharmaceutically acceptable carrier and an effective component.

Further, the present invention provides a pharmaceutical composition for LXR inhibitory activity, the pharmaceutical composition containing the compound of Chemical Formula I, a stereoisomer thereof, an enantiomer thereof, an in vivo-hydrolysable precursor thereof, or a pharmaceutically acceptable salt thereof, as a pharmaceutically acceptable carrier and an effective component.

Further, the present invention provides a composition for functional food and beverage for preventing and improving diabetes and diabetes complications (foot ulcer or renal failure), the composition containing the compound of Chemical Formula I, a stereoisomer thereof, an enantiomer thereof, an in vivo-hydrolysable precursor thereof, or a salt thereof acceptable as a food additive.

Further, the present invention provides a composition for functional food and beverage for preventing and improving vascular diseases such as hyperlipidemia, atherosclerosis, and atherosclerotic stroke, the composition containing the compound of Chemical Formula I, a stereoisomer thereof, an enantiomer thereof, an in vivo-hydrolysable precursor thereof, or a salt thereof acceptable as a food additive.

Further, the present invention provides a composition for functional food and beverage for preventing and improving alcoholic, non-alcoholic, and viral fatty liver diseases, the composition containing the compound of Chemical Formula I, a stereoisomer thereof, an enantiomer thereof, an in vivo-hydrolysable precursor thereof, or a pharmaceutically acceptable salt thereof, as a pharmaceutically acceptable carrier and an effective component.

Further, the present invention provides a composition for functional food, beverage, and cosmetics for preventing and improving obesity, the composition containing the compound of Chemical Formula I, a stereoisomer thereof, an enantiomer thereof, an in vivo-hydrolysable precursor thereof, or a salt thereof acceptable as a food additive.

Further, the present invention provides a composition for functional food and beverage for preventing and improving central nervous system disorders such as Parkinson's disease, schizophrenia, and manic-depression, the composition containing the compound of Chemical Formula I, a stereoisomer thereof, an enantiomer thereof, an in vivo-hydrolysable precursor thereof, or a pharmaceutically acceptable salt thereof, as a pharmaceutically acceptable carrier and an effective component.

Further, the present invention provides a composition for functional food and beverage for preventing and improving vascular diseases such as hyperlipidemia, atherosclerosis, and atherosclerotic stroke, the composition containing the compound of Chemical Formula I, a stereoisomer thereof, an enantiomer thereof, an in vivo-hydrolysable precursor thereof, or a salt thereof acceptable as a food additive, and the existing LXR agonist.

Further, the present invention provides a composition for functional food and beverage for preventing and improving Alzheimer's disease, the composition containing the compound of Chemical Formula I, a stereoisomer thereof, an enantiomer thereof, an in vivo-hydrolysable precursor thereof, or a salt thereof acceptable as a food additive, and the existing LXR agonist.

Further, the present invention provides a composition for functional feedstuff for preventing and improving diabetes and diabetes complications (foot ulcer or renal failure), the composition containing the compound of Chemical Formula I, a stereoisomer thereof, an enantiomer thereof, an in vivo-hydrolysable precursor thereof, or a salt thereof acceptable as a food additive.

Further, the present invention provides a composition for functional feedstuff for preventing and improving vascular diseases such as hyperlipidemia, atherosclerosis, and atherosclerotic stroke, the composition containing the compound of Chemical Formula I, a stereoisomer thereof, an enantiomer thereof, an in vivo-hydrolysable precursor thereof, or a salt thereof acceptable as a food additive.

Further, the present invention provides a composition for functional feedstuff for preventing and improving alcoholic, non-alcoholic, and viral fatty liver diseases, the composition containing the compound of Chemical Formula I, a stereoisomer thereof, an enantiomer thereof, an in vivo-hydrolysable precursor thereof, or a salt thereof acceptable as a food additive, as a pharmaceutically acceptable carrier and an effective component.

Further, the present invention provides a composition for functional feedstuff for preventing and improving obesity, the composition containing the compound of Chemical Formula I, a stereoisomer thereof, an enantiomer thereof, an in vivo-hydrolysable precursor thereof, or a salt thereof acceptable as a food additive.

Further, the present invention provides a composition for functional feedstuff for preventing and improving central nervous system disorders such as Parkinson's disease, schizophrenia, and manic-depression, the composition containing the compound of Chemical Formula I, a stereoisomer thereof, an enantiomer thereof, an in vivo-hydrolysable precursor thereof, or a salt thereof acceptable as a food additive, as a pharmaceutically acceptable carrier and an effective component.

Further, the present invention provides a composition for functional food and beverage for preventing and improving diseases through Nurr1 activation, the composition containing the compound of Chemical Formula I, a stereoisomer thereof, an enantiomer thereof, an in vivo-hydrolysable precursor thereof, or an acceptable salt thereof. The diseases include diabetes, diabetes complications (foot ulcer and renal failure), vascular diseases (hyperlipidemia, atherosclerosis, and atherosclerotic stroke), fatty livers (alcoholic, non-alcoholic, or viral fatty liver diseases), obesity, and central nervous system disorders (Parkinson's disease, schizophrenia, and manic-depression).

Further, the present invention provides a composition for functional food and beverage for preventing and improving diseases through LXR inhibitory activity, the composition containing the compound of Chemical Formula I, a stereoisomer thereof, an enantiomer thereof, an in vivo-hydrolysable precursor thereof, or an acceptable salt thereof. The diseases include diabetes, diabetes complications (foot ulcer and renal failure), vascular diseases (hyperlipidemia, atherosclerosis, and atherosclerotic stroke), fatty livers (alcoholic, non-alcoholic, or viral fatty liver diseases), obesity, and central nervous system disorders (Parkinson's disease, schizophrenia, and manic-depression).

Examples of the LXR agonist mentioned in the present invention are as follows. However, the present invention is not limited to the materials exemplified below, and is applied to all the LXR agonists [the existing LXR agonists: WO2010/054229, WO2010/039529, WO2010/023317, WO2009/150109, W02009/040289, WO2009/024550, W02009/021868, WO2008/119657, WO2008/073825, WO2007/092065, W02007/081335, WO2007/050425, WO2007/050271, WO2007/047991, W02007/002563, WO2007/002559, WO2006/109633, WO2006/073367, WO2006/073366, WO2006/073365, WO2006/073364, WO2006/073363, WO2006/066779, WO2006/046593, W02006/037480, WO2006/017384, WO2006/003923, WO2005/121093, WO2005/113499, WO2005/077124, WO2005/077122, WO2005/058834, W02005/023782, WO2005/023247, WO2005/023196, WO2005/023188, WO2005/016277 WO2005/005417, WO2005/005416, WO2004/076418, WO2004/072041, WO2004/026816, WO2004/024162, WO2004/024161, WO2004/011448, WO2004/009091, WO2003/106435, WO2003/099775, WO2003/099769, W02003/090869, WO2003/090746, WO2003/090732, WO2003/082802, WO2003/082205, WO2003/082192, WO2003/060078, WO2003/059884, WO2003/059874, WO2003/053352, WO2003/045382, WO2003/031408, WO2002/062302, WO2002/024632, WO2001/060818, WO2001/003705, WO2000/066611, WO2000/054759, WO1997/028137, EP1398032, etc.].

The LXR agonist is preferably N-(2,2,2-trifluoroethyl)-N-[4-[2,2,2-trifluoro-1-hydroxy-1-(trifluoromethyl)ethyl]phenyl]-benzenesulfonamide (T0901317) or 3-[3-[[[2-chloro-3-(trifluoromethyl)phenyl]methyl](2,2-diphenylethyl)amino]propoxy]benzeneacetic acid (GW3965).

The amount of the sesterterpene compound of Chemical Formula I, a stereoisomer thereof, an enantiomer thereof, an in vivo-hydrolysable precursor thereof, or a pharmaceutically acceptable salt thereof, which is used to achieve therapeutic effects according to the present invention is varied depending on the specific compound, administration method, subject to be treated, and disease to be treated, but depends on the conventional medicine administration amount. More preferably, the compound of Chemical Formula I may be administered in the range of an effective input amount of 1-100 mg/kg (body weight)/lday. In addition, the compound is administered one per day or several times per day within the range of an effective input amount. In addition, oral administration or topical administration may be possible depending on the kind of dosage form. The pharmaceutical composition according to the present invention may be formulated into all of the existing various forms in the case of oral administration, and may be in various forms such as tablet, powder, dry syrup, chewable tablet, granule, chewing tablet, capsule, soft capsule, pill, drink, sublingual tablet, and the like. The tablet according to the present invention may be administered to a patient in an effective amount through any bio-available form or manner, that is, an oral pathway. An appropriate form or manner may be easily selected depending on characteristics of a disease state to be treated or prevented, stage of the disease, and other related matter. If the composition according to the present invention is in a tablet form, it may further include at least one pharmaceutically acceptable vehicle, and the ratio of properties of the vehicle may be determined by dissolution and chemical properties of the selected tablet, the selected administration pathway, and the standard pharmaceutical practice.

### [Advantageous Effects]

The sesterterpene compound of Chemical Formula I according to the present invention has superior Nurr1 activation, and thus can control and maintain blood sugar, treat insulin-independent diabetes, and prevent the occurrence of diabetes complications. Further, the sesterterpene compound of Chemical Formula I according to the present invention can be used as an effective component of a composition for improving, treating, and preventing diabetes and diabetes complications (foot ulcer and renal failure) by improving insulin sensitivity through regulation of hormones associated with glucose metabolism and protecting pancreatic function to thereby control fasting blood sugar and prevent the occurrence of diabetes complications (foot ulcer and renal failure).

Further, the sesterterpene compound of Chemical Formula I according to the present invention can have a superior effect in inhibiting differentiation of adipocytes, and thus can be useful in improving, preventing, and treating obesity.

Further, the sesterterpene compound of Chemical Formula I according to the present invention may be used as an effective component of a composition for preventing, treating, and improving alcoholic, non-alcoholic, and viral fatty liver diseases by inhibiting the generation of fatty acids in the liver and promoting beta-oxidation by which the fatty acids are burned to release the heat to thereby significantly reduce fat accumulation in the liver.

Further, the sesterterpene compound of Chemical Formula I according to the present invention reduces low-density lipoprotein (LDL) cholesterol and inhibits expressions of inflammatory cytokines derived from macrophage and endotheliocyte, signaling proteins, and lipid biosynthesis enzymes, and thus can be used as an effective component of a composition for improving, treating, and preventing vascular diseases such as hyperlipidemia and atherosclerosis, alone or in a complex agent together with the existing LXR agonists that have been developed until the present time.

Further, the sesterterpene compound of Chemical Formula I according to the present invention that activates Nurr1 may be used alone as an effective component of a composition for improving, treating, and preventing brain disorders such as Parkinson's disease, schizophrenia, and manic-depression.

Further, the sesterterpene compound of Chemical Formula I according to the present invention may be used as an effective component of a composition for improving, treating, and preventing Alzheimer's disease in a complex agent together with the existing LXR agonists.

### [Brief Description of Drawings]

FIG. 1 shows a chemical formula of CMDD-X.

FIG. 2 is a graph showing activity of the compound CMDD-X on Nurr1.

FIG. 3 shows results showing selectivity of the compound CMDD-X.

FIG. 4 is a graph showing fasting blood sugar of a diabetes db/db mouse (CMDD-X-administered group).

FIG. 5 is a graph showing the plasma insulin concentration of a diabetes db/db mouse (CMDD-X-administered group).

FIG. 6 is a graph showing the plasma adiponectin concentration of a diabetes db/db mouse (CMDD-X-administered group).

FIG. 7 is a graph showing the plasma adiponectin polymer concentration of a diabetes db/db mouse (CMDD-X-administered group).

FIG. 8 is a graph showing the glucose tolerance of a diabetes db/db mouse (CMDD-X-administered group).

FIG. 9 is a graph showing the insulin tolerance of a diabetes db/db mouse (CMDD-X-administered group).

FIG. 10 is a graph showing the glucose tolerance of a high-fat feeding mouse (CMDD-X-administered group).

FIG. 11 is a graph showing the insulin tolerance of a high-fat feeding mouse (CMDD-X-administered group).

FIG. 12 is a graph showing expressions of genes that promote lipid synthesis by decomposing the glucose accumulated in the body (CMDD-X-administered group).

FIG. 13 is a graph showing expressions of genes that are involved in treating diabetes by decomposing the lipid, newly generated through decomposition of the glucose accumulated in the body, to thereby release the heat (CMDD-X-administered group).

FIG. 14 is a graph showing the blood urea nitrogen (BUN) of a diabetes disease model (CMDD-X-administered group), which is a biomarker of renal failure as a diabetes complication.

FIG. 15 is a graph showing liver function improvement of an administered material of a fatty liver disease model (CMDD-X-administered group).

FIG. 16 is a graph showing a low-density lipoprotein (LDL) cholesterol drop effect of an administered material (CMDD-X-administered group).

FIG. 17 shows graphs showing changes in expressions of genes associated with lipid synthesis in a fatty liver disease model (CMDD-X-administered group).

FIG. 18 shows graphs showing expressions of genes necessary for improving the liver function by decomposing fat to release heat in a fatty liver disease model (CMDD-X-administered group).

FIG. 19 shows graphs showing expressions of genes necessary for improving the liver function by inhibiting an inflammatory reaction in a fatty liver disease model (CMDD-X-administered group).

FIG. 20 is a graph showing the increase and decrease of a cytokine gene (TNF-α) expressed in an activated macrophage (CMDD-X-administered group).

FIG. 21 is a graph showing the increase and decrease of a cytokine gene (IL-6) expressed in an activated macrophage (CMDD-X-administered group).

FIG. 22 is a graph showing the concentration of a cytokine gene (IL-6) expressed in an activated macrophage (CMDD-X-administered group).

FIG. 23 is a graph showing the concentration of nitric acid expressed in an activated macrophage (CMDD-X-administered group).

FIG. 24 is a graph showing the increase and decrease of an inducible nitric oxide synthetase expressed in an activated macrophage (CMDD-X-administered group).

FIG. 25 is a graph showing the increase and decrease of a cytokine gene (MCP-1) expressed in an activated endotheliocyte (CMDD-X-administered group).

FIG. 26 is a graph showing the increase and decrease of an intercellular adhesion molecule gene (VCAM-1) expressed in an activated endotheliocyte (CMDD-X-administered group).

FIG. 27 shows western blotting results showing the increase and decrease of an intercellular adhesion molecule protein (VCAM-1) expressed in an activated endotheliocyte (CMDD-X-administered group).

FIG. 28 is a graph showing improvement in exercise capacity of mice after the compound was administered to the mice with induced Parkinson's disease (CMDD-X-administered group).

### [Best Mode]

Hereinafter, embodiments of the present invention will be described in detail with reference to the accompanying drawings. However, the embodiments are used to exemplify the present invention. The present invention may be variously modified and changed without being limited by the embodiments.

The present invention has an intent to disclose a compound of Chemical Formula 1, stereoisomer thereof, enantiomer thereof, in vivo-hydrolysable precursor thereof, or pharmaceutically acceptable salt thereof, and uses of these materials as an agent for preventing and treating diabetes, foot ulcer and renal failure due to the diabetes, obesity, fatty liver, vascular diseases such as hyperlipidemia, atherosclerosis, and atherosclerotic stroke, and brain disorders such as Parkinson's disease and Alzheimer's disease.

The present invention has an intent to disclose a compound of Chemical Formula 1, stereoisomer thereof, enantiomer thereof, in vivo-hydrolysable precursor thereof, or pharmaceutically acceptable salt thereof, and uses of these materials as an effective component of functional food and beverage, helpful in preventing and improving diabetes, foot ulcer and renal failure due to the diabetes, obesity, fatty liver, vascular diseases such as hyperlipidemia, atherosclerosis, and atherosclerotic stroke, and brain disorders such as Parkinson's disease and Alzheimer's disease.

The present invention has an intent to disclose a compound of Chemical Formula 1, stereoisomer thereof, enantiomer thereof, in vivo-hydrolysable precursor thereof, or pharmaceutically acceptable salt thereof, and uses of these materials as an effective component of cosmetics, helpful in preventing and improving obesity.

The present invention has an intent to disclose a compound of Chemical Formula 1, stereoisomer thereof, enantiomer thereof, in vivo-hydrolysable precursor thereof, or pharmaceutically acceptable salt thereof, and uses of these materials as an effective component of functional feedstuff, helpful in preventing and improving diabetes, foot ulcer and renal failure due to the diabetes, obesity, fatty liver, vascular diseases such as hyperlipidemia, atherosclerosis, and atherosclerotic stroke, and brain disorders such as Parkinson's disease and Alzheimer's disease.

In addition, the present invention provides a noble sesterterpene compound of Chemical Formula II.

Example 1: Synthesis of Compound 1

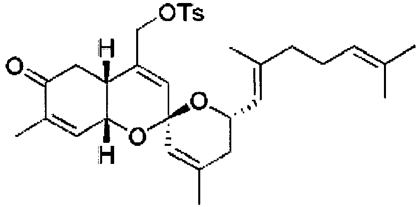

Phorbaketal A (10mg, 0.025mmol, Rho et. al., Organic Letters, 2009, 11, 5590-5593) was dissolved in dichloromethane, and then p-TsCl (5.4mg, 1.2eq) and triethylamine (0.01mmol) were put therein, followed by stirring for 5 hours. The reaction was terminated by an aqueous saturated NaHCO₃ solution and water (40ml). The organic solvent layer was washed with water twice, followed by drying over Na₂SO₄, and then concentration was conducted by using an evaporation concentrator. The resultant material was purified by using silica column chromatography, to obtain Compound 1. MS m/z 554 [M+H]⁺

Example 2: Synthesis of Compound 2

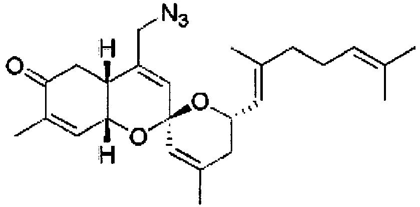

Compound 1 (10mg, 0.018mmol) was dissolved in dimethylformamide (DMF, 5m1), and NaN₃ (11.7mg, 10mmol) was put therein, and then the reaction was allowed to proceed at 70°C under nitrogen conditions for 8 hours. After the reaction liquid was cooled, ice water was put therein, followed by washing with water, and then the organic solvent layer was separated. After drying over Na₂SO₄ and distillation under reduced pressure, the obtained residue was purified by silica column chromatography, to obtain Compound 2. MS *m*/*z* 424 [M+H]⁺

Example 3: Synthesis of Compound 3

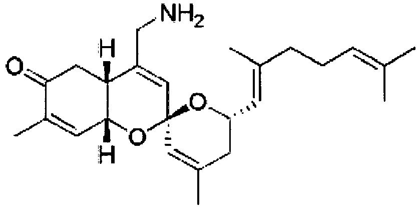

Compound 2 (10mg, 0.023mmol) was dissolved in acetonitril (5ml), and NaI (0.20mmol) was put and FeCl₃ (0.032mmol) was put therein. After the reaction liquid was stirred for 20 minutes, the reaction was terminated by adding chloroform (5 ml) thereto. After washing with an aqueous Na₂SO₃ solution and an aqueous NaHCO₃ solution, the obtained organic solvent layer was again washed with salt water. The organic solvent layer was dried over Na₂SO₄ and distilled under reduced pressure, and then the obtained residue was purified by silica column chromatograph to obtain Compound 3. MS *m*/*z* 398 [M+H]⁺

Example 4: Synthesis of Compound 4

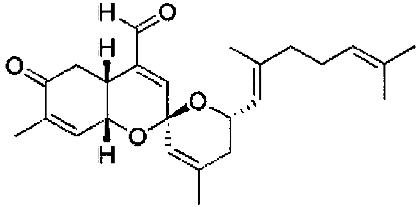

Phorbaketal A (20mg, 0.050mmol) was dissolved in dichloromethane (8ml), and then Dess-Martin periodinane (21.3mg, 0.050mmol) prepared in dichloromethane (10ml) was added thereto, followed by stirring. After 30 minutes, dichloromethane (50ml) was put in the homogeneous reaction liquid, and then 1.3M NaOH (20ml) and water (25ml) were added thereto. The organic solvent layer was distilled under reduced pressure to obtain a residue, followed by silica column chromatography, to obtain Compound 4 (15.9mg, 80%). ¹H-NMR(300MHz, CDCl₃): d1.59(3H,s), 1.67(3H,s), 1.78(3H,s), 1.79(3H,s), 1.87(3H,s), 2.12(6H,m), 2.38(1H,t), 2.71(1H,dd), 3.02(1H,d), 4.56(1H,s), 4.82(1H,t), 5.11(1H,s), 5.27(1H,d), 5.40(1H,s), 6.53(1H,s), 6.67(1H,d), 9.52(1H,s). MS *m*/*z* 397 [M+H]⁺

Example 5: Synthesis of Compound 5

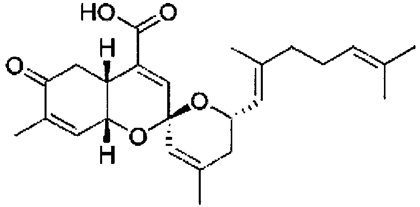

80% NaClO₂ (45.3mg, 20eq) and NaH₂PO₄·2H₂O (52mg, 15eq.) were prepared in water (5ml), and then Compound 4 (10mg, 0.025mmol) and 2-methylbut-2-ene (10ml) prepared in tert-butyl alcohol (10ml) at 0°C were added thereto. Dioxane (5ml) was added thereto, and the mixture was stirred at room temperature for 8.5 hours. After the resultant material was diluted with water (45ml), the target product was extracted with chloroform (2*30ml) and then washed with salt water (60ml) and water (40ml), followed by drying over Na₂SO₄. The residue left after distillation under reduced pressure was purified by silica column chromatography, to obtain an amorphous type Compound 5 (9.5mg, 91% yield). ¹H-NMR(300MHz, CDCl₃): d1.63(3H,s), 1.69(3H,s), 1.79(3H,s), 1.83(3H,s), 1.92(3H,s), 2.03-2.16(6H,m), 2.36(1H,t), 2.71(1H,dd), 2.99(1H,d), 4.50(1H,s), 4.76(1H,t), 5.12(1H,s) 5.24(1H,d), 5.35(1H,s), 6.39(1H,s), 6.73(1H,d) MS *m*/*z* 413 [M+H]⁺

Example 6: Synthesis of Compound 6

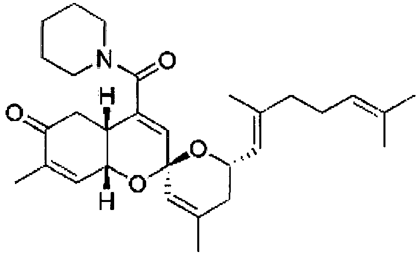

Compound 5 (10mg, 0.024mmol) was dissolved in anhydrous dichloromethane (5ml), and then oxalyl chloride (1.0ml) was put therein, followed by stirring for 3 hours. Excess oxalyl chloride was removed by distillation under reduced pressure, followed by mixing with acid chloride prepared in dichloromethane (2ml), and then piperidine (2mg, 0.024mmol) was added thereto. The mixture was stirred for 1 hour, followed by mixing with water, and then the organic solvent layer was separated and then washed with water (25ml). After drying over Na₂SO₄, the obtained residue was purified by column chromatography, to obtain Compound 6 (1.9mg, 92%). ¹H-NMR (300 MHz, CDCl₃): *d*1.56-1.68(12H, *s*) 1.76(6H, s) 1.88(3H, s) 2.04-2.13(6H, m) 2.54(2H, d) 3.92(1H, m), 3.51(4H, bs) 4.66(1H, s) 4.77(1H, s) 5.10(1H,s) 5.23(1H, d) 5.36(1H, s), 5.63(1H,s) 6.65(1H, s). MS *m*/*z* 480 [M+H]⁺

Example 7: Synthesis of Compound 7

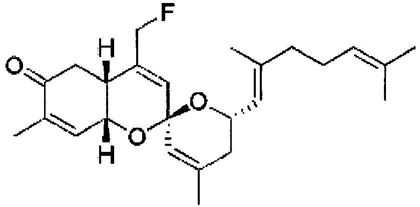

Phorbaketal A (10mg, 0.025mmol) was dissolved in dichloromethane (2ml), and then diethylaminosulfur trifluoride (DAST, Et₂NSF₃) (6.06mg, 1.5eq) prepared in dichloromethane (5ml) was slowly added thereto at -78°C. The temperature of the reaction liquid was raised to room temperature, and then mixed with water. The organic solvent layer was washed with water, followed by drying over Na₂SO₄ and distillation under reduced pressure, and the obtained residue was purified by silica gel column chromatography. 7.8mg of Compound 7 was obtained (78%). ¹H-NMR(300MHz, CDCl₃): d1.61(3H,s), 1.70(3H,s), 1.77(3H,s), 1.78(3H,s), 1.85(3H,s), 2.08-2.16(6H,m), 2.512.68(3H,m), 4.61(1H,d), 4.71-4.83(2H,m), 4.92(1H,m), 5.10(1H,d), 5.27(1H,d), 5.35(1H,s), 5.73(1H,d) 6.67(1H,d). MS m/z 401 [M+H]⁺

Example 8: Synthesis of Compound 8

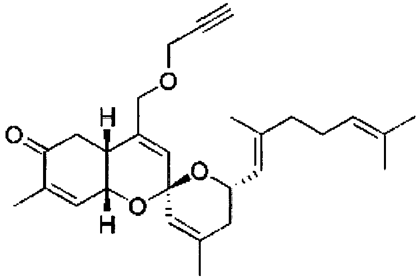

Phorbaketal A (20mg, 0.050mmol) was prepared in anhydrous DMF (10ml). Anhydrous K₂CO₃ (6.93mg, 2eq) was added thereto, and the mixture liquid was allowed react at 40°C for 30 minutes. Propargyl bromide (3-bromopropyne, 12mg, 2eq) was slowly added to the mixture liquid, and the reaction was allowed to further proceed for 6 hours while the progress of the reaction was confirmed by TLC. The reaction was terminated by water (50ml), followed by extraction with ethylacetate (3*50ml). The organic solvent layer was washed with water (50ml*2), and then dried over anhydrous Na₂SO₄. After distillation under reduced pressure, the obtained residue was purified by silica column chromatography, to obtain Compound **8** 15.2mg (70%). MS m/z 437 [M+H]⁺

Example 9: Synthesis of Compound **9**

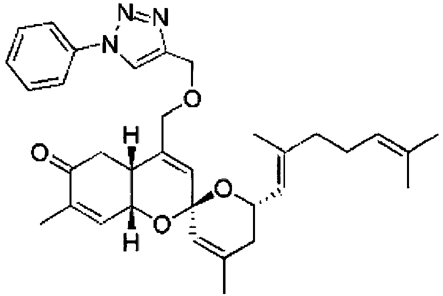

Compound 8 (10mg, 0.022mmol) and phenylazide (2.7mg, 0.022mol) were dissolved in DMF (5ml), and then 1M sodium ascorbate (0.2ml, 0.11mmol) and 1M CuSO₄ (0.1ml, 10mol%) were sequentially added while stirring at 65°C. The reaction solution was stirred at 65°C for 24 hours, and the reaction was terminated by slowly adding cold water. The precipitate generated due to addition of water was filtered, and then washed with water, followed by purification by silica column chromatography. MS m/z 556 [M+H]⁺

Example 10: Synthesis of Compound 10

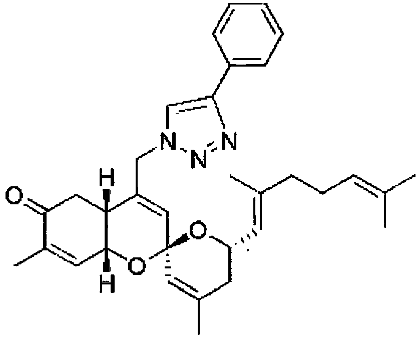

Compound 2 (10mg, 0.023mmol) and phenylacetylene (2.4mg, 0.023mmol) were prepared in DMF (5m), and then 1M sodium ascorbate (0.2ml, 0.11mmol) and 1M CuSO₄ (0.1ml, 10mol%) were sequentially added while stirring at 65°C. The reaction liquid was stirred at 65°C for 24 hours, and the reaction was terminated by slowly adding cold water. The precipitate generated due to addition of water was filtered, and then washed with water, followed by purification by silica column chromatography. MS m/z 526 [M+H]⁺

Example 11: Synthesis of Compound 11

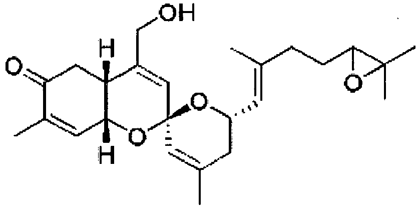

Phorbaketal A (8mg, 0.017mmol) was mixed with methanol (4ml), and K₂CO₃ (3.6mg, 0.026mmol) was added thereto, and then the mixture was stirred at room temperature for 2 hours. The reaction liquid was layer-separated by using water (50ml) and ethylacetate (3*50ml). The organic solvent layer was dried over Na₂SO₄ and then distilled under reduced pressure, and the obtained residue was purified by column chromatography, to obtain Compound 11. MS *m*/*z* 415 [M+H]⁺

Example 12: Synthesis of Compound 12

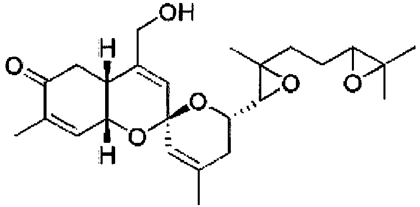

Phorbaketal A (10mg, 0.024mmol) was dissolved in dichloromethane (5ml), and then m-CPBA(10.2mg, 2.4eq) dissolved in dichloromethane (6ml) was slowly mixed therewith at 0°C. After stirring at room temperature for 3 hours, an aqueous saturated NaHCO₃ solution was put therein, followed by further stirring for 30 minutes. The reaction mixture was extracted with dichloromethane, and the organic solvent layer was washed with water, dried, and then distilled under reduced pressure. The residue was purified by silica column chromatography, to obtain Compound 12 7.7mg (70%). MS *m*/*z* 431 [M+H]⁺

Example 13: Synthesis of Compound 13

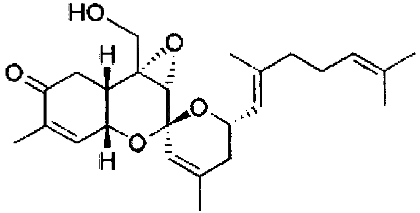

The finely broken molecular sieve (100mg) was mixed in anhydrous dichloromethane (5ml), and then cooled to - 20°C. (-)-Diethyl-tartrate (1.5mg, 0.2eq) and Ti(OiPr)₄ (2.1mg, 0.2eq) were added thereto, followed by stirring for 30 minutes. Phorbaketal A (15mg, 0.037mmol) was put therein, followed by further stirring for 30 minutes. Tert-butyl hydroperoxide (TBHP, 4mg, 1.2eq) was put therein, followed by stirring for 3 hours, and then the progress of the reaction was confirmed by TLC. The reaction was terminated by using water (10ml) at 0°C, and the stirring was further conducted at 0°C for 1 hour. 30% of an aqueous NaOH solution (2ml) and an aqueous NaCl solution (2ml) were mixed therewith, followed by further stirring for 30 minutes, and then the cellite layer was filtered. The cellite layer was washed with dichloromethane, and the organic solvent was distilled under reduced pressure, and then the obtained residue was separated by silica column chromatography to obtain Compound 13. MS m/z 415 [M+H]⁺

Example 14: Synthesis of Compound 14

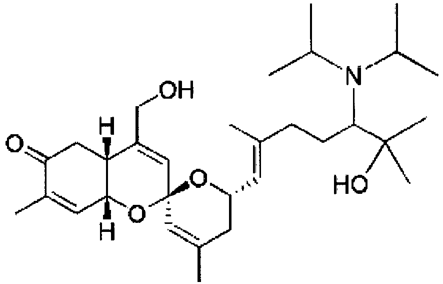

Compound 11 (10mg, 0.024mmol) was dissolved in THF (5ml), and then diisopropylamine (1.2mmol) was mixed therewith, followed by stirring for 4 hours. The temperature of the reaction liquid was lowered to 0°C, and the reaction was terminated by water (20ml). The organic solvent layer was separated, dried over anhydrous Na₂SO₄, and then distilled under reduced pressure, and the obtained residue was purified by silica column chromatography, to obtain Compound 14. MS m/z 516 [M+H]⁺

Example 15: Synthesis of Compound 15

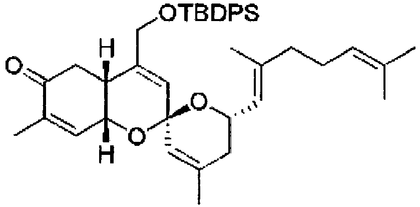

Phorbaketal A (10mg, 0.025mmol) and imidazole (3.4mg, 0.05mmol) were dissolved in DMF (10ml), and then tert-butyldiphenyl silylchloride (8.2ml, 0.03mmol) was added at 0°C, followed by stirring for 6 hours. After the reaction was finished, the reaction liquid was layer-separated by using water and ethylacetate (2*15ml), and the organic solvent layer was dried over Na₂SO₄. After distillation under reduced pressure, the obtained residue was purified by silica column chromatography, to obtain Compound 15. MS m/z 637 [M+H]⁺

Example 16: Synthesis of Compound 16

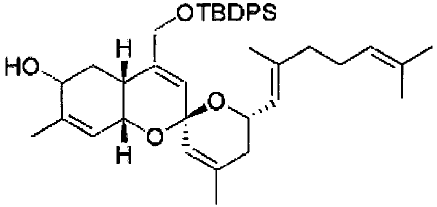

Compound 13 (10mg, 0.015mmol) was dissolved in dichloromethane (1ml), and DIBAL-H ((i-Bu₂AlH)₂, 40ul, 1.5M in toluene) was added at -78°C, followed by further stirring for 30 minutes. The reaction was terminated by using ethanol (10µℓ), and water (10ml) and NaF (20mg) were added thereto. The organic solvent layer was separated, dried, and distilled under reduced pressure, and the obtained residue was purified by silica column chromatograph, to obtain Compound 16. MS m/z 639 [M+H]⁺

Example 17: Synthesis of Compound 17

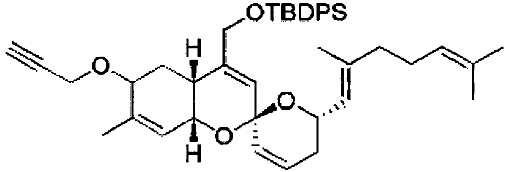

Compound 17 was obtained by using Compound 16 (10mg, 0.016mmol) as a start material through the same synthesis method as Compound 8. MS *m*/*z* 663 [M+H]⁺

Example 18: Synthesis of Compound 18

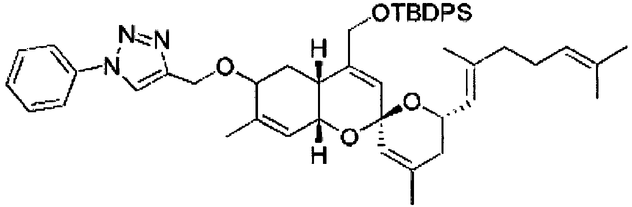

Compound 18 was obtained by using Compound 17 (10mg, 0.015mmol) as a start material through the same synthesis method as Compound 9. MS m/z 796 [M+H]⁺

Example 19: Synthesis of Compound 19

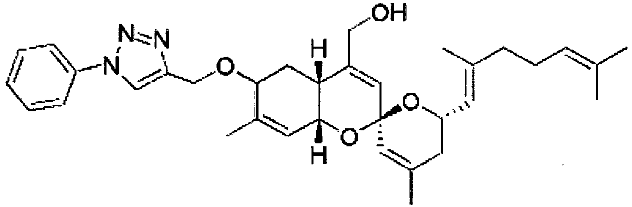

Tetra-n-butylammonium fluoride (TBAF, CH₃CH₂CH₂CH₂)₄N⁺F⁻) (4mg, 0.015 mmol) was added in Compound 18(10mg, 0.013mmol) dissolved in anhydrous THF (10ml), followed by stirring at room temperature for 4 hours. The reaction liquid was layer-separated by using an aqueous saturated ammonium chloride solution and ethylacetate, and the obtained organic solvent layer was dried over Na₂SO₄. After distillation under reduced pressure, the obtained residue was purified by silica column chromatography, to obtain Compound 19. MS *m*/*z* 558 [M+H]⁺

Example 20: Synthesis of Compound 20

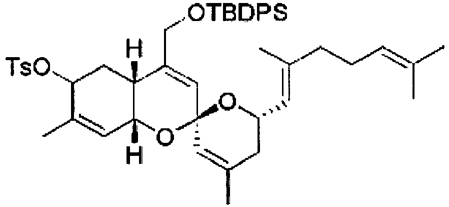

Compound 20 was obtained by using Compound 15 (10mg, 0.016mmol) through the same synthesis method as Compound 1. MS *m*/*z* 793 [M+H]⁺

Example 21: Synthesis of Compound 21

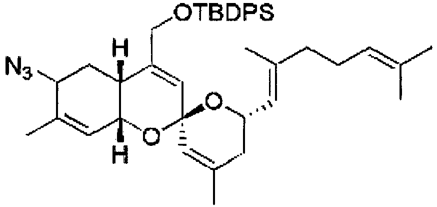

Compound 21 was obtained by using Compound 20 (10mg, 0.013mmol) through the same synthesis method as Compound 2. MS *m*/*z* 664 [M+H]⁺

Example 22: Synthesis of Compound 22

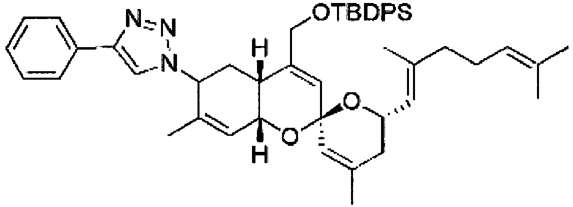

Compound 22 was obtained by using Compound 21 (10mg, 0.015mmol) as a start material through the same synthesis method as Compound 10. MS *m*/*z* 766 [M+H]⁺

Example 23: Synthesis of Compound 23

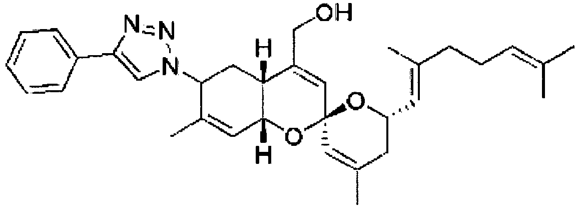

Compound 23 was obtained by using Compound 22 (10mg, 0.013mmol) as a start material through the same synthesis method as Compound 19. MS *m*/*z* 528 [M+H]⁺

Example 24: Synthesis of Compound 24

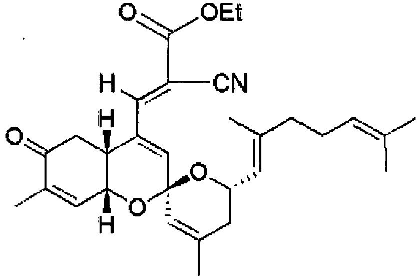

Compound **4** (10mg, 0.025mmol) was dissolved in pyridine (5ml), and then ethylcyanoacetate (5.7mg, 2eq) and a small amount of piperidine were added. The reaction liquid was stirred for 8 hours, and the progress of the reaction was confirmed by using TLC. The reaction was terminated by using water and dichloromethane, followed by layer separation. The organic solvent layer was distilled under reduced pressure, and the obtained residue was purified by silica column chromatography, to obtain Compound **24.** MS *m*/*z* 492 [M+H]⁺

Example 25: Synthesis of Compound **25**

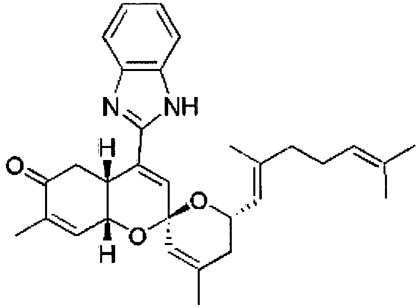

Compound **4** (10mg, 0.025mmol) and o-phenylene diamine (2.3mg, 0.025mmol) were dissolved in a water/acetonitrile (1:1) mixture liquid (3ml), and then clayzic (20mg) was put therein, followed by sufficient stirring at room temperature. Extraction with dichloromethane, washing with water, drying over Na₂SO₄, and then distillation under reduced pressure were conducted. The obtained residue was purified by silica column chromatography, to obtain Compound **25.** MS *m*/*z* 485 [M+H]⁺

Example 26: Synthesis of Compound **26**

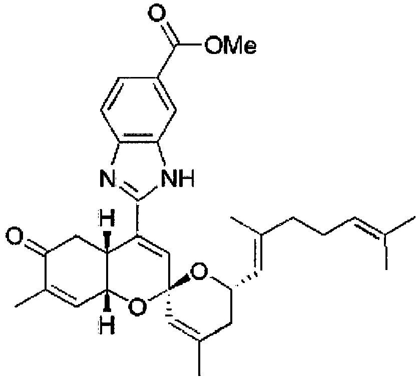

Compound **26** (9.4mg, 69%) was obtained by using Compound **4** (10mg, 0.025mmol) as a start material through the same synthesis method as Compound **25.** MS *m*/*z* 543 [M+H]⁺

Example 27: Synthesis of Compound **27**

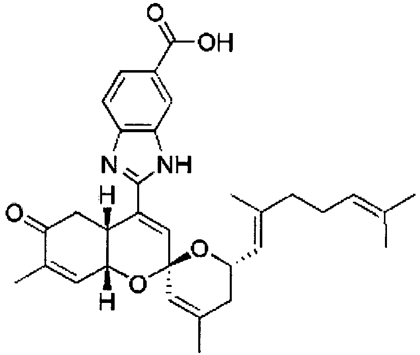

Compound **26** (10mg, 0.018mmol) and NaOH (1.5mg, 0.036mmol) were dissolved in methanol, followed by stirring at 50°C for 5 hours. After the reaction was terminated, neutralization was conducted by using water and hydrochloric acid, and then layer-separation was conducted by using ethylacetate (25ml). The organic solvent layer was dried over anhydrous Na₂SO₄ and distilled under reduced pressure. The obtained residue was purified by silica column chromatography, to obtain compound 27 (6.16mg, 65%). MS *m*/*z* 529 [M+H]⁺

Example 28: Synthesis of Compound **28**

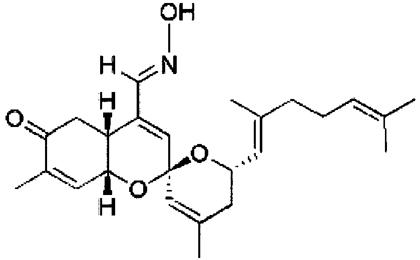

Compound **4** (10mg, 0.025mmol) was dissolved in ethanol and water (7:3) solution (3ml), and then NH₂OH.HCl (3.45mg, 2eq) and NaHCO₃ (4.2mg, 2eq) were added, followed by stirring at room temperature for 2 hours. Ice water (30 ml) was poured into the reaction liquid, and then layer-separation was conducted by using ethylacetate (2*25ml). The organic solvent layer was dried over anhydrous Na₂SO₄ and distilled under reduced pressure. The obtained residue was purified by silica column chromatography, to obtain Compound **28** (8.83mg, 86%). MS *m*/*z* 412 [M+H]⁺

Example 29: Synthesis of Compound **29**

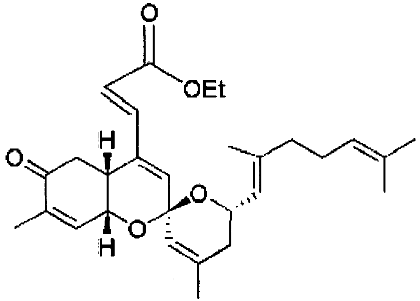

NaOH (0.7mg, 0.03mmol) was put in diethylether (5ml), and then nitrogen conditions were made. Triethyl phosphono acetate (6.7mg, 0.03mmol) dissolved in diethylether (2ml) was put therein, and the mixture was stirred for 20 minutes and then further stirred for 30 minutes at 0°C. Compound **4** (10mg, 0.025mmol) was dissolved in ether (20ml), and the reaction liquid was slowly added thereto, followed by stirring for 4 hours. Water (20ml), hydrochloric acid (5ml), and ether (4*20ml) were added thereto. The obtained organic solvent layer was dried over MgSO₄, and then distilled under reduced pressure. The obtained residue was purified by silica column chromatography, to obtain Compound **29** (9.1mg, 78%). MS m/z 467 [M+H]⁺

Example 30: Synthesis of Compound 30

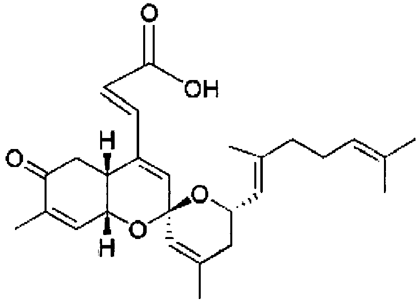

Compound **30** (6.9mg, 74%) was obtained by using Compound **29** (10mg, 0.021mmol) as a start material through the same synthesis method as Compound **27.** MS m/z 439 [M+H]⁺

Example 31: Synthesis of Compound **31**

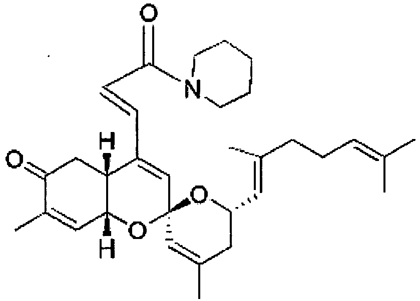

Compound **31** was obtained by using Compound **30** (10mg, 0.022mmol) as a start material through the same synthesis method as Compound **6.** MS m/z 506 [M+H]⁺

Example 32: Synthesis of Compound **32**

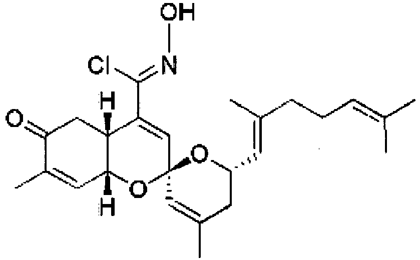

A mixture of Compound **28** (10mg, 0.024mmol) and N-chlorosuccinimide (3mg, 0.024mmol) was dissolved in dichloromethane (3ml), and then stirred at room temperature for 5 minutes. After further stirring for 30 minutes, layer-separation was conducted by using water (20ml) and dichloromethane (2*20ml). The organic solvent layer was dried over anhydrous Na₂SO₄. After distillation under reduced pressure, the obtained residue was purified by silica column chromatography, to obtain Compound **32.** MS *m*/*z* 446 [M+H]⁺

Example 33: Synthesis of Compound **33**

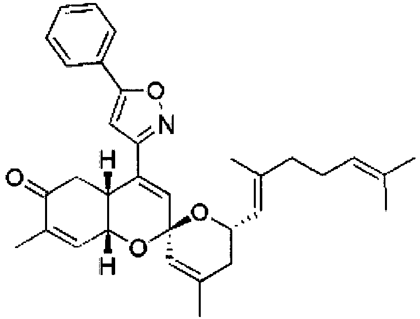

A mixture of Compound **28** (10mg, 0.024mmol) and *N-*chlorosuccinimide (3mg, 0.024mmol) was dissolved in dichloromethane (3ml), and then stirred at room temperature for 5 minutes. After Compound **32** was confirmed by TLC, phenylacetylene (10mg, 0.1mmol) and triethylamine (0.028mmol) were added to the reaction liquid. After further stirring for 30 minutes, layer-separation was conducted by using water (20ml) and dichloromethane (2*20ml). The organic solvent layer was dried over anhydrous Na₂SO₄. After distillation under reduced pressure, the obtained residue was purified by silica column chromatography, to obtain Compound 33. MS *m*/*z* 512 [M+H]⁺

Example 34: Synthesis of Compound **34**

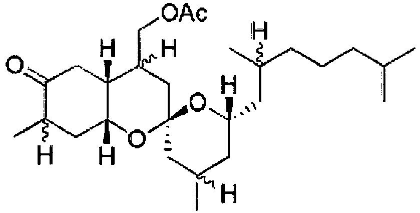

Phorbaketal acetate (10mg, 0.02mmol) dissolved in methanol (4ml) solvent was allowed to react by using 10% Pd/C (5mg) under hydrogen conditions for 10 hours. After filtering using cellite and washing with methanol, the obtained organic solvent layer was distilled under reduced pressure. The obtained residue was purified by silica column chromatography, to obtain Compound **34** (9mg, 91%). MS *m*/*z* 451 [M+H]⁺

Example 35: Synthesis of Compound **35**

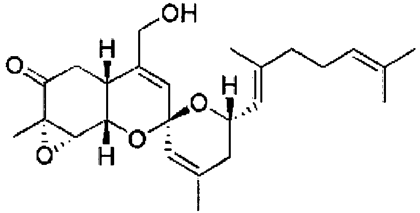

Hydrogen peroxide (4.6µℓ, 0.068mmol) and phorbaketal acetate (10mg, 0.02mmol) were allowed to react with methanol (10ml) and 6N sodium peroxide (7.5µℓ, 0.045mmol) at 0°C for 6 hours. The layer-separation was conducted by using dichloromethane and water, and the organic solvent layer was dried over Na₂SO₄. After distillation under reduced pressure, the obtained residue was purified by silica column chromatography, to obtain Compound **35** (8mg, 87%). ¹H-NMR(300MHz, CDCl₃): 1.27 (s, 3H), 1.49(s, 3H), 1.62(s, 3H), 1.98-1.86(m, 8H), 2.08-2.88(m, 5H), 2.54-2.69(m, 2H), 3.48(s, 1H), 4.09(br s, 2H), 4.75(br s, 2H), 5.11(br s, 1H), 5.26(s, 1H), 5.29(br s, 1H), 5.58(br s, 1H). MS m/z 415 [M+H]⁺

Example 36: Synthesis of Compound **36**

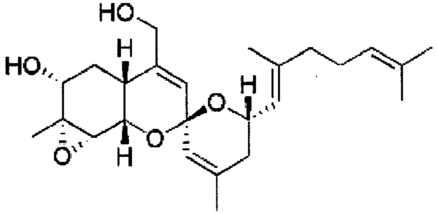

NaBH₄ (1mg, 0.033mmol) was dissolved in methanol (6ml), which was then mixed with Compound 35 (7mg, 0.016mmol) at 0°C. After the reaction liquid was stirred at room temperature for 2 hours, the solvent was distilled under reduced pressure, and the remaining residue was extracted with dichloromethane. After drying over anhydrous Na₂SO₄ and distillation under reduced pressure, the residue was purified by silica column chromatography, to obtain Compound **36** (6mg, 92%). MS *m*/*z* 417 [M+H]⁺

Example 37: Synthesis of Compound **37**

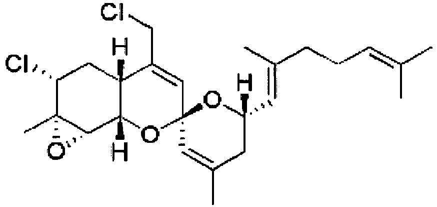

Compound 36 (5mg, 0.012mmol), TPP(12mg, 0.048mmol), and N-chlorosuccineimide (6mg, 0.48mmol) were mixed with toluene solvent, and then allowed to react at 80°C for 6 hours. After the reaction was terminated, the solvent was distilled under reduced pressure, and the residue was purified by silica column chromatography to obtain Compound **37** (5mg, 93%). MS *m*/*z* 453 [M+H]⁺

Example 38: Synthesis of Compound **38**

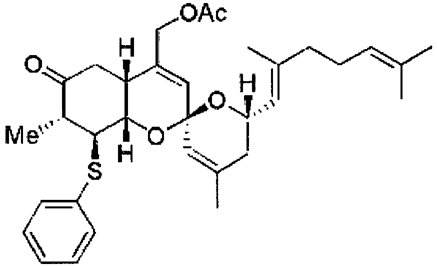

Phorbaketal acetate (10mg, 0.02mmol), together with benzenthiol (9.3µℓ, 0.09mmol) and triethylamine (0.05ml), was put in THF solvent, and then heated for 10 hours. After the reaction was terminated, the reaction liquid was layer-separated by using water (20ml) and ethylacetate. The organic solvent layer was dried, followed by purification by silica column chromatograph, to obtain Compound **38.** MS *m*/*z* 551 [M+H]⁺

Example 39: Synthesis of Compound **39**

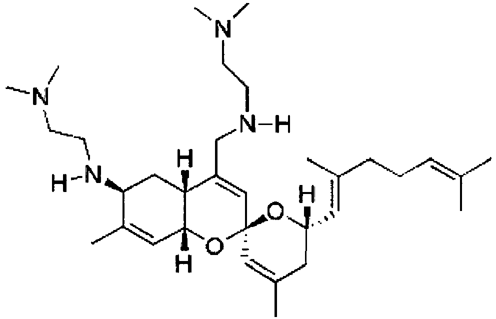

DIABL (0.5ml) was added to phorbaketal acetate (30mg, 0.06mmol) dissolved in anhydrous THF at -78°C. A saturated ammonium chloride solution was put therein, and then the layer-separation was conducted by using ethylacetate (2*20ml). The obtained organic solvent layer was dried by sodium sulfate, followed by distillation under reduced pressure. The obtained residue was used to advance a subsequent reaction. The obtained residue (17mg) was slowly mixed with anhydrous dichloromethane (10ml) and triethylamine (0.2ml) at 0°C. The reaction, together with mesyl chloride, was allowed to proceed at room temperature for 1 hour, and then the layer-separation was conducted by using dichloromethane and water, followed by concentration of the organic solvent layer. The residue was purified by using silica column chromatography, to obtain a product 18mg. This product (10mg) was reacted with N-dimethylaminopropylamine (81mg, 0.78 mmol), together with Cs₂CO₃ (100mg, 0.3mmol), in DMF at 70°C for 4 hours. Column chromatography was used to obtain Compound 39. MS *m*/*z* 541 [M+H]⁺

Example **40:** Synthesis of Compound **40**

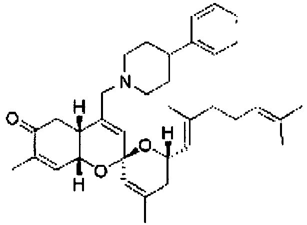

Compound **37** (7mg, 0.014mmol) and Cs₂CO₃ (50mg) were mixed with DMF (5ml), and then, together with phenylpiperazine (100mg, 0.61mmol), stirred at 80°C for 6 hours. The solvent was removed by distillation under reduced pressure, and then the residue was purified by silica column chromatography, to obtain Compound **40.** MS *m*/*z* 542 [M+H]⁺

Example 41: Synthesis of Compound **41**

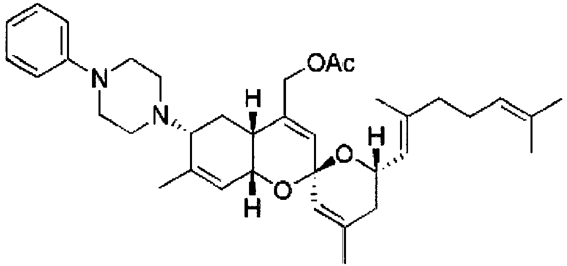

NaBH₄ (1mg, 0.033mmol) was slowly added into a methanol (6ml) of cerium chloride heptahydrate (12mg, 0.033mmol) and phorbaketal acetate (10mg, 0.02mmol). After the reaction liquid was stirred at room temperature for 4 hours, the solvent was distilled under reduced pressure. The residue was layer-separated by using dichloromethane and water, and then the organic solvent layer was dried over Na₂SO₄, followed by proceeding to the next step. The residue was dissolved in dichloromethane, and then a 85% tetrafluorboric acid diethylether complex and phenylpiperazine were slowly added at -60°C while stirring for 1 hour. The mixture was layer-separated by using water and dichloromethane, followed by distillation under reduced pressure, and the organic solvent layer was separated and purified by using silica column chromatography, to obtain Compound **41.** MS *m*/*z* 587 [M+H]⁺

Example 42: Synthesis of Compound **42**

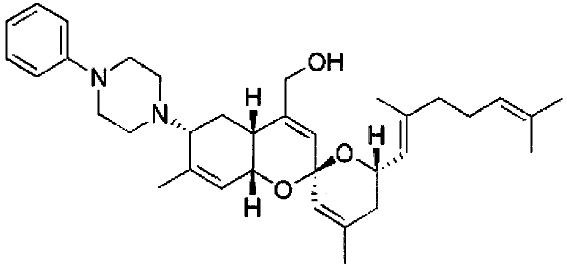

Compound **41** (3mg, 0.005mmol) was dissolved in methanol (2ml), and then K₂CO₃ (1mg) was added thereto. The reaction was stirred at room temperature for 1 hour, followed by filtration and distillation under reduced pressure, to obtain a residue. The residue was purified by using silica column chromatography, to obtain Compound **42.** MS *m*/*z* 545 [M+H]⁺

Example 43: Synthesis of Compound **43**

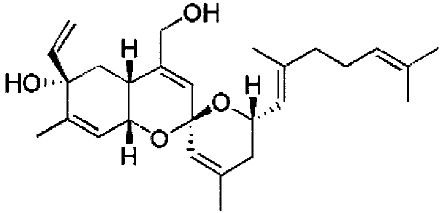

Phorbaketal acetate (10mg, 0.02mmol) was dissolved in anhydrous THF, and then **BrMg** (1ml) was added at -30°C, followed by stirring. The layer-separation was conducted by using an aqueous saturated ammonium chloride solution and ethylacetate (2*20ml), and the separated organic solvent layer was dried over anhydrous Na₂SO₄. After distillation under reduced pressure, the residue was purified by silica column chromatography, to obtain Compound **43** (8mg, 82%). ¹H-NMR(300MHz, CDCl₃): d1.52(s, 3H), 1.56-1.86(m, 9H), 2.08-2.88(m, 8H), 2.54-2.69(m, 2H), 4.3(br s, 2H), 4.75-4.83(m, 2H), 5.11-5.21(m, 3H), 5.24-5.27(m, 2H), 5.64-5.72(m, 3H), 5.82-5.95(m, 2H). MS *m*/*z* 427 [M+H]⁺

Example 44: Synthesis of Compound **44**

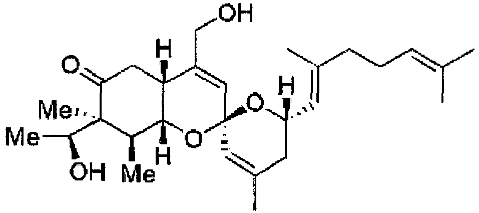

Trimethylammonium (37µℓ, 0.07mmol, 2M solution) was mixed with dichloromethane (6ml), which was then cooled to 0°C. Benzenethiol (79µℓ, 0.077mmol) was slowly added, and then the mixture liquid was stirred for 20 minutes. Compound **4** (10mg, 0.02mmol) was slowly added at -78°C, followed by further stirring for 15 minutes. THF (4ml) was added, followed by stirring for 5 minutes, and then acetaldehyde (43µℓ, 0.077mmol) was slowly added. After further stirring for 20 minutes, the layer separation was carried out by adding water (5ml) and dichloromethane (5 ml) thereto. The organic solvent layer was washed with 1N HCl (5ml), and further, the aqueous layer was extracted by using ethylacetate (2*5ml). The entire organic solvent layer was collected, washed with water (10ml) and salt water (10ml), and then dried over MgSO₄. After distillation under reduced pressure, the obtained residue was separated by using column chromatography to obtain Compound **44.** MS *m*/*z* 459 [M+H]⁺

Example 45: Synthesis of Compound **45**

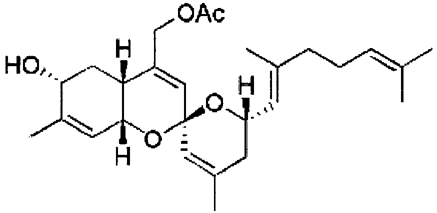

Starting from phorbaketal A (20mg, 0.05mmol) as a start material, acetic acid was allowed to react in the cyclohexane solvent using sulfuric acid as a catalyst, to thereby transform free alcohol into an acetyl group. The obtained product was used by using the same synthesis method as Compound **12** to obtain Compound **45** (19mg, 95%). MS *m*/*z* 443 [M+H]⁺

Example 46: Synthesis of Compound **46**

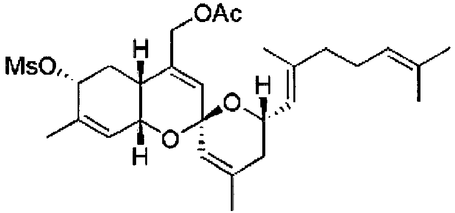

Triethylamine (1ml) and Compound **45** (15mg, 0.037mmol) were dissolved in anhydrous dichloromethane (10ml), and then methanesulfonyl chloride (8.6µℓ, 0.11mmol) was added thereto, followed by stirring at room temperature for 1 hour at 0°C. The reaction liquid was layer-separated by using water (20ml) and dichloromethane (50ml), and the organic solvent layer was washed with water and salt water, followed by drying over Na₂SO₄. Distillation under reduced pressure and separation by silica column chromatography were conducted to obtain Compound **46** (20mg, 94%). ¹H-NMR(300 MHz, CDCl₃): d1.56(s, 3H) 1.62(s, 3H) 1.69(s, 3H) 1.73(s, 3H), 1.78(s, 3H), 1.90(s, 3H), 1.98-2.05(m, 11H), 2.46(brd, 1H), 4.44(brs, 2H), 4.55-4.70(d, 2H), 4.76-4.80(m, 1H), 5.07-5.11(m, 1H), 5.25(s, 1H), 5.29(s, 1H), 5.68(brs, 1H), 5.69(brs, 1H). MS *m*/*z* 521 [M+H]⁺

Example 47: Synthesis of Compound **47**

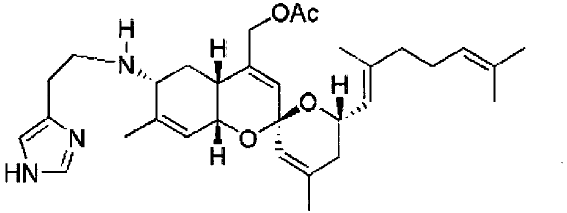

Compound **46** (15mg, 0.026mmol) was dissolved in THF (10ml), and then the temperature was lowered to 0°C. Histamine (59mg, 0.53mmol) was added, followed by stirring at 80°C for 7 hours. The layer separation was carried out by using ethylacetate (50ml) and water (3*10ml), followed by drying over Na₂SO₄. After distillation under reduced pressure, the obtained residue was purified by silica column chromatography, to obtain Compound **47.** MS *m*/*z* 536 [M+H]⁺

Example 48: Synthesis of Compound **48**

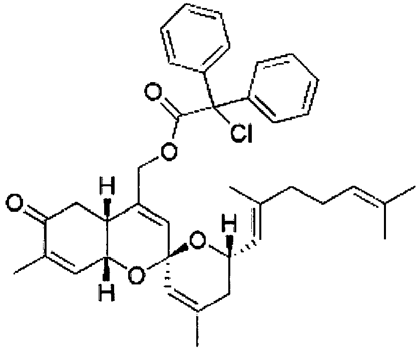

Phorbaketal A (5mg, 0.01mmol) and triethylamine (1ml) were dissolved in dichloromethane, and then 2-chloro-2,2-diphenylacetylchloride (100mg, 0.37mmol) was added, followed by stirring at room temperature for 10 hours. After the reaction was terminated, the layer-separation was conducted by using dichloromethane and water. The organic solvent layer was distilled under reduced pressure, and then the obtained residue was purified by silica column chromatography, to obtain Compound **48.** MS *m*/*z* 627 [M+H]⁺

Example 49: Synthesis of Compound **49**

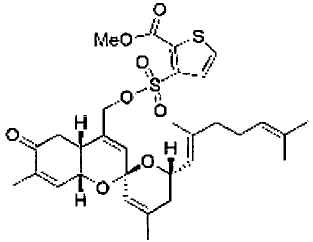

Phorbaketal A (10mg, 0.025mmol) and triethylamine (1ml) were mixed with anhydrous dichloromethane (10ml) at 0°C, and then 2-carbomethoxy-3-thiophene sulfonyl chloride (100mg, 0.41mmol) was added, followed by stirring at room temperature for 12 hours. The layer separation was carried out by using water (20ml) and dichloromethane (50ml), and the organic solvent layer was distilled under reduced pressure. The obtained residue was purified by silica column chromatography, to obtain Compound **49** (13mg, 89%). MS *m*/*z* 603 [M+H]⁺

Example 50: Synthesis of Compound **50**

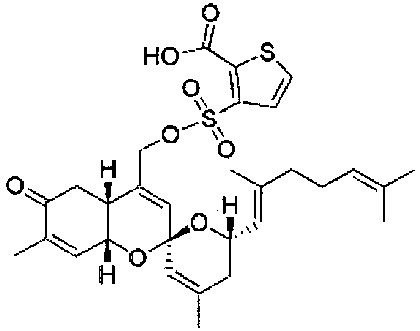

Compound **49** (10mg, 0.016mmol) was mixed with lithium hydroxide (1N, 10µℓ**)** dissolved in dioxane (10ml), followed by stirring at room temperature for 1 hour. After oxidation using an aqueous 2N HCl solution, separation by silica column chromatography was conducted to obtain Compound 50. MS *m*/*z* 589 [M+H]⁺

Example 51: Synthesis of Compound 51

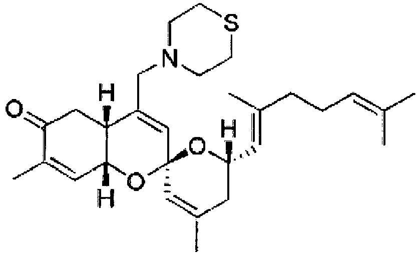

Phorbaketal A (10mg, 0.025mmol) and triethylamine (1ml) were mixed with anhydrous dichloromethane (10ml) at - 20°C, and then triflic anhydride (0.015ml, 0.088mmol) was added, followed by stirring for 30 minutes. The reaction liquid was diluted with dichloromethane (20ml), and washed with a cooled aqueous 1N HCl solution, NaHCO₃, salt water, and water. The organic solvent layer was separated, dried over Na₂SO₄ and distilled under reduced pressure. The residue was mixed with DIPEA (1ml) and THF (10ml), and then thiomorpholine (200mg) was added thereto at 0°C. After stirring for 4 hours, the layer separation was carried out by using ethylacetate (100ml) and water (3*10ml). The organic solvent layer was dried over Na₂SO₄, and then purified by silica column chromatography, to obtain Compound **51** (8mg, 71%). MS *m*/*z* 484 [M+H]⁺

Example 52: Synthesis of Compound 52

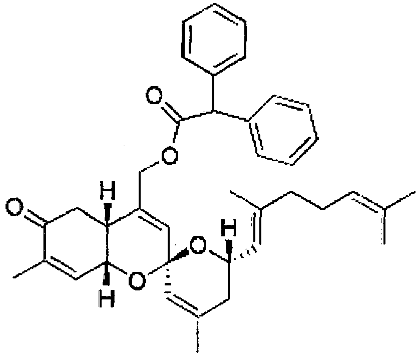

Phorbaketal A (20mg, 0.050mmol), together with triethylamine (1ml), was put in anhydrous dichloromethane (10ml) at 0°C, which was then allowed to react with diphenyl acetyl chloride (100mg, 0.43mmol), followed by stirring for 10 hours. The layer-separation was conducted by using water (20ml) and dichloromethane (30ml), followed by washing with salt water and water, drying over Na₂SO₄, distillation under reduced pressure, and separation by silica column chromatography, to obtain Compound **52.** MS *m*/*z* 593 [M+H]⁺

Example 53: Synthesis of Compound **53**

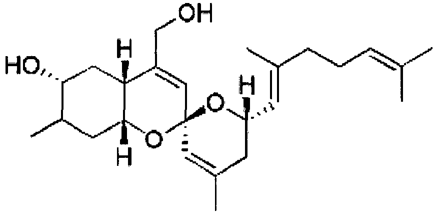

NaBH₄ (1mg, 0.033mmol) was mixed with phorbaketal A (10mg, 0.025mmol) and methanol (20ml) at 0°C. The reaction liquid was stirred for 4 hours, and then the solvent was distilled under reduced pressure. The residue was extracted with dichloromethane (3*20ml), followed by drying over Na₂SO₄ and then distillation under reduced pressure. The residue was purified by using silica column chromatography, to obtain Compound **53.** MS *m*/*z* 403 [M+H]⁺

Example 54: Synthesis of Compound **54**

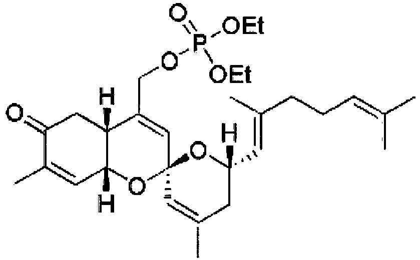

Phorbaketal A (12mg, 0.03mmol) and carbon tetrabromide (20mg, 0.36mmol), together with pyridine (0.5ml), were mixed with triethylphosphate (12µℓ, 0.075mmol) in anhydrous dichloromethane (5ml) at 0°C, and the mixture was allowed to react at room temperature for 9 hours. The reaction liquid was washed with 10% HCl and an aqueous saturated NaHCO₃ solution, and then the layer separation was carried out by using water and dichloromethane. The organic solvent layer was dried over Na₂SO₄. Purification using column chromatography was conducted to obtain Compound **54** (10mg, 63%). MS *m*/*z* 534 [M+H]⁺

Example 55: Synthesis of Compound **55**

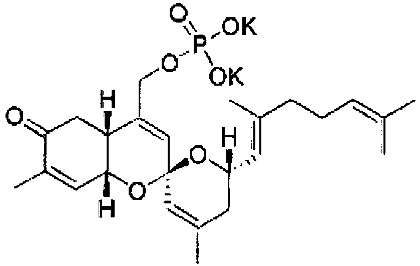

Compound **54** (9mg, 0.016mmol), together with 2,4,6-colidine (25µℓ) and bromotrimethylsilane (25µℓ, 0.16mmol), was allowed to react with anhydrous dichloromethane (3ml) at 0°C. After the reaction was allowed to proceed at room temperature for 15 hours, the solvent was removed, and then the residue was allowed to react with 2N NaOH (0.8ml) at room temperature for 4 hours. The solvent was removed, and column chromatography was used to obtain Compound **55** (7mg, 74%). MS *m*/*z* 555 [M+H]⁺

Example 56: Synthesis of Compound **56**

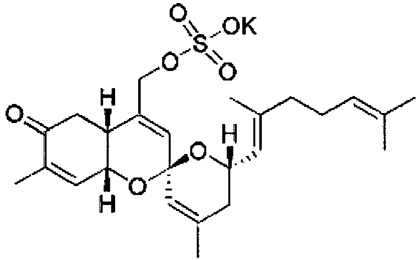

Phorbaketal A (20mg, 0.05mmol) and THF SO₃ pyridine complex (20mg, 0.13mmol) were slowly mixed with each other at 0°C. After stirring at room temperature for 1 hour, the reaction liquid was purified by silica gel column chromatography, to obtain Compound **56.** MS *m*/*z* 517 [M+H]⁺

Example 57: Synthesis of Compound 57

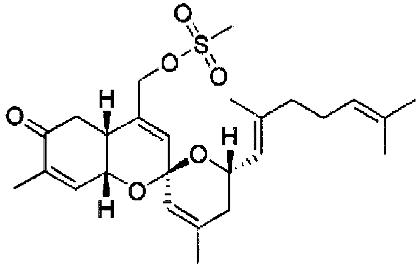

Phorbaketal A (15mg, 0.037mmol) and triethylamine (1ml) were mixed with anhydrous dichloromethane (10ml) at 0°C, and then methane sulfonyl chloride (8.6µℓ, 0.11mmol) was added, followed by stirring at room temperature for 1 hours. The layer separation was carried out by using water (20ml) and dichloromethane (20ml), and the organic solvent layer was distilled under reduced pressure. The obtained residue was purified by silica column chromatography, to obtain Compound 57. MS *m*/*z* 477 [M+H]⁺

Example 58: Synthesis of Compound 58

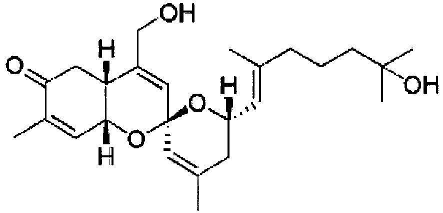

Phorbaketal A (10mg, 0.025mmol) and Hg(OAC)₃ were put and allowed to react with each other in a mixture solvent of THF (5ml) and water (1ml) for 2 hours. After THF was removed by distillation under reduced pressure, water (5ml) was added and NaOH (20mg) and NaBH₄ (910mg) were added, followed by reaction for 30 minutes. The layer separation was carried out by using water (10ml) and dichloromethane (20ml), and the organic solvent layer was distilled under reduced pressure. The obtained residue was purified by silica column chromatography, to obtain Compound 58. MS *m*/*z* 417 [M+H]⁺

Example 59: Synthesis of Compound 59

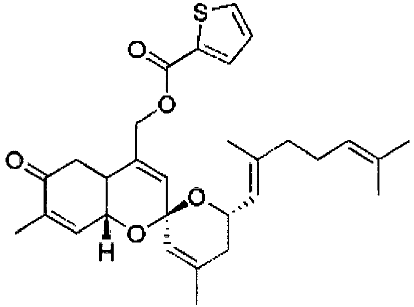

Phorbaketal A (15mg, 0.038mmol), 2-thiophene carbonylchloride (5.50mg, 1eq), and DIEA(N,N-diisopropylethylamine) (0.01mmol) were mixed with dichloromethane, followed by stirring at room temperature for 2 hours. Ice water (30 ml) was poured into the reaction liquid, and then reaction was terminated by using ethylacetate (2*25ml). The organic solvent layer was collected, and then dried by using anhydrous sodium sulfate and distilled under reduced pressure. The obtained residue was purified by silica column chromatography to obtain Compound **59** (16.5mg, 86%). ¹H-NMR (300 MHz, CDCl₃): d 1.60(3H, s) 1.68 (3H, s) 1.76 (3H, s) 1.78 (3H, s) 1. 84 (3H, s) 2.07-2.20(6H, m) 2.56-2.74(3H, t) 4.64(1H, s), 4.70-4.88(3H, t) 5.10(1H, s) 5.26(1H, d) 5.36(1H, s), 5.77(1H, s) 6.66(1H, s) 7.12(1H, m) 7.57(1H, d) 7.81(1H, s). MS *m*/*z* 509 [M+H]⁺

Example 60: Synthesis of Compound 60

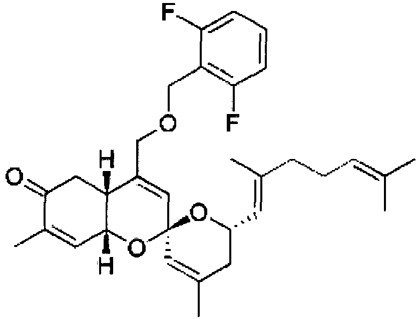

NaH (0.72mg, 1.2eq), phorbaketal A (10mg, 0.025mmol), and 2,2-difluorobenzylbromide (5.2mg, 1eq) were put in THF(5ml), followed by stirring at 0°C for 4 hours. The reaction was terminated by using water and ethylacetate (3*25ml), and the obtained organic solvent layer was dried by using sodium sulfate. After distillation under reduced pressure, the residue was purified by silica column chromatography, to obtain Compound **60** (11.1mg, 85%). ¹H-NMR (300 MHz, CDCl₃): *d*1.60(3H, s) 1.68(3H, s) 1.74(3H, s) 1.76(3H, s) 1.82(3H, s) 2.04-2.12(6H, m) 2.47-2.63(2H, t) 3.92-4.10(2H, m), 4.55(2H, s) 4.76(3H, t) 5.09(1H, s) 5.26(1H, d) 5.32(1H, s), 5.66(1H, s) 6.64(1H, s) 6.88(2H, m) 7.22(1H, m). MS *m*/*z* 525 [M+H]⁺

Example 61: Synthesis of Compound **61**

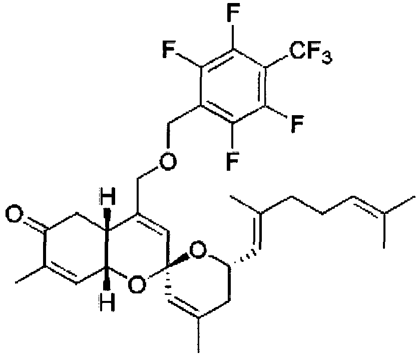

Compound **61** was obtained at a yield of 91% by employing the same synthesis method as Compound **60** while 2,3,5,6-tetrafluoromethyl-4-trifluoromethane-benzylbromide was used in stead of 2,2-difluorobenzylbromide. ¹H-NMR (300 MHz, CDCl₃): *d*1.61(3H,s) 1.68(3H, s) 1.75(3H, s) 1.76(3H, s) 1.82(3H, s) 2.08-2.11(6H, m) 2.51-2.76(3H, m) 4.47(2H, s) 4.53-4.64(3H, m), 4.80(1H, s) 5.10(1H, s) 5.27(1H, d) 5.35(1H, s), 5.78(1H, s) 6.68(1H, d). MS *m*/*z* 629 [M+H]⁺

Example 62: Synthesis of Compound **62**

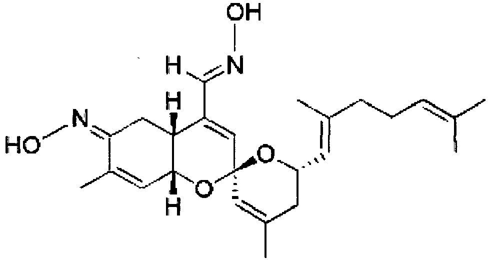

Compound **5** (10mg, 0.025mmol) was dissolved in an ethanol/water (7:3) mixture liquid (3ml), and then NH₂OHHCl (3.9mg, 2.2eq) and NaOH (4.0mg, 4eq) were put therein. Then, the reaction was allowed to proceed at room temperature for 2 hours. The reaction liquid was put in ice water (30ml) and the layer separation was carried out by using ethylacetate (2*25ml). The organic solvent layer was collected, and then dried by using NaSO₄. The organic solvent was distilled under reduced pressure, and the obtained residue was purified by column chromatography to obtain Compound **62.** MS m/z 427 [M+H]⁺

Example 63: Synthesis of Compound **63**

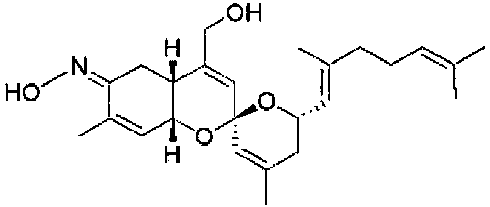

Phorbaketal acetate (10mg, 0.022mmol) was dissolved in an ethanol/water (7:3) mixture liquid (3ml), and then NH₂OHHCl (1.5mg, 1eq) and NaOH (1.8mg, 2eq) were put therein. Then, the reaction was allowed to proceed at room temperature for 2 hours. The reaction liquid was put in ice water (30ml) and the layer separation was carried out by using ethylacetate (2*25ml). The organic solvent layer was collected, and then dried by using Na₂SO₄. The organic solvent was distilled under reduced pressure, and the obtained residue was purified by column chromatography to obtain Compound **63.** MS *m*/*z* 414 [M+H]⁺

Example 64: Synthesis of Compound **64**

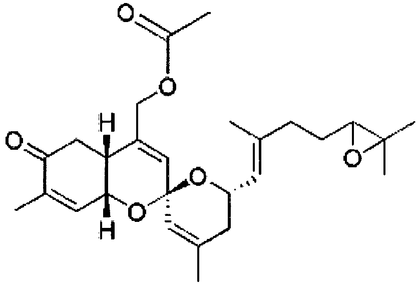

Phorbaketal acetate (10mg, 0.022mmol) was put in CH₂Cl₂ (5ml) at 0°C, and *m*-CPBA (meta-chloroperoxybenzoic acid) (3.9mg, 1eq) was dissolved in CH₂Cl₂ (6ml), which were then slowly mixed. After the reaction at room temperature for 3 hours, an aqueous saturated NaHCO₃ solution was added thereto, followed by stirring for 30 minutes. The layer separation was carried out by using CH₂Cl₂, and then the organic solvent layer was dried. After distillation under reduced pressure, the residue was separated by column chromatography, to obtain Compound **64.** MS *m*/*z* 457 [M+H]⁺

Example 65: Synthesis of Compound **65**

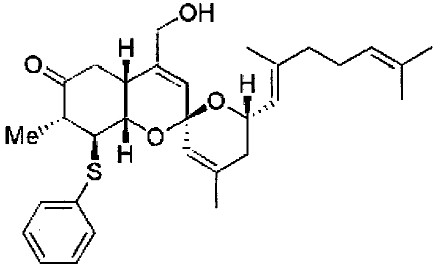

Compound **38** (6mg) was dissolved in methanol (2ml), which was then mixed with K₂CO₃ (1mg), followed by stirring for 1 hour. The reaction liquid was subjected to filtration, concentration, and silica column chromatography, to obtain Compound **65.** ¹H-NMR(300MHz, CDCl₃)d: 1.27(d, 3H), 1.50(s, 3H), 1.58(s, 3H), 1.65(s, 3H), 1.72(s, 3H), 1.80(s, 3H), 1.98-2.05(m, 2H), 2.11(s, 6H), 2.61(dd, 1H), 2.94(br ddd, 1H), 3.42-3.48(m, 1H), 3.78-3.81(m, 1H), 4.34(br s, 2H), 4.71-4.81(m, 1H), 5.03-5.12(m, 1H), 5.27(d, 1H), 5.52(s, 1H), 5.70(br s, 1H) 7.22-7.31(m, 5H). MS *m*/*z* 509 [M+H]⁺

Example 66: Synthesis of Compound **66**

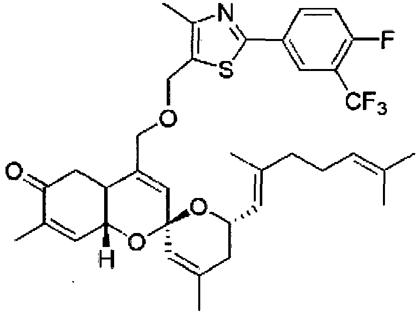

Sodium hydride (0.7mg, 1.2eq) was added to a THF (5ml) mixture liquid of phorbaketal A (10mg, 0.025mmol) and thiazole derivative 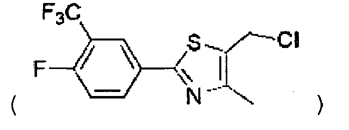 (7.72mg, 1eq)at 0°C, followed by stirring at room temperature for 4 hours. The layer separation was carried out by using water and ethylacetate (3*20ml), and the organic solvent layer was separated, and then dried over anhydrous Na₂SO₄. The solvent was removed by distillation under reduced pressure, and the residue was purified by chromatography to obtain Compound **66.** MS *m*/*z* 672 [M+H]⁺

Example 67: Synthesis of Compound **67**

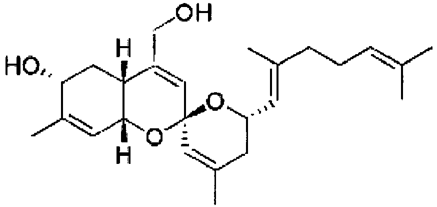

Phorbaketal acetate (20mg, 0.045mmol) was dissolved in THF (4ml), and then mixed with L-selectride (1.0M in THF, 0.07ml) under nitrogen conditions while the temperature was lowered to -78°C. After the reaction liquid was stirred at -78°C for 30 minutes, the temperature was raised to room temperature. An aqueous 10% NaOH solution was put in the reaction liquid, and then the organic solvent layer was separated. The aqueous layer was layer-separated by using ethylacetate (3ml*5), and the separated organic solvent layer was collected and then dried over anhydrous Na₂SO₄. After distillation under reduced pressure, the residual was purified by column chromatography, to obtain Compound **67.** MS *m*/*z* 401 [M+H]⁺

Example 68: Synthesis of Compound **68**

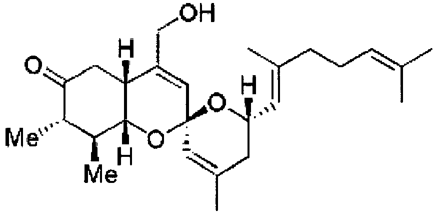

Copper cyanide (50mg, 0.56mmol) was dissolved in diethyl ether (10ml), and then mixed with MeLi (1.6M in diethyl ether, 23mg, 1.12 mol) at -10°C, followed by stirring for 1 hour. Compound **43** (15mg, 0.033mmol) wad added thereto, followed by stirring for 3 hours. After the layer separation was carried out by using an aqueous saturated ammonium chloride solution and ethylacetate, the organic solvent layer was dried, followed by separation by silica column chromatography, to obtain Compound **68.** MS *m*/*z* 415 [M+H]⁺

Example 69: Synthesis of Compound **69**

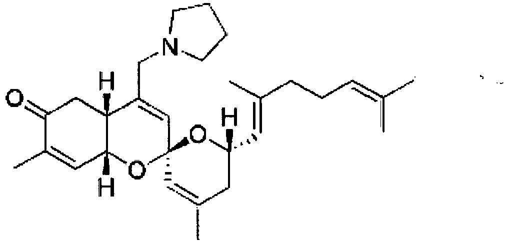

Compound 37 (6mg, 0.012mmol) and Cs₂CO₃ (50mg) were mixed with DMF (5ml)), and then, together with phenylpiperazine (1ml, 12.19mmol), stirred at 80°C for 6 hours. The solvent was removed by distillation under reduced pressure, and then the residue was purified by silica column chromatography, to obtain Compound 69 (3mg, 65%). MS *m*/*z* 452 [M+H]⁺

Example 70: Synthesis of Compound 70

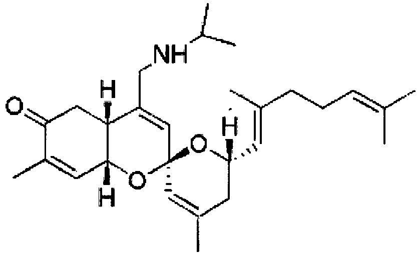

Compound 37 (6mg, 0.012mmol) and Cs₂CO₃ (50mg) were mixed in isopropylamine (5ml, 12.19mmol), and the mixture was stirred for 6 hours while being heated. The solvent was removed by distillation under reduced pressure, and then the residue was purified by silica column chromatography, to obtain Compound 70. MS *m*/*z* 440 [M+H]⁺

Example 71: Synthesis of Compound 71

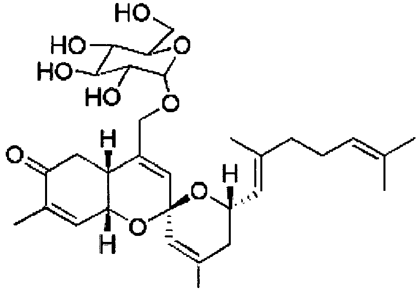

Phorbaketal (20mg, 0.05mmol), α-D-glucopyranocyl bromide tetrabenzoate (82mg, 0.125mmol), and molecular sieve (0.5g) were mixed with dichloromethane, and then silver triplet (10mg, 0.03mmol) was slowly mixed therewith at 0°C. The mixture was stirred at room temperature for 8 hours, followed by filtration, and washing with water, and distillation under reduced pressure. The obtained residue was purified by silica column chromatography to obtain Compound 71. MS m/z 561 [M+H]⁺

Example 72: Synthesis of Compound 72

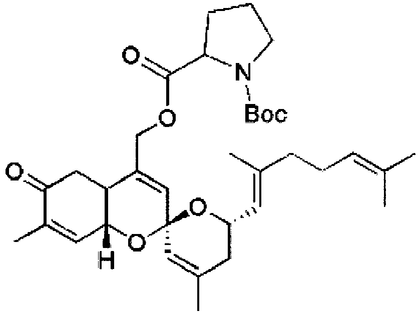

Phorbaketal (10mg, 0.025mmol), DCC(N,N'-dicyclohexylcarbodiimide) (6.18mg, 0.030mmol), and N-boc proline (0.62mg, 0.025mmol) were mixed with dichloromethane (5 ml) while 4-dimethylaminopyridine (DMAP) functioning as a catalyst was put therein, and then the stirring was conducted until an ester structure was completed at room temperature. The reaction liquid was washed to remove *N, N-*dicyclohexyl urea. The residue was washed with water (3*30ml) and 5% acetic acid (3*30ml) and again washed with water (3*30ml), and then dried over anhydrous NaSO₄. The solvent was removed, and the residue remaining after distillation under reduced pressure was purified by column chromatography, to obtain Compound 72. MS *m*/*z* 596 [M+H]⁺

The following compounds (Compounds 73-81) were separated and purified from Phorbas sponge by the following procedure. The sponge was freeze-dried, and then the dry weight 500g was subjected to extraction with a methanol and CH₂Cl₂ (1:1) mixture solution. The extraction liquid was layer-separated by using water and CH₂Cl₂. The organic solvent layer was layer-separated by using an aqueous 90% methanol solution and n-hexane. The aqueous 90% methanol solution layer was fractionized by using reverse phase silica column chromatography. Hereinafter, specific separation conditions of the examples are as follows.

**[Table 1]**

| Compound No. | Column 1, r.t (min) | Column 2, r.t (min) |
|---|---|---|
| 73 | 33 | 55 (45% MeCN in H₂O) |
| 74 | 12.5 | 49 (45% MeCN in H₂O) |
| 75 | 33 | 56 (48% MeCN in H₂O) |
| 76 | 44 | |
| 77 | 25.5 | |
| 78 | 66 | |
| 79 | 44 | 53 (48% MeCN in H₂O) |
| 80 | 28 | 44 (43% MeCN in H₂O) |
| 81 | 28 | 47 (43% MeCN in H₂O) |
| Column: HPLC column 1 [Luna C18 AXIA, 250x21 mm, separation solvent conditions [aqueous 50% MeCN solution, flow rate 8ml/min], HPLC column 2. [Shiseido C18 MGII, 10×250 mm] elution rate [2ml/min] | | |

Example 73: Compound 73

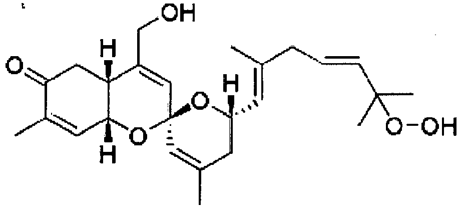

¹H-NMR (300MHz, CDCl₃) d: 1.29 (s, 6H), 1. 75 (s, 3H), 1.76(s, 3H) 1.81(s, 3H), 1.87(m, 1H), 2.04(m, 1H), 2.43(m, 1H), 2.57(s, 1H), 2.59(s, 1H), 2.77(s, 1H), 4.06(s, 2H), 4.49(m, 1H), 4.75(s, 1H), 5.27(d, 1H), 5.29(s, 1H), 5.54(s, 1H), 5.61(d, 1H), 5.66(d, 1H), 6.70(m, 1H); MS *m*/*z* 431 [M+H]⁺

Example 74: Compound 74

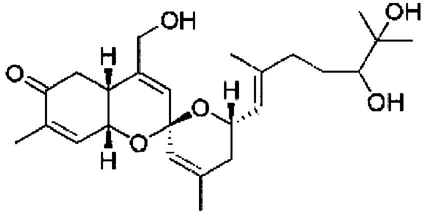

¹H-NMR(300MHz, CDCl₃)d: 1.14 (s, 3H), 1.17 (s, 3H), 1.40(d, 1H), 1.76(s, 3H), 1.78(m, 1H), 1.81(s, 3H), 1.82(s, 3H), 1.87(m, 1H), 2.05(m, 1H), 2.08(m, 1H), 2.19(m, 1H), 2.33(m, 1H), 2.44(m, 1H), 2.58(m, 1H), 2.59(m, 1H), 3.27(m, 1H), 4.07(s, 2H), 4.51(m, 1H), 4.77(m, 1H), 5.28(d, 1H), 5.30(s, 1H), 5.55(s, 1H), 6.71(m, 1H); MS *m*/*z* 433 [M+H]⁺

Example 75: Compound 75

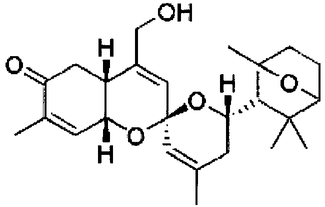

¹H-NMR(300MHz, CDCl₃)d: 1.11(s, 1H), 1.18 (s, 1H), 1.29(s, 1H), 1.44(m, 1H), 1.51(s, 1H), 1.67(s, 1H), 1.69(s, 1H), 1.76(s, 1H), 1.81(s, 1H), 1.83(m, 1H), 2.01(s, 1H), 2.02(s, 1H), 2.44(m, 1H), 2.59(m, 1H), 2.61(s, 1H), 2.63(s, 1H), 4.00(s, 1H), 4.08(m, 2H), 4.49(m, 1H), 5.24(s, 1H), 5.51(s, 1H), 6.66(m, 1H); MS *m*/*z* 415 [M+H]⁺

Example 76: Compound 76

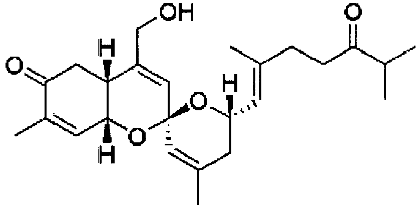

¹H-NMR(300MHz, CDCl₃)d: 1.07(s, 3H), 1.08(s, 3H) 1.76(s, 3H), 1.80(s, 3H), 1.82(s, 3H), 1.86(m, 1H), 2.01(m, 1H), 2.03(s, 1H), 2.04(m, 1H), 2.28(m, 2H), 2.44(m, 1H), 2.59(m, 1H), 2.60(s, 1H), 2.67(m, 2H), 4.05(s, 1H), 4.08(s, 2H), 4.50(m, 1H), 4.74(m, 1H), 5.24(m, 1H), 5.30(s, 1H), 5.54(s, 1H), 6.71(m, 1H); MS *m*/*z* 415 [M+H]⁺

Example 77: Compound 77

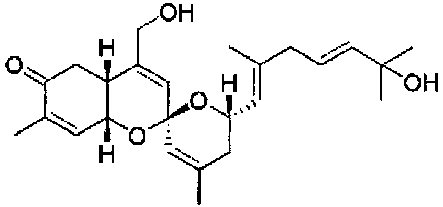

¹H-NMR(300MHz, CDCl₃)d: 1.27(s, 6H), 1.75(s, 3H), 1.76(s, 3H), 1.81(s, 3H), 1.86(m, 1H), 2.04(m, 1H), 2.43(m, 1H), 2.57(m, 1H), 2.58(m, 1H), 2.75(d, 1H), 4.06(br, 2H), 4.49(m, 1H), 4.75(m, 1H), 5.27(d, 1H), 5.29(s, 1H), 5.53(s, 1H), 5.60(m, 1H), 5.66(m, 1H), 6.09(m, 1H); MS *m*/*z* 415 [M+H]⁺

Example 78: Compound 78

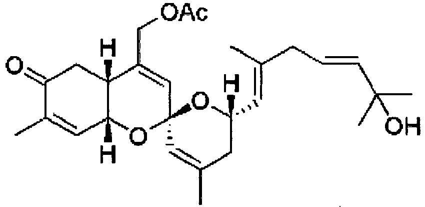

¹H-NMR(300MHz, CDCl₃)d: 1.27(s, 6H), 1.75(s, 3H), 1.76(s, 3H), 1.81(s, 3H), 1.86(m, 1H), 2.04(m, 1H), 2.43(m, 1H), 2.57(m, 1H), 2.58(m, 1H), 2.75(d, 1H), 4.49(m, 1H), 4.60(m, 2H), 4.75(m, 1H), 5.27(d, 1H), 5.29(s, 1H), 5.59(s, 1H), 5.60(m, 1H), 5.66(m, 1H), 6.09(m, 1H); MS *m*/*z* 457 [M+H]⁺

Example 79: Compound 79

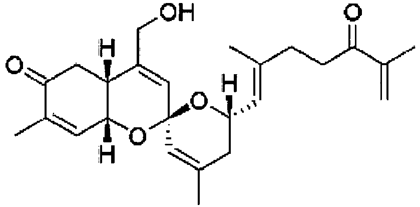

¹H-NMR(300MHz, CDCl₃)d: 1.76 (s, 3H), 1.80 (s, 3H), 1.82(s, 3H), 1.86(s, 3H), 1.86(m, 1H), 2.01(m, 1H), 2.03(s, 1H), 2.04(m, 1H), 2.34(t, 2H), 2.44(m, 1H), 2.59(m, 1H), 2.60(s, 1H), 2.90(s, 2H), 4.05(s, 2H), 4.08(s, 2H), 4.50(m, 1H), 4.74(m, 1H), 5.24(m, 1H), 5.30(s, 1H), 5.54(s, 1H), 5.86(d, 1H), 6.09(s, 1H), 6.71(m, 1H); MS *m*/*z* 413 [M+H]⁺

Example 80: Compound 80

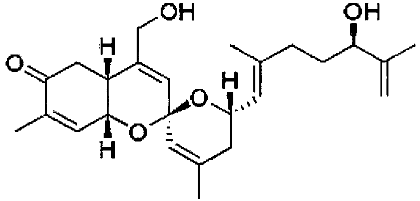

¹H-NMR(300MHz, CDCl₃)d: 1.65(m, 2H), 1.71 (s, 3H), 1.75(s, 3H), 1.79(s, 3H), 1.81(s, 3H), 1.86(m, 1H), 2.04(m, 1H), 2.11(m, 2H), 2.43(m, 1H), 2.57(m, 1H), 2.58(m, 1H), 3.99(m, 1H), 4.06(br, 2H), 4.49(m, 1H), 4.75(m, 1H), 4.82(s, 1H), 4.92(s, 1H), 5.27(d, 1H), 5.29(s, 1H), 5.53(s, 1H), 6.09(m, 1H); MS *m*/*z* 415 [M+H]⁺

Example 81: Compound 81

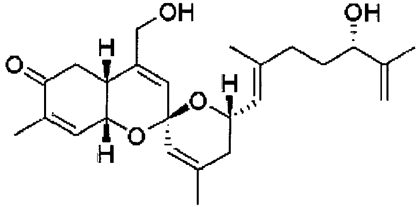

¹H-NMR(300MHz, CDCl₃)d: 1.65(m, 2H), 1.71 (s, 3H), 1.75(s, 3H), 1.79(s, 3H), 1.81(s, 3H), 1.86(m, 1H), 2.04(m, 1H), 2.11(m, 2H), 2.43(m, 1H), 2.57(m, 1H), 2.58(m, 1H), 3.99(m, 1H), 4.06(br, 2H), 4.49(m, 1H), 4.75(m, 1H), 4.82(s, 1H), 4.92(s, 1H), 5.27(d, 1H), 5.29(s, 1H), 5.53(s, 1H), 6.09(m, 1H); MS *m*/*z* 415 [M+H]⁺

**Example 82: Verification on Activity Efficacy and Measurement of Selectivity of Compounds of Chemical Formula I According to Present Invention with respect to Nuclear Receptor (Nurr1)**

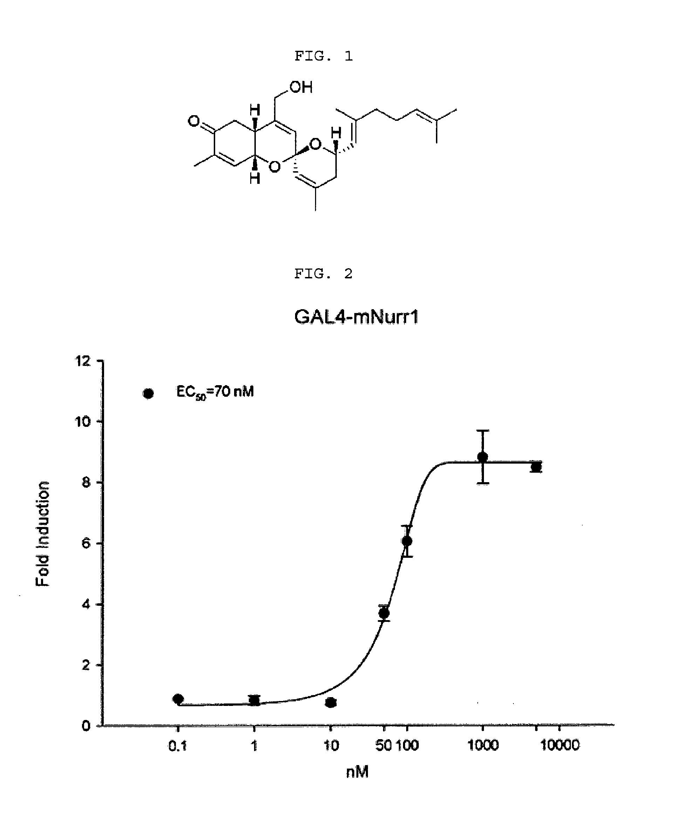

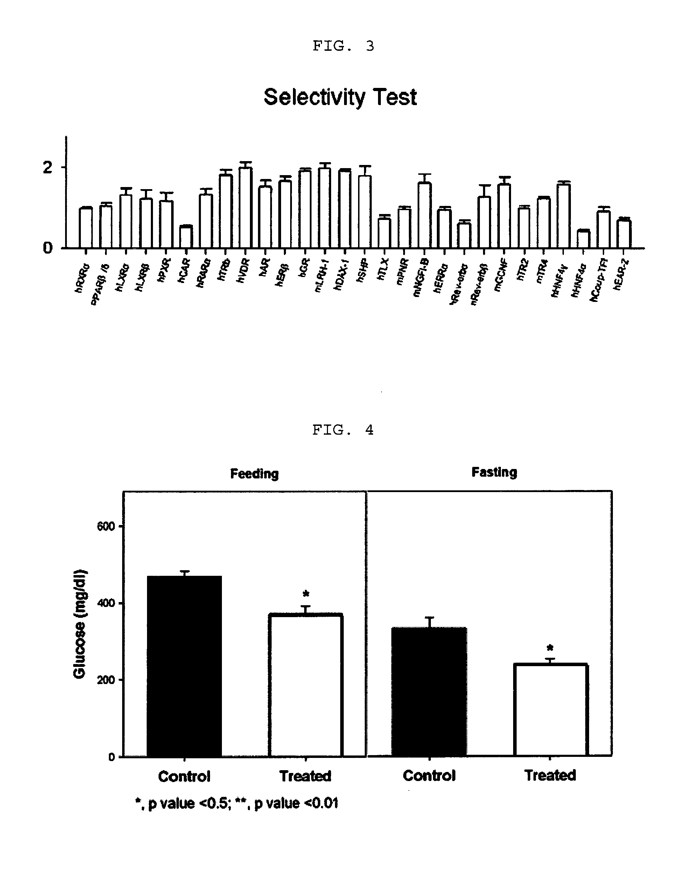

Animal cell line CV-1 was used in transfection search. Cells were cultured in DMEM medium within a cell incubator containing 5% carbon dioxide at 37°C. The medium contained 10% fetal bovine serum (FBS), 100 U/ml penicillin, and 100*µ*g/ml streptomycin. On day 1 of the experiment, CV1 cells were seeded in a 96-well plate at 5,000cells/well. On day 2, the seeded cells were transfected with plasmid expressing GAL-mNurr1, plasmid expressing luciferase gene, and plasmid expressing β-galactosidase by using a transfection reagent, Superfect (QIAGEN). After 16 hours, the transfected cells were treated with compounds of Chemical Formula I dissolved in dimethylsulfoxide (DMSO) by the concentrations. Cells treated with dimethylsulfoxide having the final concentration of 1% were used as a negative control group. After culturing for 24 hours, the cells were lysed by using a lysis buffer, and luciferin was added thereto to measure the luciferase activity by a luminometer. After addition of an ONPG reagent, β-galactosidase activity was measured by an ELISA reader. The measured luciferase value was corrected by the activity value of β-galactosidase. The experiment results showed that CMDD-X (FIG. 1) had an EC₅₀ value of 70 nM on Nurr1 (FIG. 2). Compound 1, Compound 4, Compound 7, and Compound 8 showed EC₅₀ values of 28 nM, 7 nM, 32 nM, and 25 nM, respectively. Other compounds of Chemical Formula I showed similar activity values similar to that of CMDD-X. In addition, in order to measure selectivity on various nuclear receptors (RXR, PPAR, PXR, CAR, ER, LRH-1, GR, NGFI-B, ERR, Rev-erb, GCNF, TR, HNF4, COUP, EAR, VDR, AR, DAX-1, TLX, and VDR), activities on various nuclear receptors were measured by employing the same method. CMDD-X never showed activity on the other nuclear receptors, and thus exhibited excellent selectivity (FIG. 3). Other compounds of Chemical Formula I never showed activities on the other nuclear receptors, and thus exhibited excellent selectivity. Accordingly, it was verified that the compounds of Chemical Formula I were compounds selective to Nurr1.

**Example 83: Verification on Activity Efficacy and Measurement of Selectivity of Compounds of Chemical Formula I According to Present Invention on Nuclear Receptor (LXR)**

Animal cell line CV-1 was used in transfection search. Cells were cultured in DMEM medium within a cell incubator containing 5% carbon dioxide at 37°C. The medium contained 10% fetal bovine serum (FBS), 100 U/ml penicillin, and 100*µ*g/ml streptomycin. On day 1 of the experiment, CV1 cells were seeded in a 96-well plate at 5,000cells/well. On day 2, the seeded cells were transfected with plasmid expressing GAL-hLXRa or GAL-hLXRß, plasmid expressing luciferase gene, and plasmid expressing β-galactosidase, by using a transfection reagent, Superfect (QIAGEN). After 16 hours, the transfected cells were treated with compounds of Chemical Formula I dissolved in dimethylsulfoxide (DMSO) by the concentrations (here, the final concentration of the LXR agonist, T0901517, was 500 nM). Cells treated with dimethylsulfoxide having the final concentration of 1% were used as a negative control group. After culturing for 24 hours, the cells were lysed by using a lysis buffer, and luciferin was added thereto to measure the luciferase activity by a luminometer. After addition of an ONPG reagent, β-galactosidase activity was measured by an ELISA reader. The measured luciferase value was corrected by the activity value of β-galactosidase. The experiment results showed that CMDD-X had an IC₅₀ value of 20 nM on LXR. In addition, Compound 1, Compound 4, Compound 7, and Compound 8 showed IC₅₀ values of 21 nM, 5 nM, 11 nM, and 85 nM, respectively. Other compounds of Chemical Formula I also showed similar antagonistic activity values similar to that of CMDD-X. In addition, in order to measure selectivity on various nuclear receptors (RXR, PPAR, PXR, CAR, ER, LRH-1, GR, NGFI-B, ERR, Rev-erb, GCNF, TR, HNF4, COUP, EAR, VDR, AR, DAX-1, TLX, and VDR), antagonistic activity on various nuclear receptors were measured by employing the same method (only agonists for respective nuclear receptors were exchanged). CMDD-X never showed antagonistic activity on the other nuclear receptors, and thus exhibited excellent selectivity. Other compounds of Chemical Formula I never showed antagonistic activities on the other nuclear receptors, and thus exhibited excellent selectivity. Accordingly, it was verified that the compounds of Chemical Formula I are antagonist compounds selective to LXR.

**Example 84: Verification on Anti-diabetic Efficacy of Compounds of Chemical Formula I**

In the present example, in order to verify efficacy to prevent and treat diabetes of the compound of Chemical Formula I (CMDD-X or Compound 7) according to the present invention, BKS.Cg-+Lepr^{db}/+Lepr^{db} *(db*/*db)* mice as an insulin-independent diabetes animal model and general mice (C57BL/6J) fed with high-fat feedstuff were used. After the disease animal model was orally administered with CMDD-X, the diabetes-related index between the administered group and the control group was measured to verify the efficacy as an agent for preventing and treating diabetes. BKS.Cg-+Lepr^{db}/+Lepr^{db} (db/db) mice (male, 7-week old) as type 2 diabetes animal model were purchased and used. The experiment animals were adapted to a housing environment for 1 week while pellet type general diet (lab-chow) was supplied, and then divided into 2 groups of 18 mice each by randomized block design to have similar blood sugar and body weight. For general mice fed with high-fat feedstuff as another animal model, 6-week old C57BL/6J mice was purchased, and then were adapted to a lap environment for 2 weeks. After that, high-fat diet containing 35% fat diet (weight ratio, Research Diets. Co. Ltd.) was supplied for 14 weeks. The efficacy to prevent and treat diabetes by CMDD-X was observed through oral administration. A mouse fed with only 0.5% carboxylmethyl cellulose, as a medicine delivery, was used as a negative control group. The medicine was orally administered to the *db*/*db* mice for 4 weeks, a total of 28 days, and to the C57BL/6J mice, which was fed with the high-fat diet, for the last 6 weeks, at a dose of 10 mg/kg/day. The experiment animals after administration were fasted for 15 hours before autopsy, and then the blood taken from the orbital venous treated with heparin. After that, the plasma was separated by centrifugation of 1,000×g at 4°C for 15 minutes, and then refrigerated at -70°C until analysis was conducted to measure the insulin and Adiponectin concentrations. The pancreatic tissue was fixed in 10% formaldehyde solution containing PBS. The other organ tissues were rapidly cooled in the liquid nitrogen immediately after removal thereof, and kept at -70°C and then analyzed. T-test was conducted with respect to the control group and the administered group, a significant difference of less than 5% (P<0.05) was determined as having have a statistical significance.

In order to measure the blood sugar reduction efficacy of the compound of Chemical Formula I (CMDD-X or Compound 7), the blood was collected through tail veins of the *db*/*db* mice before experiment ending, after feeding, and after stomach was empty for 15 hours, and the blood sugar was measured by using a glucose oxidase method. After food intake, the blood sugar of the CMDD- X-administered group was lower than that of the control group by 23.6%, and the blood sugar at the time of an empty stomach was reduced by about 26.3% (FIG. 4). In the Compound 7- administered group, the blood sugar after food intake and the blood sugar at the time of an empty stomach were 27% and 28%, respectively.

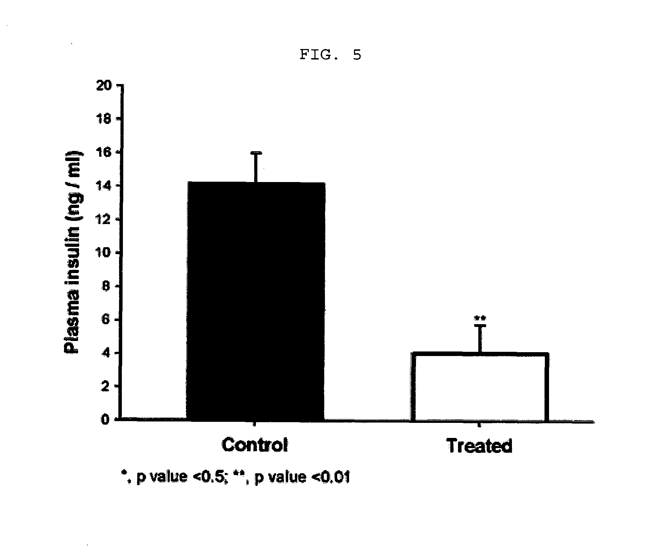

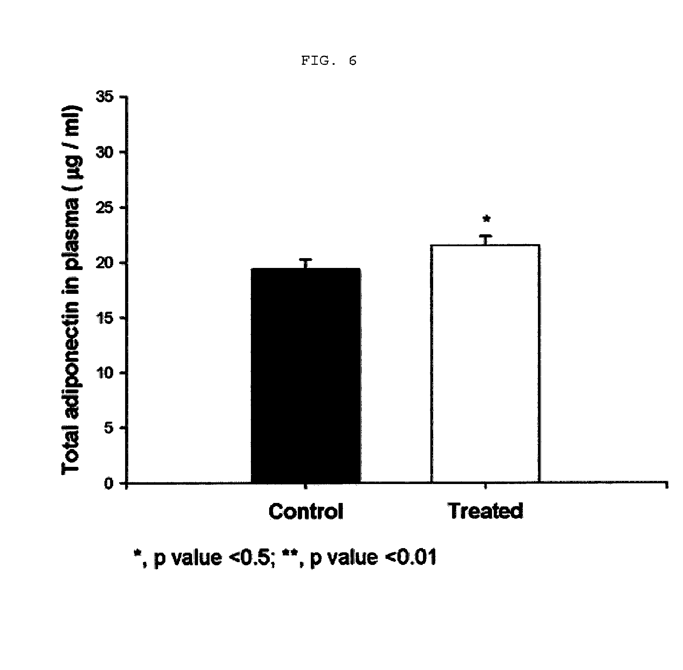

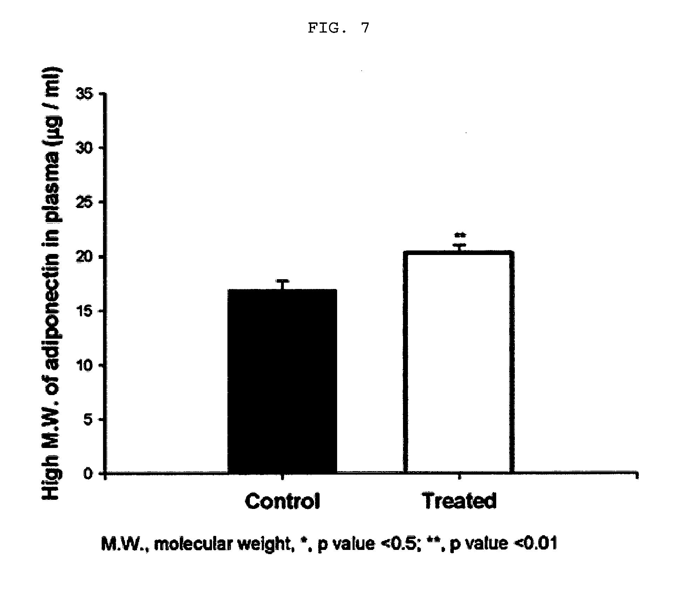

The insulin and adiponectin concentrations of the plasma, obtained from the orbital venous of the *db*/*db* mice orally administered with CMDD-X (or Compound 7) for 4 weeks, were measured by using an Elisa Kit. The plasma insulin concentrations of the control group and the CMDD-X-administered group were shown in FIG. 5. The blood insulin concentration of the CMDD-X-administered group was significantly reduced to 70% that of the control group. The blood insulin concentration of the Compound 7-administered group was also significantly reduced to 73% that of the control group. The total amount of Adiponectin, which is a molecular marker confirming improvement in diabetes of the CMDD-X-administered group, was significantly increased (p<0.05, FIG. 6). Moreover, the Adiponectin polymer having a substantially physiological function showed a statistically significant increase (p<0.01, FIG. 7). Therefore, it was verified that CMDD-X and Compound 7 have superior efficacy in insulin control and blood sugar drop.

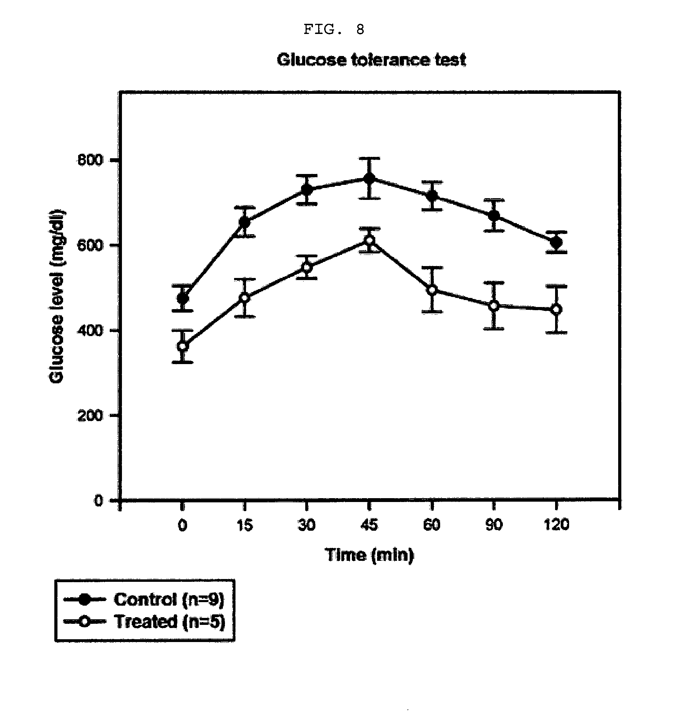

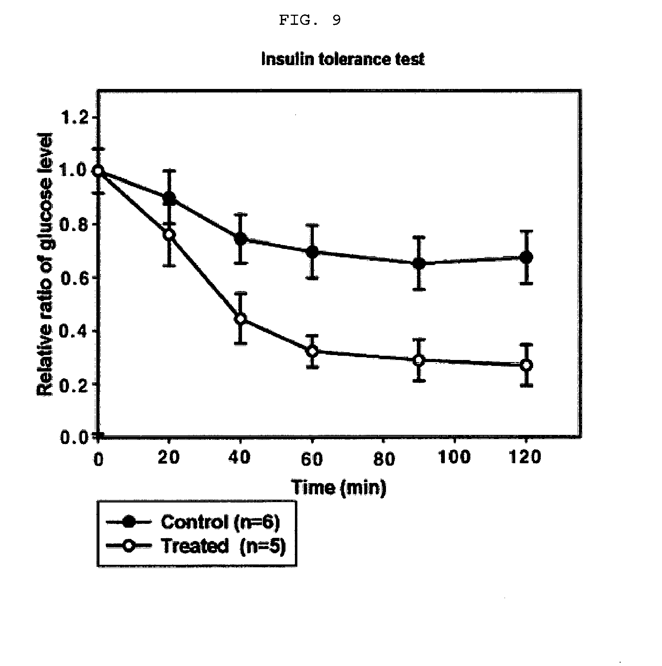

On the end day of the experiment, the *db*/*db* mice were fasted for 15 hours, and then intraperitoneally administered with a glucose solution of 1g per 1 kg body weight. The blood was collected from the tails of the *db*/*db* mice according to the time. The results of measurement of blood sugar by the times of the CMDD-X-administered group and the control group were shown in FIG. 8. As experimental results, the blood sugar level of the CMDD-X-administered group was increased 45 minutes before and after sugar administration, and significantly decreased at 120 minutes. Whereas, in the control group, the glucose sugar level was not recovered to a level before glucose administration even at 120 minutes. The results of the insulin tolerance test (3unit/kg) of the CMDD-X-administered group were shown in FIG. 9. The blood sugar of the CMDD-X-administered group was significantly decreased as compared with the control group for 40 minutes after insulin administration, and the blood sugar tended to be very effectively decreased as compared with the control group. The final blood sugar of the CMDD-X-administered group was decreased as compared with the control group by 57%. The final blood sugar of the Compound 7-administered group was decreased as compared with the control group by 58%.

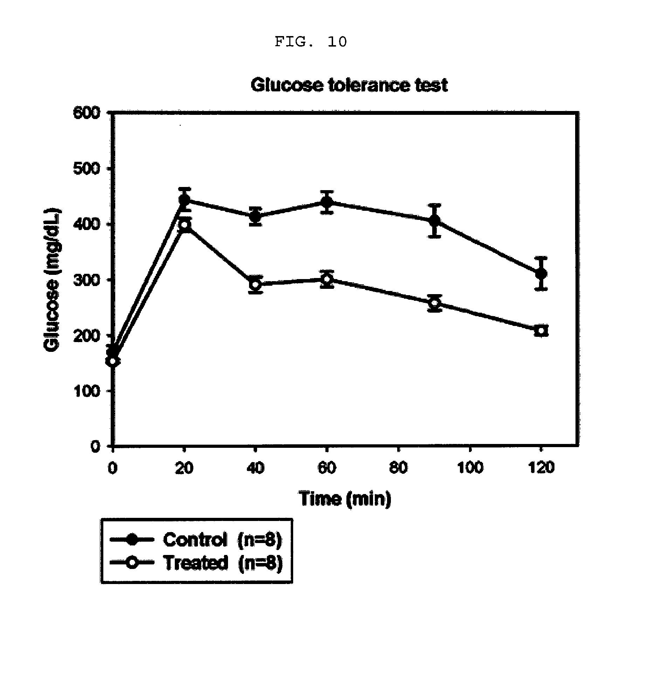

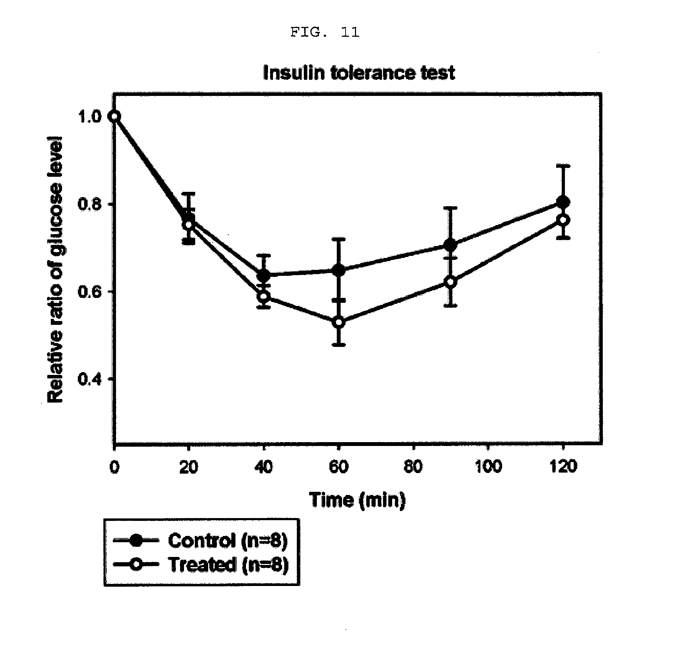

On the end day of experiment, the disease animal with diabetes induced by high-fat diet were also fasted for 15 hours in the same manner as the *db*/*db* mice, and then intraperitoneally administered with a glucose solution of 1g per 1 kg body weight. The blood was collected from the tails of the mice according to the time. The results of checking of the blood sugar level by the times of the CMDD-X-administered group and the control group were shown in FIG. 10. As a result of the experiment, it appeared that the blood sugar of the CMDD-X-administered group was more promptly recovered as compared with the control group. Likewise, the results of the insulin tolerance test (1 unit/kg) of the CMDD-X-administered group were shown in FIG. 11. It was confirmed that the blood sugar of the administered group was significantly decreased for 40 minutes after insulin administration as compared with the control group, and the blood sugar was promptly decreased and then promptly recovered as compared with the control group. Even in the case of administration of Compound 7, similar efficacy was exhibited.

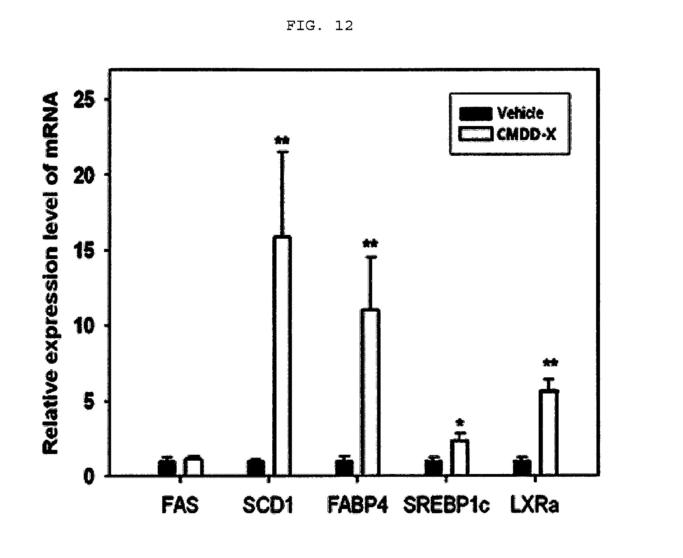

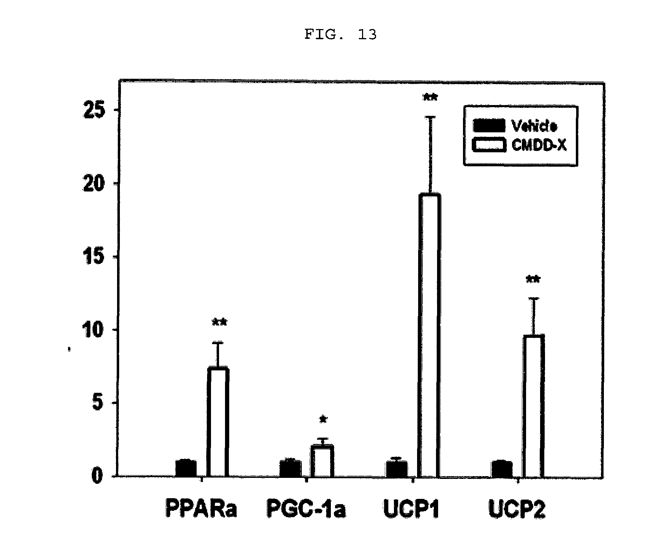

Gene expression analysis was conducted to establish the mechanism. The gene analysis was conducted by using micro-array and the Q-RT PCR method. In the mice fed with CMDD-X (or Compound 7), expression of Gyk as a glycolysis biomarker was increased (1.3 times), and expressions of LXR, SREBP1c, and SCD1, which are genes of synthesizing fatty acid by using decomposed glucose, were very increased (FIG. 12). Expressions of PPARa and UCP, which are genes for burning the newly generated fat to release heat, were increased (FIG. 13). Therefore, CMDD-X increased Adiponectin, to thereby decompose glucose and transform it into fat, and then again decompose the fat to release heat, and thus exhibited anti-diabetes efficacy. The mice fed with Compound 7 also showed the same mechanism.

**Example 85: Verification on Efficacy of Compounds of Chemical Formula I According to Present Invention on Renal Failure as a Diabetes Complication**

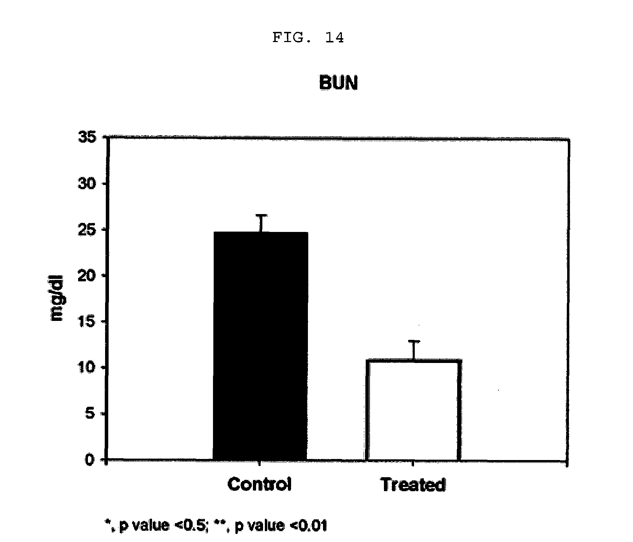

In order to verify the efficacy to treat renal failure as a diabetes complication, a *db*/*db* disease animal model was used. 8-week old *db*/*db* mice were orally administered with the compound of Chemical Formula I (CMDD-X or Compound 7) at a dose of 10 mg/kg/day for 4 weeks, and then the blood urea nitrogen as a marker to treat renal failure was measured. The results of the CMDD-X-administered group were shown in FIG. 14. Compound 7 showed somewhat superior efficacy as compared with CMDD-X. Therefore, the compound of Chemical Formula I showed superior efficacy on renal failure as a diabetes complication.

**Example 86: Verification on Fatty Liver Treatment Efficacy of Compound of Chemical Formula I According to Present Invention**

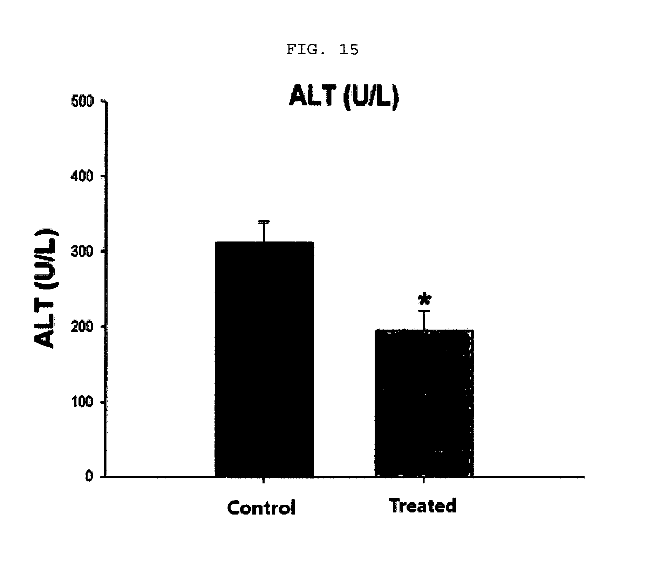

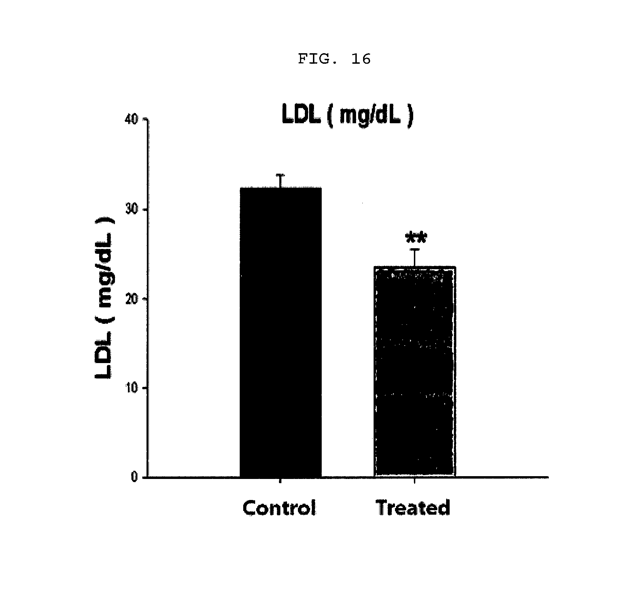

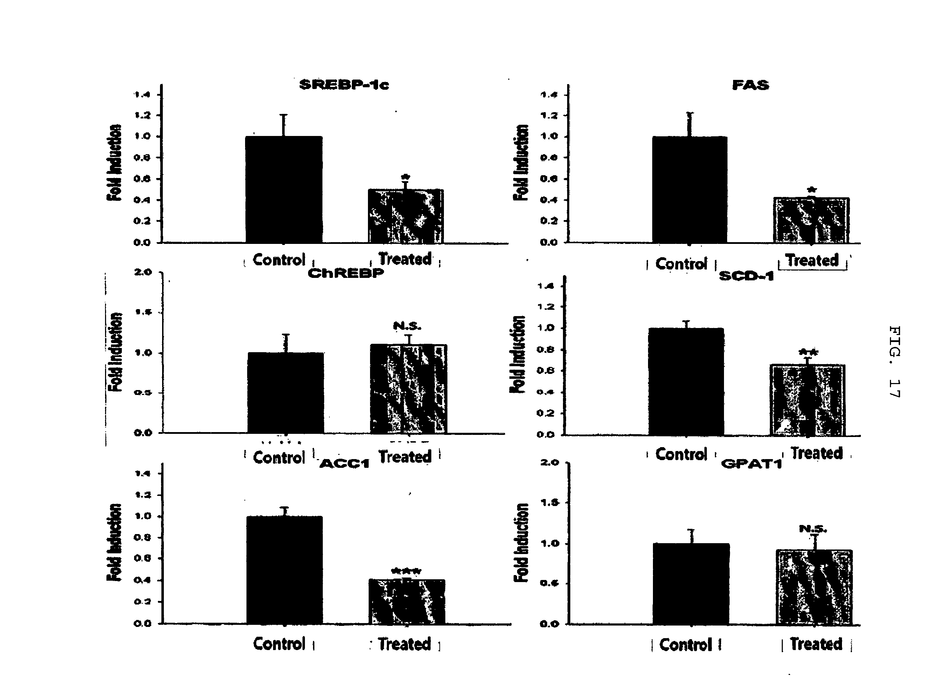

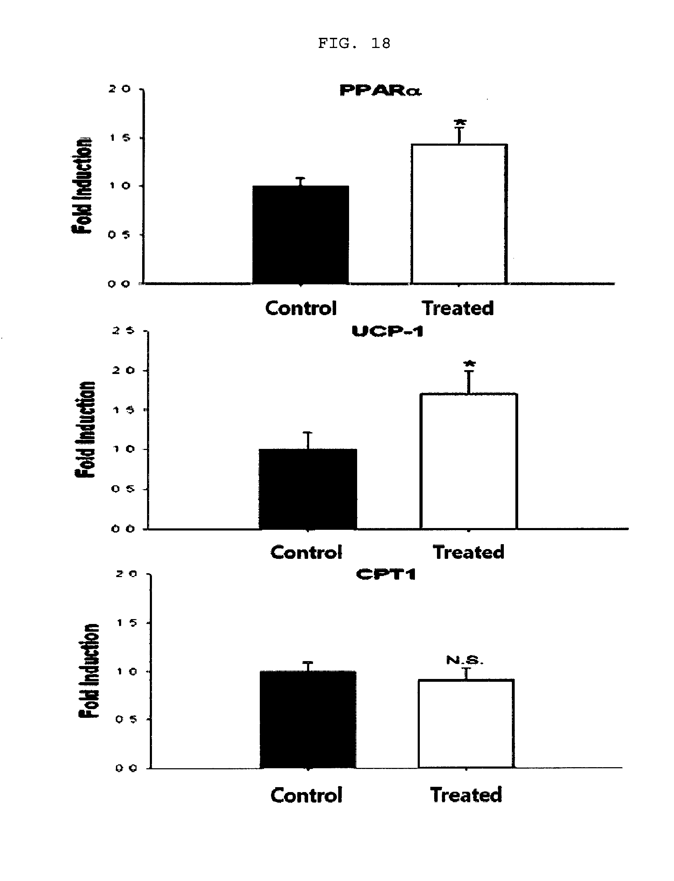

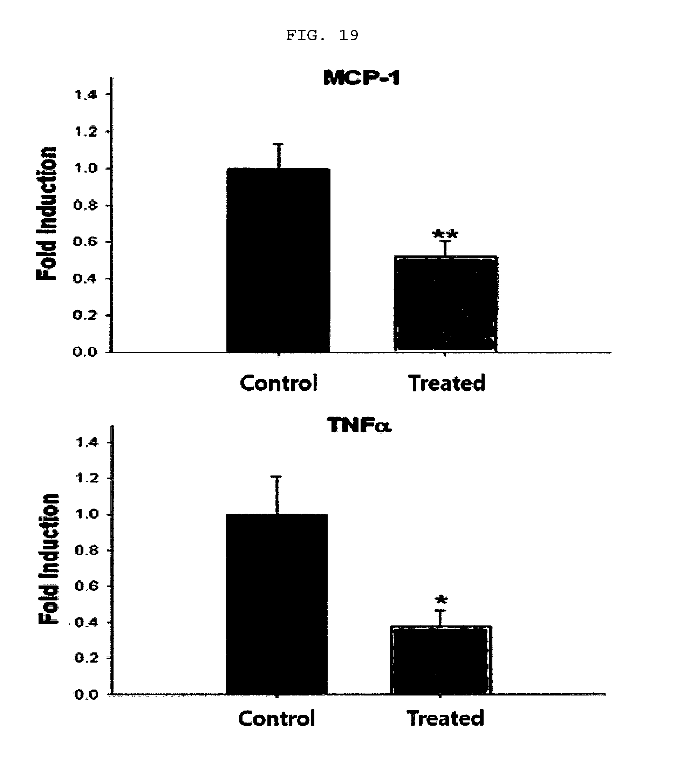

In order to verify efficacy to prevent and treat fatty liver of the compounds according to the present invention, a fatty liver occurrence model by a medicine, a fatty liver occurrence model by high fat, and a fatty liver occurrence model by methione-deficient diet (MCD) were used. The mice fed with general diet (chow diet) was simultaneously administered with a nuclear receptor LXR agonist (T0901317) to induce fatty liver, and then the efficacy to prevent and treat fatty liver by the compound of Chemical Formula I was tested. After the disease animal model was orally administered with CMDD-X (or Compound 7), the fatty liver-related index between the administered group and the control group was measured to verify the efficacy to prevent and treat fatty liver. The mouse fed with only 0.75% carboxylmethyl cellulose, as a medicine delivery, was used as a negative control group. First, the C57BL/6J mouse fed with general feedstuff was orally administered with the LXR agonist [T0901317 (10 mg/kg)] once per day for 5 days, to thereby induce fatty liver. In addition, the compound of Chemical Formula I (CMDD-X and Compound 7, 10mg/kg) and the LXR agonist were simultaneously administered, to verify efficacy to prevent and treat fatty liver. The C57BL/6J mouse was fed with 35% high-fat feedstuff for 8 weeks, to induce fatty liver, and then CMDD-X (or Compound 7), together with high-fat diet, was orally administered at a dose of 10 mg/kg/day for 6 weeks. The ob/ob animal model was fed with MCD diet for 4 weeks to induce fatty liver and, at the same time, was orally administered with the compound of Chemical Formula I (CMDD-X or Compound 7, 10mg/kg), to verify the efficacy to prevent and treat the occurrence of fatty liver. As an analysis result, fat absorption into the liver was inhibited in all of the fatty liver occurrence model by medicine, the fatty liver occurrence model by high fat, and the fatty liver occurrence model by MCD. Moreover, the blood LDL was decreased and the liver function was significantly improved by inhibiting fatty acid biosynthesis and decomposing the accumulated fatty acid to release heat. This resulted in the reduction in ALT, which is a liver-function biomarker, and the reduction in MCP-1 and TNFa, which are inflammatory cytokines (FIGS. 15 to 19). In addition, the administration of Compound 7 also showed similar effects to the CMDD-X-administered group.

**Example 87: Verification on Antiatherogenic Efficacy of Compound of Chemical Formula I According to Present Invention**

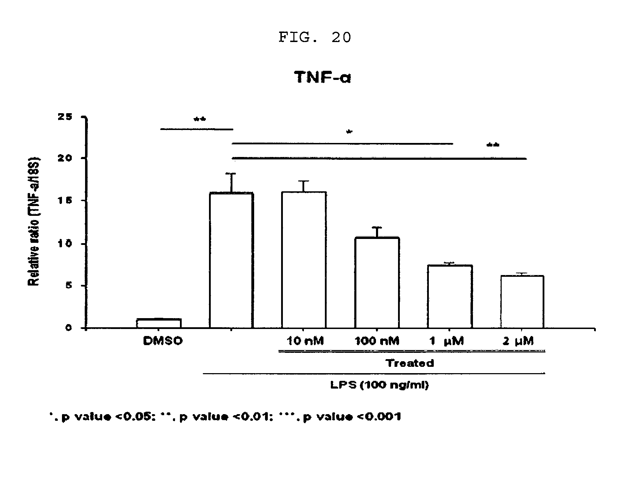

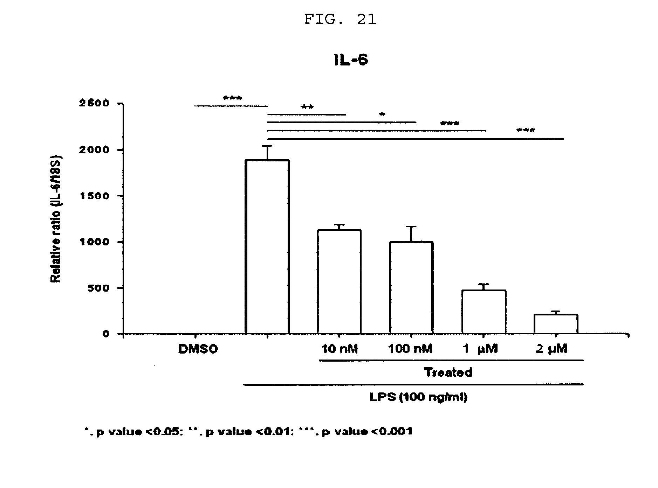

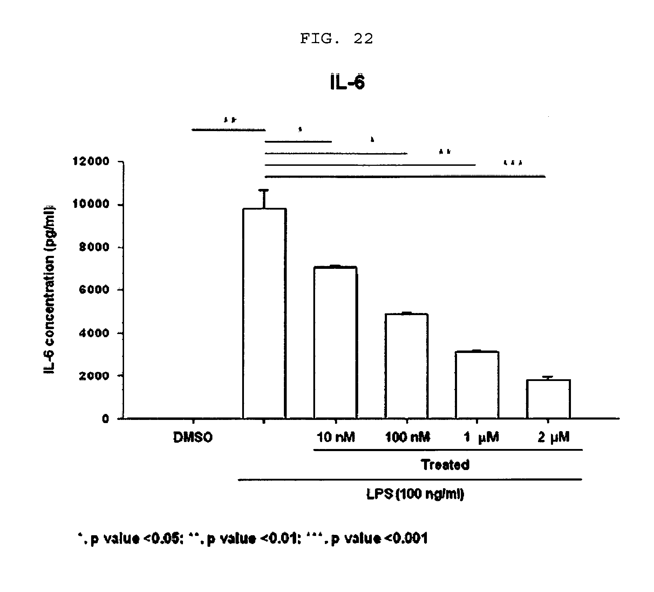

In order to test the antiatherogenic efficacy of the compound of Chemical Formula I (e.g., CMDD-X or Compound 7) according to the present invention, Raw264.7 macrophage activated by LPS and HUVEC endotheliocyte activated by TNF-α were used. Raw264.7 macrophage was cultured in DMEM medium within a cell incubator containing 5% carbon dioxide at 37°C. The medium contained 10% fetal bovine serum (FBS), 100 U/ml penicillin, and 100*µ*g/ml streptomycin. One day before compound treatment, raw264.7 cells were seeded on a 6-well plate at 50,000 cells/well. The next day, the cells were pre-treated with compound CMDD-X, for 1 hour, by the concentrations, and then were treated with 100 ng/ml LPS for 24 hours, and the qRT-PCR was conducted. TNF-α and IL-6 are cytokines involved in the occurrence of atherosclerosis, and generated mainly in the macrophage to induce an inflammatory reaction and induce leukocytes, to thereby advance atherosclerosis. As the compound CMDD-X concentration increased, expressions of TNF-α and IL-6 genes significantly decreased (FIGS. 20 and 21). In addition, the enzyme-linked immunosorbent assay (ELISA) test was conducted from the compound CMDD-X - treated medium. IL-6 secretion was significantly reduced (FIG. 22), which was consistent with the effects of the proceeding. In addition, the administration of Compound 7 also showed similar effects to the CMDD-X-administered group.

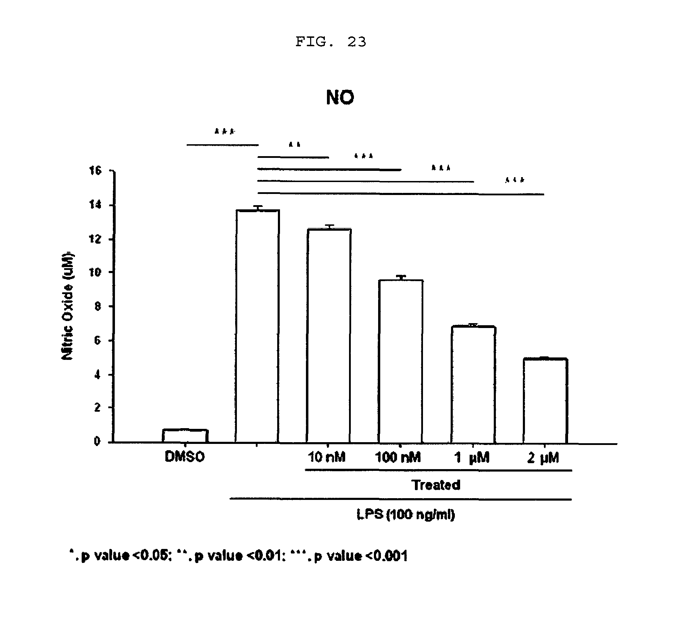

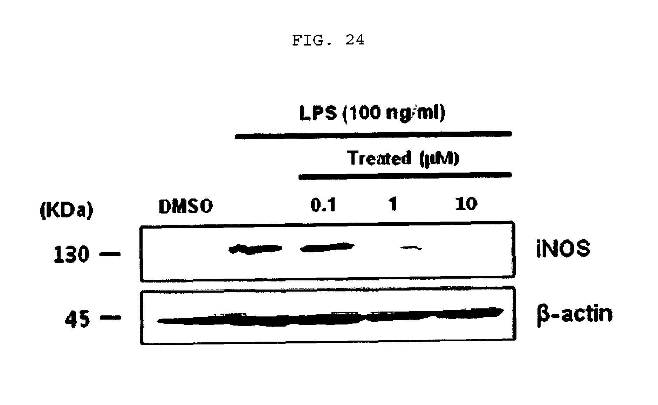

The nitric oxide (NO) concentration in the medium of the raw264.7 macrophage treated in the experiment was measured. NO is generated in the macrophage or foam cell present in a lesion site where atherosclerosis is advanced. This causes oxidative stress in the blood to generate oxLDL, and is involved in the rupture of the atherosclerosis progression site. In addition, it may be seen from iNOS previously reported and the apoE-deficient mouse results that NO generated by iNOS functions as an important factor in the occurrence and progression of atherosclerosis (Detmers PA et. al., J. Immunol. 2000, 3430-3435). As a result of NO assay, as the compound CMDD-X concentration increased, expression of NO significantly decreased (FIG. 23). In order to confirm whether or not iNOS expression is expressed by the compound CMDD-X, western blotting was conducted with respect to Raw264.7 macrophage activated with 100 ng/ml LPS for 8 hours after a 1 hour-pretreatment. As the result, the expression of iNOS increased by LPS was reduced by the compound CMDD-X (FIG. 24). In addition, the administration of Compound 7 also showed similar effects to the CMDD-X-administered group.

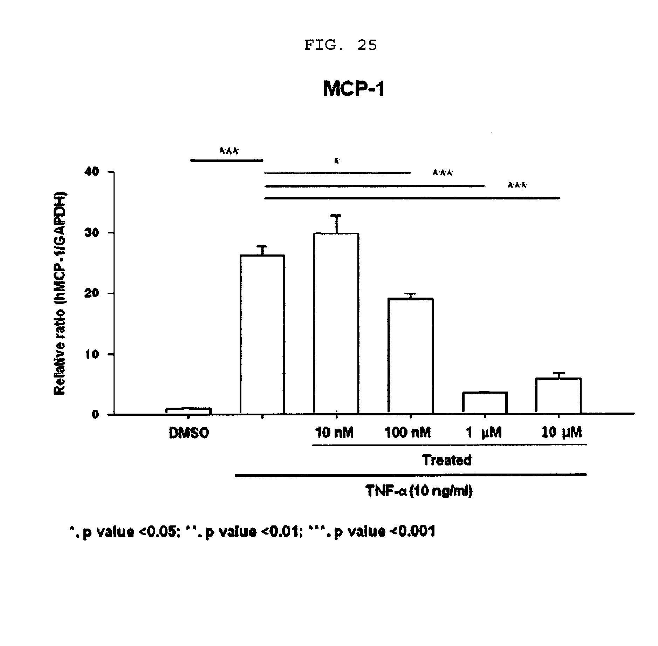

MCP-1, which is cytokine importantly involved in the atherosclerosis, is generated in the endotheliocyte to promote induction of monocytes into the inflammatory site and thus contributes to formation of atherosclerosis early lesions (Gerszten RE et al, NATURE, 1999, 718-723). HUVEC endotheliocytes were cultured in EGM-2 medium within a cell incubator containing 5% carbon dioxide at 37°C. One day before treatment with the compound CMDD-X, HUVEC endotheliocytes were seeded on a 6-well plate at 30,000cells/well. The next day, the cells were pre-treated with compound CMDD-X, for 1 hour, by the concentrations, and then were treated with 10 ng/ml TNF-α for 6 hours, and the qRT-PCR was conducted. As a result of qRT-PCR, as the compound CMDD-X concentration increased, expression of MCP-1 significantly decreased (FIG. 25). In addition, the administration of Compound 7 also showed similar effects to the CMDD-X-administered group.

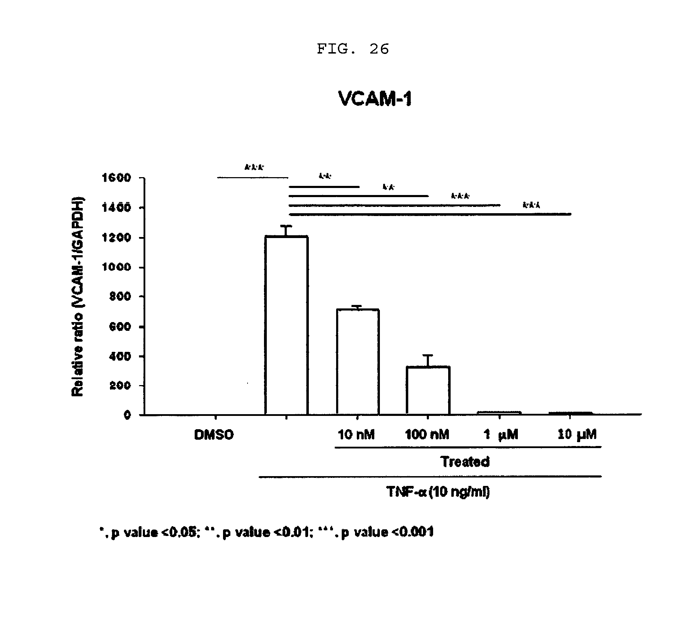

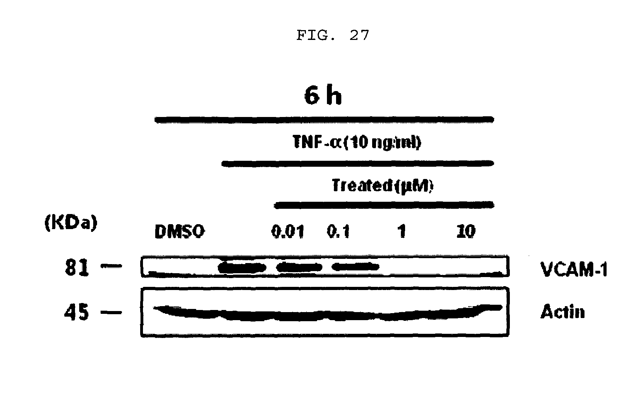

VCAM-1, which is another important factor of atherosclerosis, mediates attachment of monocytes in the early first stage of atherosclerosis and is expressed at the edge of the lesion to be involved in the expansion of the lesion (Nakashima Y *et al.,* Arterioscler Thromb Vasc Biol, 1998, 842-851). In order to verify efficacy of the compound CMDD-X on expression of the intercellular adhesion molecule gene (VCAM-1) in the HUVEC endotheliocytes and expression of protein, the samples were obtained by the same experiment and then qRT-PCR and Western experiments were conducted. As a result, expression of VCAM-1 was significantly decreased by the compound CMDD-X and expression of VCAM-1 protein was significantly decreased (FIGS. 26 and 27).

**Example 88: Verification on Parkinson's Disease Treatment Efficacy of Compound of Chemical Formula I According to Present Invention**

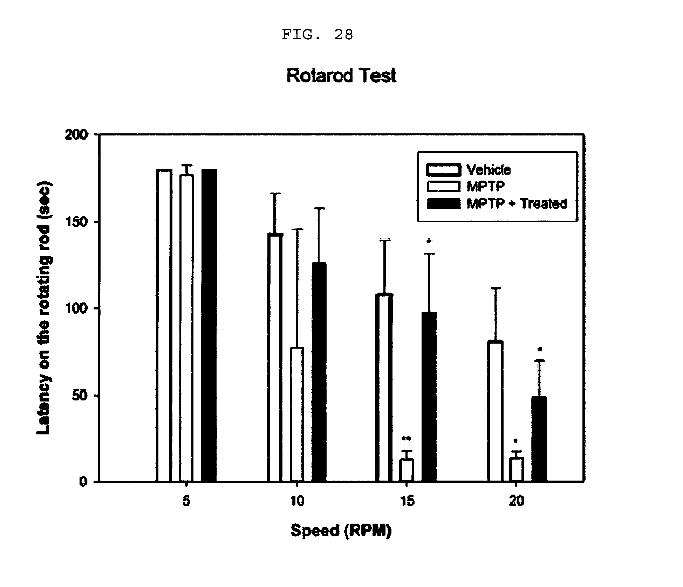

In the present example, in order to verify efficacy to prevent and treat Parkinson's Disease of the compound of Chemical Formula I according to the present invention in the Parkinson's Disease as one of neurodegenerative diseases, mice administered with 1-methyl-4-phenyl-1,2,3,6-tetrahydropyridine (MPTP) were used. The efficacy to prevent and treat the Parkinson's Disease was verified by administering the compound of Chemical Formula I (CMDD-X or Compound 7) to a Parkinson's Disease -induced disease model and then measuring exercise capacity of the mice. C57BL/6 mice (male, 12-week old) were used. The mice were adapted to a housing environment for 1 week while pellet type general diet was supplied, and then divided into 3 groups of 5 mice each to have similar body weights. Before MPTP administration, the compound CMDD-X was orally administered at a concentration of 5 mg/kg. After 3 hours, probenecid as MPTP efflux inhibitor was intraperitoneally administered to the mice at a concentration of 250 mg/kg, and, after 2 hours, 25 mg/kg of MPTP was intraperitoneally administered. Administration was conducted once per one day for a total of 5 days, and, after 3 days, exercise capacity of the mice was measured by using the Rotarod. The exercise capacity test was conducted by measuring the time while the mouse placed on the rod rotating at a predetermined speed was maintained on the rod without falling. The maximum 180 seconds were given, the speed was gradually increased, and the same break time was given. As a result of test, exercise capacity of the compound CMDD-X-administered group was recovered to a similar level to normal mice (FIG. 28). Compound 7 also showed the same results as CMDD-X. Therefore, the compound CMDD-X and Compound 7 showed superior efficacy in preventing and treating the Parkinson's Disease and neurodegenerative diseases.

**Example 89: Preparation of Formulation Containing Compound of Chemical Formula I as Effective Component**

The compound of Chemical Formula I of the present invention (e.g., CMDD-X or Compound 7) may be orally or non-orally administered to mammals including human beings. The use of the compound is not limited to a particular formulation, and may be formulated into oral administration, drip liquid, tablet, trituration, liquid suppository, external application, patch, intravenous injection, powder, granule, sugar-coated tablet, capsule, pill, suspension, liquid, ampoule, injection, or the like, and may be applied to any other shape of medicine.

Preparative Example 1: Preparation of Tablet

The tablet was prepared by the conventional method while mixing the following components.

Compound of Chemical Formula I (CMDD-X, Compound 7) 10mg

Lactose 95 mg

hydroxypropylcellulose 3 mg

Calcium carboxymethylcellulose 8 mg

Magnesium stearate 0.5 mg

Preparative Example 2: Preparation of Powder

The powder was prepared by the conventional method while mixing the following components.

Compound of Chemical Formula I (CMDD-X, Compound 7) 15 mg

Lactose 20 mg

Starch 15 mg

Magnesium stearate: adequate amount

Preparative Example 3: Preparation of Capsule

The hard capsule was prepared by the conventional method while mixing the following components.

Compound of Chemical Formula I (CMDD-X, Compound 7) 20 mg

Lactose 30 mg

Starch 25 mg

Talc 2 mg

Magnesium stearate adequate amount

Preparative Example 4: Preparation of Soft Capsule

Contents of the soft capsule were prepared by the conventional method while mixing the following components. The soft capsule was prepared by the conventional method using gelatin 132 mg, concentrated glycerin 50 mg, 70% D-sorbitol 6 mg, adequate amount of ethylvanillin as fragrance ingredient, and Carnauba Wax as a coating agent per one capsule.

Compound of Chemical Formula I (CMDD-X, Compound 7) 20 mg

Polyethyleneglycol 400 400 mg

Concentrated glycerin 50 mg

Purified water 35 mg

Preparative Example 5: Preparation of Suspension

The suspension was prepared by the conventional method while mixing the following components.

Compound of Chemical Formula I (CMDD-X, Compound 7) 15 mg

Isomerized sugar 10 g

Sugar 25 mg

Calcium carboxymethylcellulose 50 mg

Lemony flavor adequate amount

Total preparation amount was adjusted to 100 ml by using purified water.

Preparative Example 6: Preparation of Injection

Each ampoule (2 ml) containing contents of the following components was prepared by the conventional method while mixing the following components.

Compound of Chemical Formula I (CMDD-X, Compound 7) 10mg

Mannitol 180 mg

Sterile distilled water for injection 2974 mg

Disodium phosphate 26 mg

Preparative Example 7: Preparation of Ointment

Ointments (ointment examples 1 to 3) were prepared by the conventional method while mixing the following components.

[Ointment Example 1]

Compound of Chemical Formula I (CMDD-X, Compound 7) 0.5 wt%

White Petrolatum 70 wt%

Mineral oil 5 wt%

Polyoxylstearyl ether 5 wt%

Polyethylene glycol (ex.: PEG 400 or USP) 19.2 wt%

Butylhydroxyanisol 0.3 wt%

[Ointment Example 2]

Compound of Chemical Formula I (CMDD-X, Compound 7) 0.5 wt%

White Petrolatum 70 wt%

Mineral oil 5 wt%

Polyoxylstearyl ether 5 wt%

Polyethylene glycol (ex.: PEG 400 or USP) 19.2 wt%

Butylhydroxyanisol 0.2 wt%

Paraben (ex.: Methylparaben or propylparaben) 0.1 wt%

[Ointment Example 3]

Compound of Chemical Formula I (CMDD-X, Compound 7) 0.01 to 2 wt%

White Petrolatum 30 to 75 wt%

Mineral oil 2 to 10 wt%

Polyoxylstearyl ether 0.3 to 10 wt%

Polyethylene glycol (ex.: PEG 400 or USP) 2 to 45 wt%

Butylhydroxyanisol 0 wt% or 0.002 to 2.5 wt%

Paraben (ex.: methylparaben or propylparaben) 0 wt% or 0.01 to 1.5 wt%

Preparative Example 8: Preparation of Cream

Creams for external local use (cream examples 1 and 2) were prepared by the conventional method while mixing the following components.

[Cream Example 1]

Compound of Chemical Formula I (CMDD-X, Compound 7) 0.1 wt%

White Petrolatum 40 wt%

Mineral oil 11 wt%

Polyoxylstearyl ether 8.5 wt%

Propyleneglycol 18 wt%

Butylhydroxyanisol 0.02 wt%

Purified water adequate amount (22.38 wt%)

[Cream Example 2]

Compound of Chemical Formula I (CMDD-X, Compound 7) 0.01 to 3 wt%

White Petrolatum 30 to 75 wt%

Mineral oil 2 to 10 wt%

Polyoxylstearyl ether 0.3 to 10 wt%

Propyleneglycol 0.3 to 45 wt%

Butylhydroxyanisol 0.002 to 2.5 wt%

Paraben (ex.: Methylparaben or propylparaben) 0.01 to 3.5 wt%

Purified water appropriate amount (2 to 30 wt%)

Preparative Example 9: Preparation of Beverage

The beverage was prepared to have a total volume of 100 mL by the conventional method while mixing an adequate amount of purified water with the following components.

Compound of Chemical Formula I (CMDD-X, Compound 7) 2 mg

Citric acid 9 g

White sugar 9 g

Glucose 2.8 g

DL-malic acid 0.25 g

Purified water adequate amount

Preparative Example 10: Preparation of Food

10-1. Preparation of Seasoning for Cooking

Seasoning for cooking for health promotion was prepared by using 0.001-0.2 parts by weight of Compound of Chemical Formula 1 (CMDD-X, Compound 7).

10-2. Preparation of Flour Based Food

Food for health promotion was prepared by using the flour added with 0.001-0.2 parts by weight of Compound of Chemical Formula 1 (CMDD-X, Compound 7) to make bread, cakes, cookies, crackers, and noodles.

10-3. Preparation of Dairy Products

Various dairy products such as butter, ice cream, milk powder, and baby food, were prepared by using milk added with 0.001-0.2 parts by weight of Compound of Chemical Formula 1 (CMDD-X, Compound 7).

10-4. Preparation of Juice

Juice for health promotion was prepared by adding 0.001-0.2 parts by weight of Compound of Chemical Formula 1 (CMDD-X, Compound 7) to 1,000 Mℓ of juice of vegetable such as tomato or carrot or fruit such as apple, grape, orange, or pineapple.

Preparative Example 11: Preparation of Feedstuff for Animal

1 kg of feedstuff for animal was prepared by the conventional method while mixing the following components.

Compound of Chemical Formula I (CMDD-X, Compound 7) 0.1 g

Casein 200 g

Corn starch 400 g

Dyestrose 130 g

Sugar 100 g

Cellulose 50 g

Soybean oil 70 g

Antioxidant (ex.: t-butylhydroquinone) 0.02 g

Inorganic substance (ex.: salt mix) 35 g

Vitamin (ex.: vitamin mix) 10 g

L-cystine 3 g

Choline bitartrate 2 g

### [Industrial Applicability]

The sesterterpene compound of Chemical Formula I according to the present invention has superior Nurr1 activation, and thus can control and maintain blood sugar, treat insulin-independent diabetes, and prevent the occurrence of diabetes complications. Further, the sesterterpene compound of Chemical Formula I according to the present invention can be used as an effective component of a composition for improving, treating, and preventing diabetes and diabetes complications (foot ulcer and renal failure) by improving insulin sensitivity through regulation of hormones associated with glucose metabolism and protecting pancreatic function to thereby control fasting blood sugar and prevent the occurrence of diabetes complications (foot ulcer and renal failure).

Further, the sesterterpene compound of Chemical Formula I according to the present invention can have a superior effect in inhibiting differentiation of adipocytes, and thus can be useful in improving, preventing, and treating obesity.

Further, the sesterterpene compound of Chemical Formula I according to the present invention may be used as an effective component of a composition for preventing, treating, and improving alcoholic, non-alcoholic, and viral fatty liver diseases by inhibiting the generation of fatty acids in the liver and promoting beta-oxidation by which the fatty acids are burn to release the heat to thereby significantly reduce fat accumulation in the liver.

Further, the sesterterpene compound of Chemical Formula I according to the present invention reduces low-density lipoprotein (LDL) cholesterol and inhibits expressions of inflammatory cytokines derived from macrophage and endotheliocyte, signaling proteins, and lipid biosynthesis enzymes, and thus can be used as an effective component of a composition for improving, treating, and preventing vascular diseases such as hyperlipidemia and atherosclerosis, alone or in a complex agent together with the existing LXR agonists that have been developed until the present time.

Further, the sesterterpene compound of Chemical Formula I according to the present invention that activates Nurr1 may be used alone as an effective component of a composition for improving, treating, and preventing brain disorders such as Parkinson's disease, schizophrenia, and manic-depression.

Further, the sesterterpene compound of Chemical Formula I according to the present invention may be used as an effective component of a composition for improving, treating, and preventing Alzheimer's disease in a complex agent together with the existing LXR agonists.

## Claims

1. A sesterterpene compound represented by Chemical Formula I below: 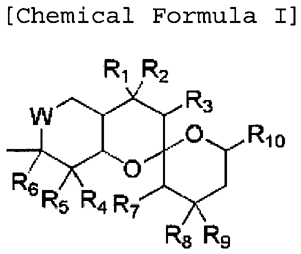 [in Chemical Formula I,
W is C(=A) or CR₁₁R₁₂, A is O, S, or NR₁₃, and R₁₃ is hydrogen, OH, (C1-C8)alkyl, (C2-C8)alkenyl, (C2-C8)alkynyl, (C3-C8)cycloalkyl, (C6-C20)aryl or (C4-C20)heteroaryl;
R₁₁ and R₁₂ each are independently hydrogen, -L-R₁₄, halogen, -N₃, (C2-C20)heteroaryl, (C6-C20)aryl, -NR₁₅R₁₆, 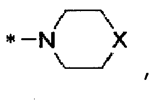 (C2-C8)alkenyl, or (C2-C8)alkynyl, but both R₁₁ and R₁₂ are not hydrogen;
L is O, NR₁₃, S, SO₂, or Se;
R₁₄ is hydrogen, (C1-C8)alkyl, (C1-C8)alkylcarbonyl, (C6-C20)aryl, -SO₂R₁₇, -(CH₂)ₘ-R₁₈, (C2-C8)alkenyl, (C2-C8)alkynyl, (C3-C8)cycloalkyl, (C4-C20)heteroaryl, amino
acid, glucose, or 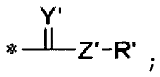 R₁₇ is (C6-C20)aryl, (C1-C8)alkyl, (C2-C8)alkenyl, (C2-C8)alkynyl, (C3-C8)cycloalkyl, (C4-C20)heteroaryl, amino acid, glucose, or 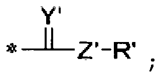 R₁₅ and R₁₆ each are independently hydrogen, (C1-C8)alkyl, - (CH₂)ₘ-R₁₈, (C2-C8)alkenyl, (C2-C8)alkynyl, (C3-C8)cycloalkyl, (C6-C20)aryl, (C4-C20)heteroaryl, amino acid, glucose, or 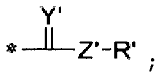 R₁₈ is (C2-C8)alkenyl, (C2-C8)alkynyl, -NR₁₉R₂₀, or (C2-C20)heteroaryl;
X is a single bond, CR₂₀, O, S, or NR₂₀;
R₁₉ and R₂₀ each are independently hydrogen, (C1-C8)alkyl, (C6-C20)aryl, (C2-C8)alkenyl, (C2-C8)alkynyl, (C3-C8)cycloalkyl, (C4-C20)heteroaryl, amino acid, or 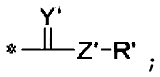 R₁ is -CH₂R₂₁, -COOH, -C(=O)R₂₂, (C2-C20)heteroaryl, 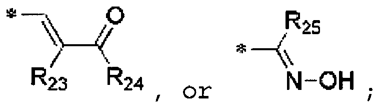 R₂₁ is -OR₂₆, N₃, -NR₁₅R₁₆, halogen, CN, NO₂, (C2-C20)heteroaryl, 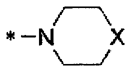 , -SR₂₀, -SO₂R₁₇, -SeR₂₀, glucose, or amino acid;
R₂₂ to R₂₅ each are independently hydrogen, CN, halogen, (C1-C8)alkyl, OR₁₄, 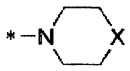 , or -NR₁₅R₁₆;
R₂₆ is hydrogen, (C1-C8)alkyl, (C6-C20)aryl, (C1-C8)alkyldi(C6-C20)arylacetyl, halo(C1-C8)alkyldi(C6-C20)arylacetyl, (C1-C8)alkyldi(C6-C20)arylsilyloxy, - (CH₂)mR₂₇, -C(O)R₂₇, -S(=O)₂R₂₈, -P(=O) (R₂₈)₂, (C2-C8)alkenyl, (C2-C8)alkynyl, (C3-C8)cycloalkyl, (C4-C20)heteroaryl, amino acid, 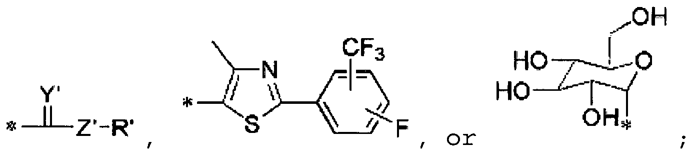 Y' is O, S, or NR"; Z' is a single bond, NH, O, S, or Se; R' and R" each are independently hydrogen, (C1-C8)alkyl, (C2-C8)alkenyl, (C2-C8)alkynyl, (C3-C8)cycloalkyl, (C6-C20)aryl, or (C4-C20)heteroary;
R₂₇ is (C1-C8)alkyl, (C2-C8)alkenyl, (C2-C8)alkynyl, (C6-C20)aryl, (C2-C20)heteroaryl, 5- to 6- membered heterocycloalkyl, or 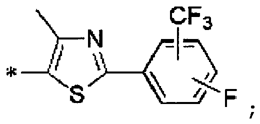 R₂₈ is (C1-C8)alkyl, (C6-C20)aryl, (C1-C8)alkoxy, (C6-C20)aryloxy, (C2-C20)heteroaryl, OH, or OM;
M is alkali metal;
R₄ is hydrogen, (C1-C8)alkyl, or -L-R₂₉; R₂₉ is hydrogen, (C1-C8)alkyl, (C1-C8)alkylcarbonyl, (C6-C20)aryl, or - SO₂R₁₇;
R₆ is hydrogen, (C1-C8)alkyl, or OH;
R₂, R₃, R₅, R₇ and R₈ each are independently hydrogen, (C1-C8)alkyl, (C6-C20)aryl, or (C2-C20)heteroaryl, and R₂ and R₃, R₅ and R₆, and R₇ and R₈ are independently linked to each other to form a double bond, linked via oxygen (O) to form epoxide, or linked via sulfur (S) to form thiirane;
R₉ is (C1-C8)alkyl, CHO, or COOH, and R₉ may form a double bond together with R₈;
R₁₀ is -(CH₂)ₘR₃₀, CHO, COOH, 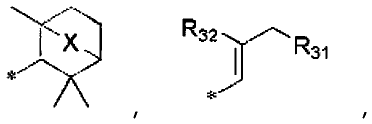 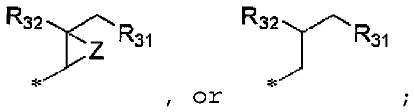 R₃₀ is hydrogen, halogen, OH, -NR₁₅R₁₆, SH, or SeH;
R₃₁ is (C1-C8)alkyl, 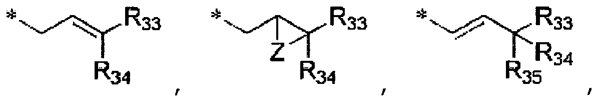 or 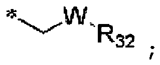 R₃₂ to R₃₅ each are independently hydrogen, (C1-C8)alkyl, (C6-C20)aryl, -OH, -SH, -NR₁₅R₁₆, -O-OH, amino acid, glucose,
or 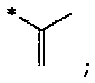 Z is O or S;
m is an integer of 1 to 5;
the heteroaryl of R₁ and R₂₁, the alkyl, aryl, and heteroaryl of R₂, R₃, R₅, R₇ and R₈, the alkyl of R₄, R₆, R₉, R₂₂ to R₂₅ and R₃₁, the heteroaryl, aryl, alkenyl, and alkynyl of R₁₁ and R₁₂, the alkyl, alkenyl, alkynyl, cycloalkyl, aryl, heteroaryl of R₁₃, R₁₅, R₁₆, R₁₉, R₂₀, R' and R", the alkyl, alkylcarbonyl, aryl, alkenyl, alkynyl, cycloalkyl, and heteroaryl of R₁₄, the aryl and alkyl of R₁₇ and R₃₂ to R₃₅, the alkenyl, alkynyl, and heteroaryl of R₁₈, the alkyl, aryl, alkyldiarylsilyloxy, alkenyl, alkynyl, cycloalkyl, heteroaryl of R₂₆, the alkyl, alkenyl, alkynyl, aryl, heteroaryl, heterocycloalkyl of R₂₇, the alkyl, aryl, alkoxy, aryloxy, and heteroaryl of R₂₈, the alkyl, alkylcarbonyl, and aryl of R₂₉ each may be further substituted with at least one substituent selected from a group consisting of (C1-C8)alkyl, halogen, (C6-C20)aryl, (C6-C20)ar(C1-C8)alkyl, halo(C1-C8)alkyl, cyano, nitro, (C1-C8)alkoxy, (C6-C20)aryloxy, (C1-C8)alkylthio, (C6-C20)arylthio, amino, mono- or di- (C1-C8)alkylamino, mono- or di- (C6-C20)arylamino, (C1-C8)alkyl(C6-C20)arylamino, (C1-C8)alkylcarbonyl, (C6-C20)arylcarbonyl, (C1-C8)alkoxycarbonyl, (C6-C20)aryloxycarbonyl, (C2-C20)heteroaryl, hydroxy, formyl, and carboxyl; and
the heteroaryl and heterocycloalkyl contains at least one hetero atom selected from N, O, and S.]

2. The sesterterpene compound of claim 1, wherein the compound of Chemical Formula I is selected from the compounds below: 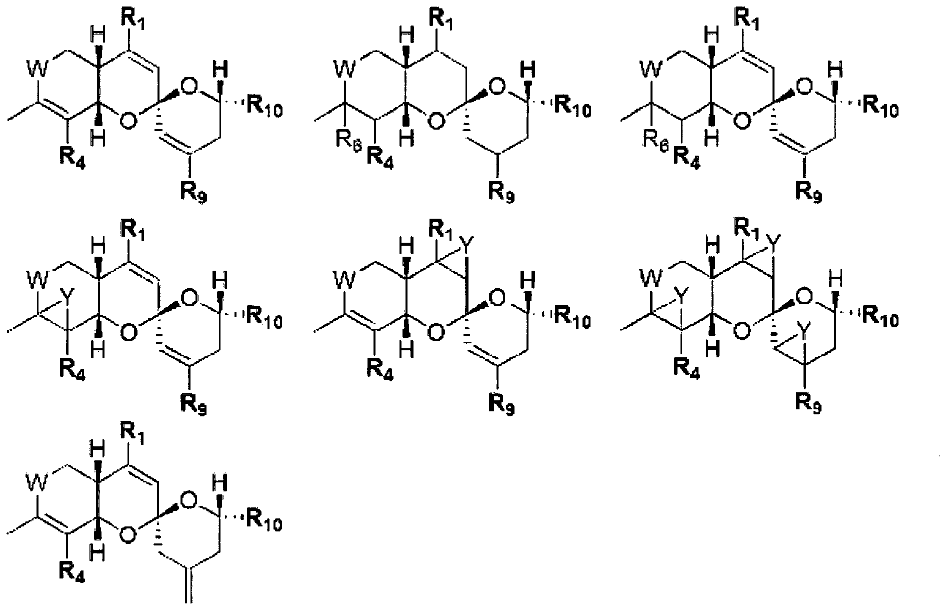 [Y is O or S;
W is 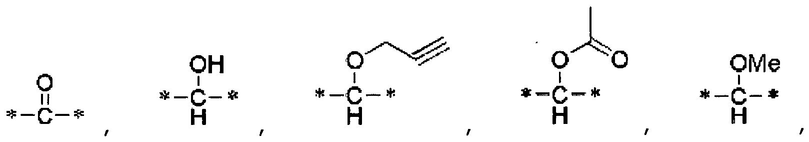 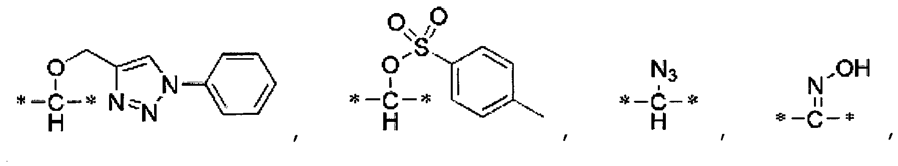 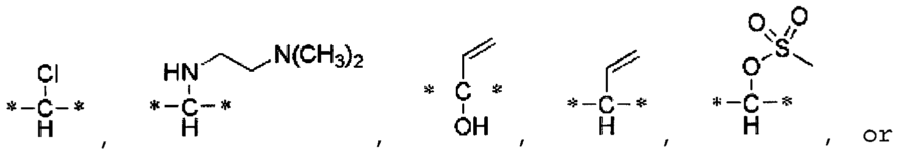 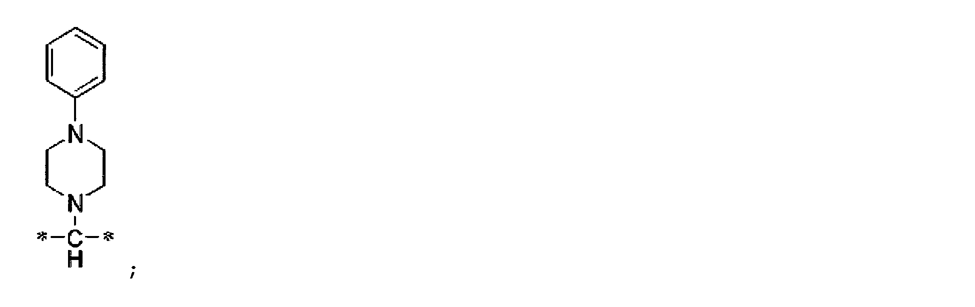 R₁ is 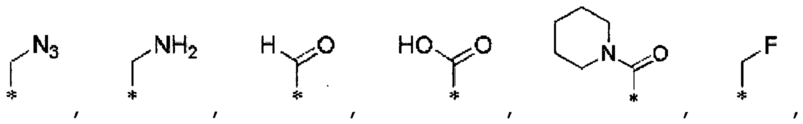 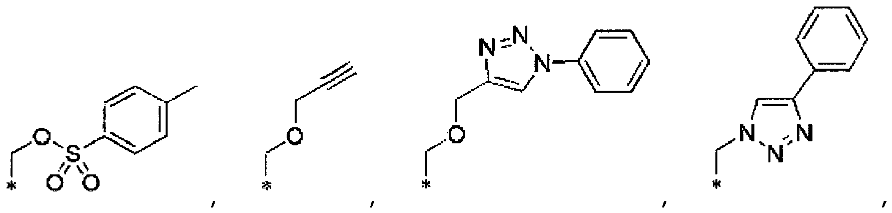 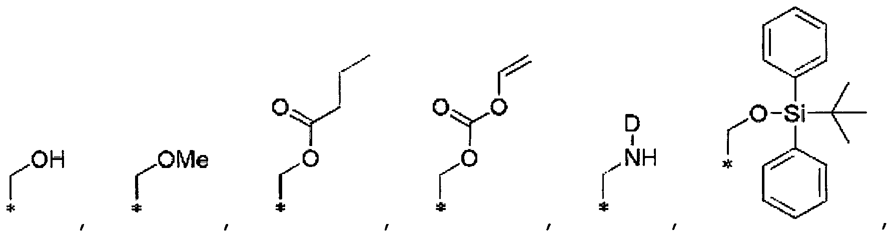 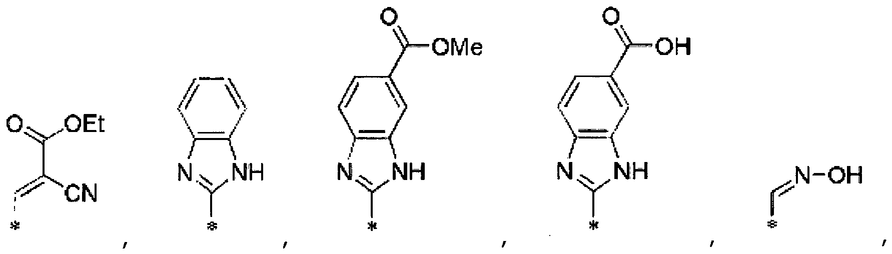 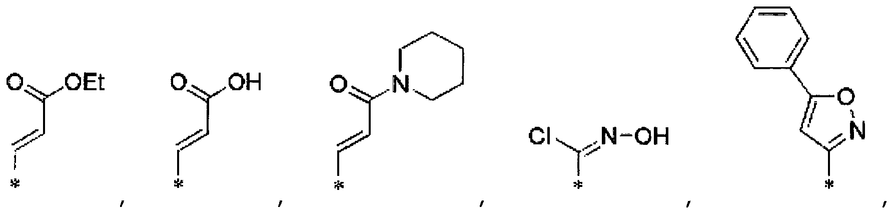 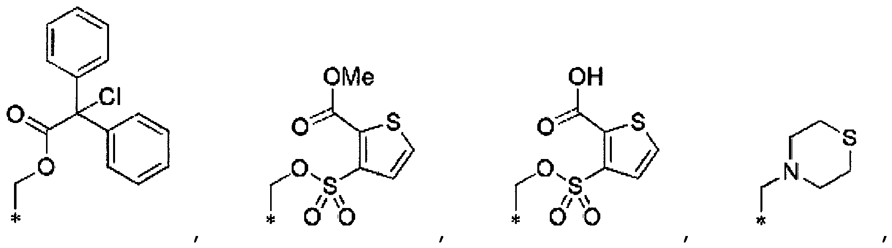 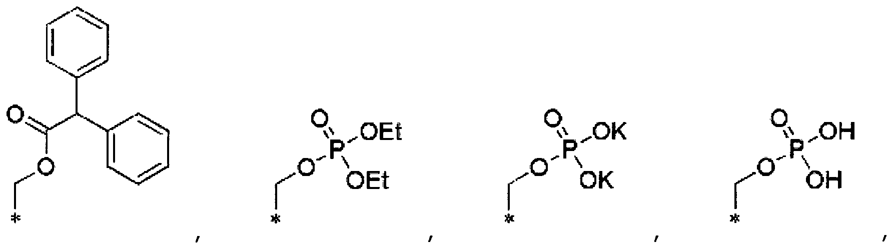 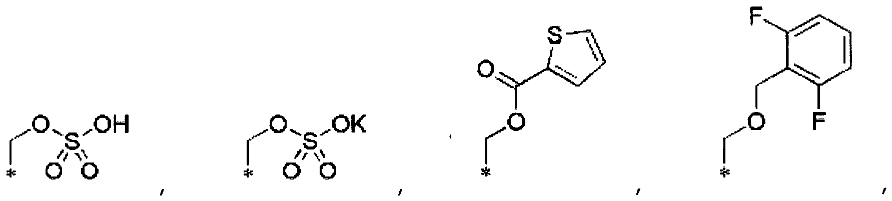 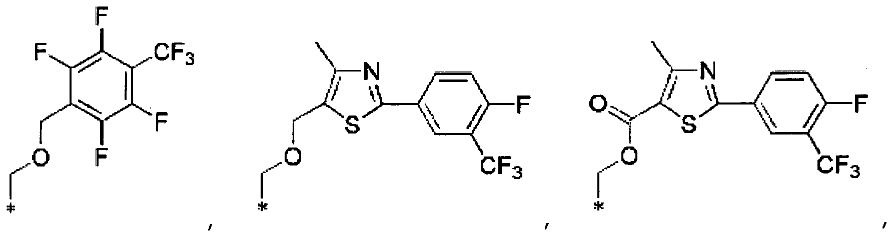 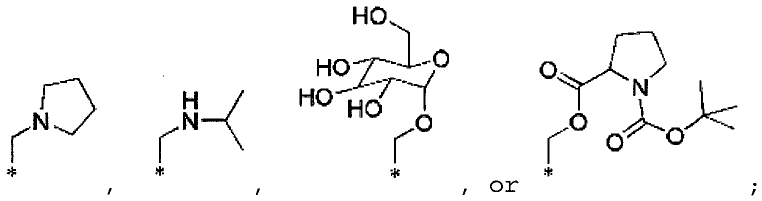 D is amino acid;
R₄ is H, CH₃, OH, SH, SeH, OTs, OMe, OAc, C(CN)₂, or 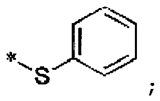 R₆ is H or 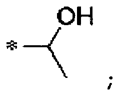 R₉ is CH₃, CH₂OH, CHO, or COOH;
R₁₀ is CH₂CH₃, CHO, COOH, CH₂Cl, CH₂F, CH₂NH₂, CH₂OH, CH₂SH, CH₂SeH,

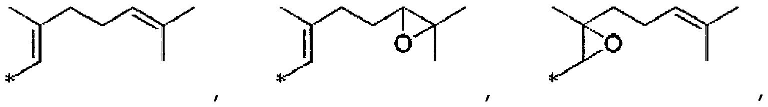

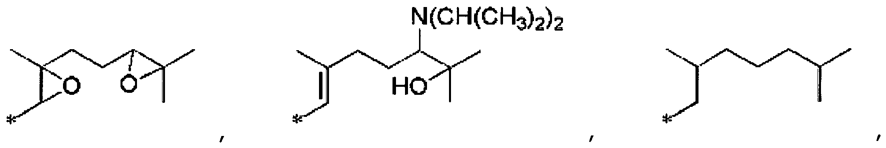

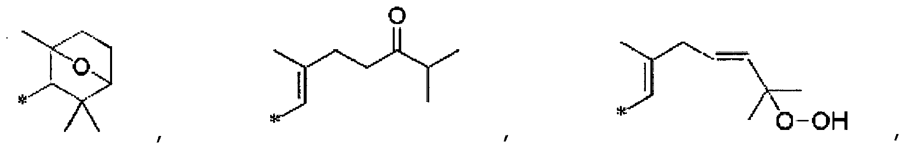

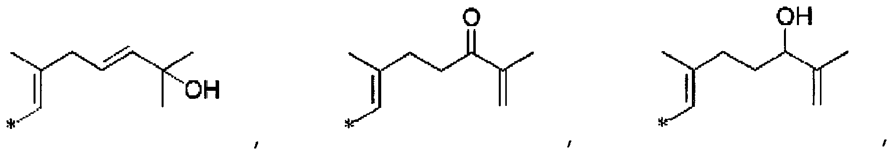

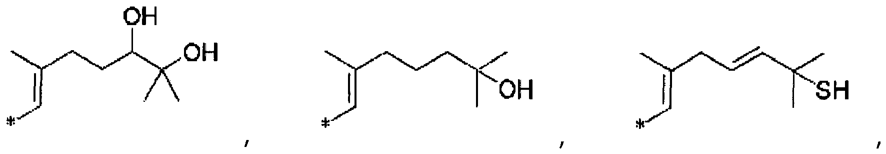

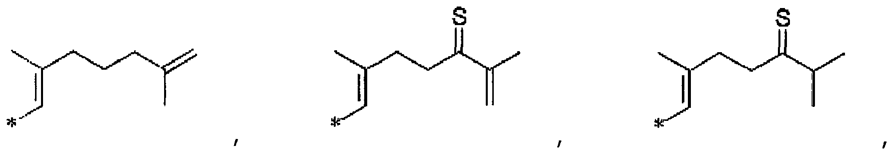

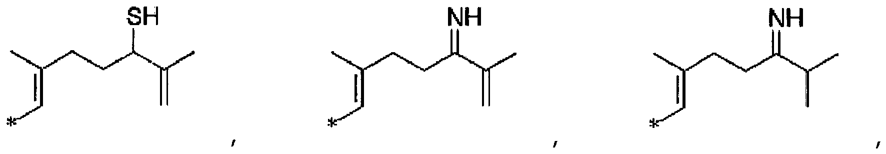

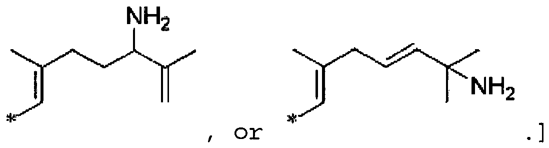

3. A pharmaceutical composition for preventing and treating diabetes, diabetes complications, vascular diseases, fatty liver disease, or obesity, the pharmaceutical composition containing the sesterterpene compound of Chemical Formula I of claim 1, a stereoisomer thereof, an enantiomer thereof, an in vivo-hydrolysable precursor thereof, or a pharmaceutically acceptable salt thereof.

4. The pharmaceutical composition of claim 3, wherein the diabetes complications foot ulcer and renal failure.

5. The pharmaceutical composition of claim 3, wherein the vascular diseases are hyperlipidemia, atherosclerosis, and atherosclerotic stroke.

6. The pharmaceutical composition of claim 3, wherein the fatty liver diseases are alcoholic, non-alcoholic, or viral fatty liver diseases.

7. A pharmaceutical composition for preventing and treating vascular diseases, the pharmaceutical composition containing the sesterterpene compound of Chemical Formula I of claim 1, a stereoisomer thereof, an enantiomer thereof, an in vivo-hydrolysable precursor thereof, or a pharmaceutically acceptable salt thereof and an LXR agonist.

8. The pharmaceutical composition of claim 7, wherein the vascular diseases are hyperlipidemia, atherosclerosis, and atherosclerotic stroke.

9. The pharmaceutical composition of claim 7, wherein the LXR agonist is N-(2,2,2-trifluoroethyl)-N-[4-[2,2,2-trifluoro-1-hydroxy-1-(trifluoromethyl)ethyl]phenyl]-benzenesulfonamide (T0901317) or 3-[3-[[[2-chloro-3-(trifluoromethyl)phenyl]methyl](2,2-diphenylethyl)amino]propoxy]benzeneacetic acid (GW3965).

10. A pharmaceutical composition for preventing and treating central nervous system diseases, the pharmaceutical composition containing the sesterterpene compound of Chemical Formula I of claim 1, a stereoisomer thereof, an enantiomer thereof, an in vivo-hydrolysable precursor thereof, or a pharmaceutically acceptable salt.

11. The pharmaceutical composition of claim 10, wherein the central nervous system diseases are Parkinson's disease, schizophrenia, and manic-depression.

12. A pharmaceutical composition for preventing and treating Alzheimer's disease, the pharmaceutical composition containing the sesterterpene compound of Chemical Formula I of claim 1, a stereoisomer thereof, an enantiomer thereof, an in vivo-hydrolysable precursor thereof, or a pharmaceutically acceptable salt thereof, and an LXR agonist.

13. The pharmaceutical composition of claim 12, wherein the LXR agonist is N-(2,2,2-trifluoroethyl)-N-[4-[2,2,2-trifluoro-1-hydroxy-1-(trifluoromethyl)ethyl]phenyl]-benzenesulfonamide (T0901317) or 3-[3-[[[2-chloro-3-(trifluoromethyl)phenyl]methyl](2,2-diphenylethyl)amino]propoxy]benzeneacetic acid (GW3965).

14. A pharmaceutical composition for preventing and treating diseases through Nurr1 activation, the pharmaceutical composition containing the sesterterpene compound of Chemical Formula I of claim 1, a stereoisomer thereof, an enantiomer thereof, an in vivo-hydrolysable precursor thereof, or a pharmaceutically acceptable salt.

15. A pharmaceutical composition for preventing and treating diseases through LXR inhibitory activity, the pharmaceutical composition containing the sesterterpene compound of Chemical Formula I of claim 1, a stereoisomer thereof, an enantiomer thereof, an in vivo-hydrolysable precursor thereof, or a pharmaceutically acceptable salt.

16. A composition for functional food and beverage for preventing and improving diabetes, diabetes complications, vascular diseases, fatty liver disease, or obesity, the composition containing the sesterterpene compound of Chemical Formula I of claim 1, a stereoisomer thereof, an enantiomer thereof, an in vivo-hydrolysable precursor thereof, or a salt thereof acceptable as a food additive.

17. The composition of claim 16, wherein the diabetes complications are foot ulcer or renal failure.

18. The composition of claim 16, wherein the vascular diseases are hyperlipidemia, atherosclerosis, and atherosclerotic stroke.

19. The composition of claim 16, wherein the fatty liver diseases are alcoholic, non-alcoholic, or viral fatty liver diseases.

20. A composition for functional food and beverage for preventing and improving vascular diseases, the composition containing the sesterterpene compound of Chemical Formula I of claim 1, a stereoisomer thereof, an enantiomer thereof, an in vivo-hydrolysable precursor thereof, or a salt thereof acceptable as a food additive, and an LXR agonist.

21. The composition of claim 20, wherein the vascular diseases are hyperlipidemia, atherosclerosis, or atherosclerotic stroke.

22. The composition of claim 20, wherein the LXR agonist is N-(2,2,2-trifluoroethyl)-N-[4-[2,2,2-trifluoro-1-hydroxy-1-(trifluoromethyl)ethyl]phenyl]-benzenesulfonamide (T0901317) or 3-[3-[[[2-chloro-3-(trifluoromethyl)phenyl]methyl](2,2-diphenylethyl)amino]propoxy]benzeneacetic acid (GW3965).

23. A composition for functional food and beverage for preventing and improving central nervous system diseases, the composition containing the sesterterpene compound of Chemical Formula I of claim 1, a stereoisomer thereof, an enantiomer thereof, an in vivo-hydrolysable precursor thereof, or a salt thereof acceptable as a food additive.

24. The composition of claim 23, wherein the central nervous system diseases are Parkinson's disease, schizophrenia, and manic-depression.

25. A composition for functional food and beverage for preventing and treating Alzheimer's disease, the composition containing the sesterterpene compound of Chemical Formula I of claim 1, a stereoisomer thereof, an enantiomer thereof, an in vivo-hydrolysable precursor thereof, or a salt thereof acceptable as a food additive, and an LXR agonist.

26. The composition of claim 25, wherein the LXR agonist is N-(2,2,2-trifluoroethyl)-N-[4-[2,2,2-trifluoro-1-hydroxy-1-(trifluoromethyl)ethyl]phenyl]-benzenesulfonamide (T0901317) or 3-[3-[[[2-chloro-3-(trifluoromethyl)phenyl]methyl](2,2-diphenylethyl)amino]propoxy]benzeneacetic acid (GW3965).

27. A composition for functional cosmetics for preventing and improving obesity, the composition containing the sesterterpene compound of Chemical Formula I of claim 1, a stereoisomer thereof, an enantiomer thereof, an in vivo-hydrolysable precursor thereof, or a salt thereof acceptable as a cosmetic additive.

28. A composition for functional food and beverage for preventing and improving diseases through Nurr1 activation, the composition containing the sesterterpene compound of Chemical Formula I of claim 1, a stereoisomer thereof, an enantiomer thereof, an in vivo-hydrolysable precursor thereof, or a salt thereof acceptable as a food additive.

29. A composition for functional food and beverage for preventing and improving diseases through LXR inhibitory activity, the composition containing the sesterterpene compound of Chemical Formula I of claim 1, a stereoisomer thereof, an enantiomer thereof, an in vivo-hydrolysable precursor thereof, or a salt thereof acceptable as a food additive.

30. A composition for functional feedstuff for preventing and improving diabetes, diabetes complications, vascular diseases, fatty liver disease, or obesity, the composition containing the sesterterpene compound of Chemical Formula I of claim 1, a stereoisomer thereof, an enantiomer thereof, an in vivo-hydrolysable precursor thereof, or a salt thereof acceptable as a food additive.

31. The composition of claim 30, wherein the diabetes complications are foot ulcer or renal failure.

32. The composition of claim 30, wherein the vascular diseases are hyperlipidemia, atherosclerosis, and atherosclerotic stroke.

33. The composition of claim 30, wherein the fatty liver diseases are alcoholic, non-alcoholic, or viral fatty liver diseases.

34. A composition for functional feedstuff for preventing and improving vascular diseases, the composition containing the sesterterpene compound of Chemical Formula I of claim 1, a stereoisomer thereof, an enantiomer thereof, an in vivo-hydrolysable precursor thereof, or a salt thereof acceptable as a food additive, and an LXR agonist.

35. The composition of claim 34, wherein the vascular diseases are hyperlipidemia, atherosclerosis, or atherosclerotic stroke.

36. The composition of claim 34, wherein the LXR agonist is N-(2,2,2-trifluoroethyl)-N-[4-[2,2,2-trifluoro-1-hydroxy-1-(trifluoromethyl)ethyl]phenyl]-benzenesulfonamide (T0901317) or 3-[3-[[[2-chloro-3-(trifluoromethyl)phenyl]methyl](2,2-diphenylethyl)amino]propoxy]benzeneacetic acid (GW3965).

37. A composition for functional feedstuff for preventing and improving central nervous system diseases, the composition containing the sesterterpene compound of Chemical Formula I of claim 1, a stereoisomer thereof, an enantiomer thereof, an in vivo-hydrolysable precursor thereof, or a salt thereof acceptable as a food additive.

38. The composition of claim 37, wherein the central nervous system diseases are Parkinson's disease, schizophrenia, and manic-depression.

39. A composition for functional feedstuff for preventing and improving Alzheimer's disease, the composition containing the sesterterpene compound of Chemical Formula I of claim 1, a stereoisomer thereof, an enantiomer thereof, an in vivo-hydrolysable precursor thereof, or a salt thereof acceptable as a food additive, and an LXR agonist.

40. The composition of claim 39, wherein the LXR agonist is N-(2,2,2-trifluoroethyl)-N-[4-[2,2,2-trifluoro-1-hydroxy-1-(trifluoromethyl)ethyl]phenyl]-benzenesulfonamide (T0901317) or 3-[3-[[[2-chloro-3-(trifluoromethyl)phenyl]methyl](2,2-diphenylethyl)amino]propoxy]benzeneacetic acid (GW3965).
